# EUROPEAN PATENT SPECIFICATION

(11) **EP 2 614 156 B1**
(45) Date of publication and mention of the grant of the patent: **01.08.2018**
(21) Application number: 11823878.1
(22) Date of filing: 07.09.2011
(51) Int. Cl.: C12Q 1/68, G01N 33/50, C12N 15/09, G01N 33/487

(54) **CONTROL OF DNA MOVEMENT IN A NANOPORE AT ONE NUCLEOTIDE PRECISION BY A PROCESSIVE ENZYME**
BIS AUF EIN NUKLEOTID GENAUE STEUERUNG DER DNA-BEWEGUNG IN EINER NANOPORE MITHILFE EINES PROZESSIVEN ENZYMS
CONTRÔLE DU MOUVEMENT DE L'ADN DANS UN NANOPORE PRÉCIS AU NUCLÉOTIDE PRÈS PAR UNE ENZYME PROCESSIVE

(30) Priority: 30.07.2011 US 201161574236 P; 07.09.2010 US 402903 P; 30.07.2011 US 201161574240 P; 30.07.2011 US 201161574237 P; 30.07.2011 US 201161574239 P; 30.07.2011 US 201161574238 P; 30.07.2011 US 201161574235 P; 30.07.2011 US 201161574233 P
(43) Date of publication of application: 17.07.2013
(73) Proprietor: The Regents of the University of California, Oakland, CA 94607 (US)
(72) Inventor: CHERF, Gerald Maxwell, Santa Cruz, CA 95064 (US); LIEBERMAN, Kathy R., Santa Cruz, CA 95064 (US); LAM, Christopher Evan, San Francisco, CA 94116 (US); DOODY, Michael, Mountain View, CA 94040 (US); AKESON, Mark A., Santa Cruz, CA 95064 (US)
(74) Representative: J A Kemp
(86) International application number: PCT/US2011/001552
(87) International publication number: WO 2012/033524

(56) References cited:
- US-A1- 2010 035 260
- Anonymous: "Nanopore Sequencing Takes More Small Steps - Bio-IT World", , 3 December 2010 (2010-12-03), XP055097439, Retrieved from the Internet: URL:http://www.bio-itworld.com/BioIT_Artic le.aspx?id=103211 [retrieved on 2014-01-21]
- JEFFREY PERKEL: "Making Contact with Sequencing's Fourth Generation", BIOTECHNIQUES, vol. 50, no. 1, 9 February 2011 (2011-02-09), pages 93-95, XP055097485,
- PENG JING ET AL: "Robust properties of membrane-embedded connector channel of bacterial virus phi29 DNA packaging motor", MOLECULAR BIOSYSTEMS, vol. 6, no. 10, 1 January 2010 (2010-01-01), page 1844, XP055062619, ISSN: 1742-206X, DOI: 10.1039/c003010d
- DAVID WENDELL ET AL: "Translocation of double-stranded DNA through membrane-adapted phi29 motor protein nanopores", NATURE NANOTECHNOLOGY, vol. 4, no. 11, 27 November 2009 (2009-11-27), pages 765-772, XP055062614, ISSN: 1748-3387, DOI: 10.1038/nnano.2009.259
- COCKROFT, S. L. ET AL.: 'A single-molecule nanopore device detects DNA polymerase activity with single-nucleotide resolution' JOURNAL OF THE AMERICAN CHEMICAL SOCIETY vol. 130, no. 3, 01 January 2008, pages 818 - 820
- DEAMER, D.: 'Nanopore analysis of nucleic acids bound to exonucleases and polymerases' ANNUAL REVIEW OF BIOPHYSICS. vol. 39, 09 June 2010, pages 79 - 90
- BENNER, S. ET AL.: 'Sequence-specific detection of individual DNA polymerase complexes in real time using a nanopore' NATURE NANOTECHNOLOGY. vol. 2, no. 11, 28 October 2007, pages 718 - 724
- LIEBERMAN, K. R. ET AL.: 'Processive replication of single NA molecules in a nanopore catalyzed by phi29 DNA polymerase' JOURNAL OF THE AMERICAN CHEMICAL SOCIETY vol. 132, no. 50, 01 December 2010, pages 17961 - 17972
- Kate R. Lieberman ET AL: "Processive Replication of Single DNA Molecules in a Nanopore Catalyzed by phi29 DNA Polymerase", Journal of the American Chemical Society, vol. 132, no. 50, 22 December 2010 (2010-12-22), pages 17961-17972, XP055028641, ISSN: 0002-7863, DOI: 10.1021/ja1087612

## Description

### Field of the Invention

Disclosed herein are devices and methods that can regulate the time at which an individual polymer in a mixture is acted upon by another compound, for example, an enzyme. The invention is of particular use in the fields of molecular biology, structural biology, cell biology, molecular switches, molecular circuits, and molecular computational devices, and the manufacture thereof. The invention also relates to methods of using the compositions to diagnose whether a subject is susceptible to cancer, autoimmune diseases, cell cycle disorders, or other disorders.

### Background

The disclosure relates to the field of compositions, methods, and apparatus for characterizing polynucleotides and other polymers.

Determining the nucleotide sequence of DNA and RNA in a rapid manner is a major goal of researchers in biotechnology, especially for projects seeking to obtain the sequence of entire genomes of organisms. In addition, rapidly determining the sequence of a polynucleotide is important for identifying genetic mutations and polymorphisms in individuals and populations of individuals.

Nanopore sequencing is one method of rapidly determining the sequence of polynucleotide molecules. Nanopore sequencing is based on the property of physically sensing the individual nucleotides (or physical changes in the environment of the nucleotides (that is, for example, an electric current)) within an individual polynucleotide (for example, DNA and RNA) as it traverses through a nanopore aperture. In principle, the sequence of a polynucleotide can be determined from a single molecule. However, in practice, it is preferred that a polynucleotide sequence be determined from a statistical average of data obtained from multiple passages of the same molecule or the passage of multiple molecules having the same polynucleotide sequence. The use of membrane channels to characterize polynucleotides as the molecules pass through the small ion channels has been studied by Kasianowicz et al. (Proc. Natl. Acad. Sci. USA. 93:13770-13773, 1996, incorporate herein by reference) by using an electric field to force single stranded RNA and DNA molecules through a 1.5 nanometer diameter nanopore aperture (for example, an ion channel) in a lipid bilayer membrane. The diameter of the nanopore aperture permitted only a single strand of a polynucleotide to traverse the nanopore aperture at any given time. As the polynucleotide traversed the nanopore aperture, the polynucleotide partially blocked the nanopore aperture, resulting in a transient decrease of ionic current. Since the length of the decrease in current is directly proportional to the length of the polynucleotide, Kasianowicz et al. (1996) were able to determine experimentally lengths of polynucleotides by measuring changes in the ionic current.

Baldarelli et al. (U.S. Pat. No. 6,015,714) and Church et al. (U.S. Pat. No. 5,795,782) describe the use of nanopores to characterize polynucleotides including DNA and RNA molecules on a monomer by monomer basis. In particular, Baldarelli et al. characterized and sequenced the polynucleotides by passing a polynucleotide through the nanopore aperture. The nanopore aperture is imbedded in a structure or an interface, which separates two media. As the polynucleotide passes through the nanopore aperture, the polynucleotide alters an ionic current by blocking the nanopore aperture. As the individual nucleotides pass through the nanopore aperture, each base/nucleotide alters the ionic current in a manner that allows the identification of the nucleotide transiently blocking the nanopore aperture, thereby allowing one to characterize the nucleotide composition of the polynucleotide and perhaps determine the nucleotide sequence of the polynucleotide.

One disadvantage of previous nanopore analysis techniques is controlling the rate at which the target polynucleotide is analyzed. As described by Kasianowicz, et al. (1996), nanopore analysis is a useful method for performing length determinations of polynucleotides. However, the translocation rate is nucleotide composition dependent and can range between 10⁵ to 10⁷ nucleotides per second under the measurement conditions outlined by Kasianowicz et al. (1996). Therefore, the correlation between any given polynucleotide's length and its translocation time is not straightforward. It is also anticipated that a higher degree of resolution with regard to both the composition and spatial relationship between nucleotide units within a polynucleotide can be obtained if the translocation rate is substantially reduced.

Recently, the properties of DNA or RNA molecules bound to nucleic acid processing enzymes have been analyzed at a nanopore orifice. The complexes studied include those of single-stranded DNA with *Escherichia coli* Exonuclease I (Hornblower, B.; Coombs, A.; Whitaker, R. D.; Kolomeisky, A.; Picone, S. J.; Meller, A.; Akeson, M. Nat. Methods. 2007, 4, 315-317), RNA with the bacteriophage phi8 ATPase (Astier, Y.; Kainov, D. E.; Bayley, H.; Tuma, R.; Howorka, S. Chemphyschem. 2007, 8, 2189-2194), and primer/template DNA substrates bound to the 3'-5'-exonuclease deficient versions of two A-family DNA polymerases, the Klenow fragment of *E. coli* DNA polymerase (KF(exo-)) and bacteriophage T7 DNA polymerase (T7DNAP(exo-)) (Benner, S.; Chen, R. J.; Wilson, N. A.; Abu-Shumays, R.; Hurt, N.; Lieberman, K. R.; Deamer, D. W.; Dunbar, W. B.; Akeson, M. Nat. Nanotechnol. 2007, 2, 718-724; Cockroft, S. L.; Chu, J.; Amorin, M.; Ghadiri, M. R. J. Am. Chem. Soc. 2008, 130, 818-820; Gyarfas, B.; Olasagasti, F.; Benner, S.; Garalde, D.; Lieberman, K. R.; Akeson, M. ACS. Nano. 2009, 3, 1457-1466; Hurt, N.; Wang, H.; Akeson, M.; Lieberman, K. R. J. Am. Chem. Soc. 2009, 131, 3772-3778; Wilson, N. A.; Abu-Shumays, R.; Gyarfas, B.; Wang, H.; Lieberman, K. R.; Akeson, M.; Dunbar, W. B. ACS. Nano. 2009, 3, 995-1003. We have demonstrated that T7DNAP(exo-) could replicate and advance a DNA template held in the α-hemolysin (α-HL) nanopore against an 80 mV applied potential (Olasagasti, F.; Lieberman, K. R.; Benner, S.; Cherf, G. M.; Dahl, J. M.; Deamer, D. W.; Akeson, M., Nat. Nanotechnol. 2010, advance online publication, doi:10.1038/nnano.2010.2177). However, due to the low stability of the T7DNAP(exo-)-DNA complex under load, diminished signal to noise ratio at 80 mV potential, and the high turnover rate of the polymerase, it was difficult to detect ionic current steps that reported more than three sequential nucleotide additions during replication. International Patent Application No. PCT/US2008/004467 and related US Patent Application Nos. 12/080,684 and 12/459,059 disclose a number of technologies that comprise α-hemolysin nanopores coupled with several exemplary DNA polymerases that may be used with the technologies disclosed herein.

There is currently a need to provide compositions and methods that can be used in characterization of polymers, including polynucleotides and polypeptides, as well as diagnosis and prognosis of diseases and disorders. There is also a need in the art to provide systems and methods that can detect single nucleotides in a timeframe that can be used to distinguish not only between individual nucleotides in a polynucleotide but also the chemical characteristics of the individual nucleotide. In particular there is also a need to provide compositions that are tolerant in vitro to elevated concentrations of salts.

### Brief Description of the Invention

The inventors have surprisingly demonstrated that Phi29 DNA polymerase acts like a molecular brake controlling the movement of a polynucleotide through a pore along the field resulting from an applied voltage. The polymerase is surprisingly capable of controlling the movement of a polynucleotide through a pore in three modes, namely the polymerase mode, the exonuclease mode and the unzipping mode. The polymerase mode and exonuclease modes are based on the normal activity of the enzyme. When both polymerase and exonuclease activity are inhibited, Phi29 DNA polymerase surprisingly unzips dsDNA when pulled through a nanopore by a strong applied field. This has been termed unzipping mode. Unzipping mode implies that it is the unzipping of dsDNA above or through the enzyme, and importantly, it is the requisite force required to disrupt the interactions of both strands with the enzyme and to overcome the hydrogen bonds between the hydridised states. Herein we describe how Phi29 DNA polymerase can act as a molecular brake for a polynucleotide, enabling sufficient controlled movement through a nanopore for sequencing.

Accordingly, the invention provides a method of sequencing a target polynucleotide, comprising:
(a) contacting the target polynucleotide with a transmembrane pore and a Phi29 DNA polymerase such that the Phi29 DNA polymerase controls the movement of the target polynucleotide through the pore during the target polynucleotide interactions with the pore; and
(b) measuring a current passing through the pore during interaction and thereby determining the sequence of the target polynucleotide, wherein
steps (a) and (b) are carried out with a voltage applied across the pore while the Phi29 DNA polymerase is bound to the target polynucleotide, and further wherein steps (a) and (b) are carried out:
(I) in the absence of free nucleotides and the absence of an enzyme cofactor such that the Phi29 DNA polymerase moves the target polynucleotide through the pore with the field resulting from the applied voltage, optionally wherein the method further comprises: (c) lowering the voltage applied across the pore such that the Phi29 DNA polymerase moves the target polynucleotide through the pore in the opposite direction to steps (a) and (b) and nucleotides in the target polynucleotide interact with the pore; and (d) measuring the current passing through the pore during each interaction and thereby proof reading the sequence of the target polynucleotide obtained in step (b), wherein steps (c) and (d) are also carried out with a voltage applied across the pore;
   or
(II) in the absence of free nucleotides and the presence of an enzyme cofactor such that the Phi29 DNA polymerase moves the target polynucleotide through the pore with the field resulting from the applied voltage, optionally wherein the method further comprises: (c) adding free nucleotides such that the Phi29 DNA polymerase moves the target polynucleotide through the pore in the opposite direction to steps (a) and (b) and nucleotides in the target polynucleotide interact with the pore; and (d) measuring the current passing through the pore during each interaction and thereby proof reading the sequence of the target polynucleotide obtained in step (b), wherein steps (c) and (d) are also carried out with a voltage applied across the pore;
   or
(III) in the presence of free nucleotides and an enzyme cofactor such that the Phi29 DNA polymerase moves the target polynucleotide through the pore against the field resulting from the applied voltage, optionally wherein the method further comprises: (c) removing the free nucleotides such that the Phi29 DNA polymerase moves the target polynucleotide through the pore in the opposite direction to steps (a) and (b) and nucleotides in the target polynucleotide interact with the pore; and (d) measuring the current passing through the pore during each interaction and thereby proof reading the sequence of the target polynucleotide obtained in step (b), wherein steps (c) and (d) are also carried out with a voltage applied across the pore.

The invention also provides a system for determining the nucleotide sequence of a polynucleotide in a sample, the system comprising:
an electrical source, an anode, a cathode,
   a *cis* chamber, a *trans* chamber, wherein the *cis* and the *trans* chambers are separated by a thin film, the thin film having a nanopore,
a conducting solvent,
a Phi29 DNA polymerase for controlling movement of the polynucleotide through the nanopore along a field resulting from an applied voltage, and
   a plurality of dNTP molecules.

The invention further provides a kit for sequencing a target polynucleotide comprising (a) an apparatus comprising an electrical source, an anode, a cathode, a *cis* chamber and a *trans* chamber, wherein the *cis* and the *trans* chambers are separated by a thin film, the thin film having a nanopore; and (b) a Phi29 DNA polymerase.

Also disclosed herein is a method of sequencing a target polynucleotide, comprising:
a. contacting the target polynucleotide with a transmembrane pore and a Phi29 DNA polymerase such that the polymerase controls the movement of the target polynucleotide through the pore and a proportion of the nucleotides in the target polynucleotide interacts with the pore; and
b. measuring the current passing through the pore during each interaction and thereby determining the sequence of the target polynucleotide, wherein steps (a) and (b) are carried out with a voltage applied across the pore.

The method is preferably carried out in one of three modes as follows:
(1) steps (a) and (b) are preferably carried out in the presence of free nucleotides and an enzyme cofactor such that the polymerase moves the target polynucleotide through the pore against the field resulting from the applied voltage;
(2)steps (a) and (b) are preferably carried out in the absence of free nucleotides and the presence of an enzyme cofactor such that the polymerase moves the target polynucleotide through the pore with the field resulting from the applied voltage; or
(3) steps (a) and (b) are preferably carried out in the absence of free nucleotides and the absence of an enzyme cofactor such that the polymerase moves the target polynucleotide through the pore with the field resulting from the applied voltage.

The disclosure also provides a method of forming a sensor for sequencing a target polynucleotide, comprising: (a) contacting a pore with a Phi29 DNA polymerase in the presence of the target polynucleotide; and (b) applying a voltage across the pore to form a complex between the pore and the polymerase; and thereby forming a sensor for sequencing the target polynucleotide.

The disclosure also provides a method of increasing the rate of activity of a Phi29 DNA polymerase, comprising: (a) contacting the Phi29 DNA polymerase with a pore in the presence of a polynucleotide; and (b) applying a voltage across the pore to form a complex between the pore and the polymerase; and thereby increasing the rate of activity of a Phi29 DNA polymerase.

The disclosure also provides use of a Phi29 DNA polymerase to control the movement of a target polynucleotide through a pore.

The invention also provides a kit for sequencing a target polynucleotide comprising (a) a pore and (b) a Phi29 DNA polymerase.

The disclosure also provides an analysis apparatus for sequencing target polynucleotides in a sample, comprising a plurality of pores and a plurality of Phi29 DNA polymerases.

The disclosure also provides a system for determining the nucleotide sequence of a polynucleotide in a sample, the system comprising an electrical source, an anode, a cathode, a *cis* chamber, a *trans* chamber, wherein the *cis* and the *trans* chambers are separated by a thin film, the thin film having a plurality of apertures (pores), wherein each aperture (pore) is between about 0.25 nm and about 4 nm in diameter, a conducting solvent, a processive DNA modifying enzyme, a plurality of dNTP molecules, and a metal ion co-factor.

In one embodiment, the system further comprises at least one species of ddNTP molecule. In another embodiment, the system further comprises an ammeter. In one preferred embodiment, the aperture diameter is about 2 nm. In another embodiment, the conducting solvent is an aqueous solvent. In an alternative embodiment the conducting solvent is a non-aqueous solvent. In another embodiment, the processive DNA modifying enzyme is a DNA polymerase. In another embodiment, the processive DNA modifying enzyme is tolerant to at least 0.6 M monovalent salt. In another embodiment, the concentration of the monovalent salt is at saturation. In another embodiment, the concentration of the monovalent salt is between 0.6 M and at saturation. In another embodiment, the processive DNA modifying enzyme is isolated from a mesophile or a virus naturally infecting a mesophile. In another embodiment, the processive DNA modifying enzyme is isolated from a halophile or a virus naturally infecting a halophile. In another embodiment, the processive DNA modifying enzyme is isolated from an extreme halophile or a virus naturally infecting an extreme halophile.

In another embodiment, the processive DNA modifying enzyme is selected from a bacterium from the group consisting of *Haloferax, Halogeometricum, Halococcus, Haloterrigena, Halorubrum, Haloarcula, Halobacterium, Salinivibrio costicola, Halomonas elongata, Halomonas israelensis, Salinibacter rube, Dunaliella salina, Staphylococcus aureus, Actinopolyspora halophila, Marinococcus halophilus,* and *S. costicola.* In another embodiment, the processive DNA modifying enzyme is selected from the group consisting of phi29 DNA polymerase, T7 DNA polymerase, His 1 DNA polymerase, and His 2 DNA polymerase, Bacillus phage M2 DNA polymerase, Streptococcus phage CP1 DNA polymerase, enterobacter phage PRD1 DNA polymerase, and variants threof.

In a preferred embodiment, the processive DNA modifying enzyme is phi29 DNA polymerase.

In another embodiment, the processive DNA modifying enzyme is from a moderate halophile, wherein the moderate halophile is selected from the group consisting of *Pseudomonas, Flavobacterium, Spirochaeta, Salinivibrio, Arhodomonas,* and *Dichotomicrobium.*

In another embodiment, the disclosure provides an apparatus for determining the nucleotide sequence of a polynucleotide in a sample, the apparatus comprising an electrical source, an anode, a cathode, a *cis* chamber, a *trans* chamber, wherein the *cis* and the *trans* chambers are separated by a thin film, the thin film having a plurality of apertures (pores), wherein each aperture (pore) is between about 0.25 nm and about 4 nm in diameter, a conducting solvent, a processive DNA modifying enzyme, a plurality of dNTP molecules, and a metal ion co-factor.

In one embodiment, the apparatus further comprises at least one species of ddNTP molecule. In another embodiment, the apparatus further comprises an ammeter. In one preferred embodiment, the aperture diameter is about 2 nm. In another embodiment, the conducting solvent is an aqueous solvent. In an alternative embodiment the conducting solvent is a non-aqueous solvent. In another embodiment, the processive DNA modifying enzyme is a DNA polymerase. In another embodiment, the processive DNA modifying enzyme is tolerant to at least 0.6 M monovalent salt. In another embodiment, the concentration of the monovalent salt is at saturation. In another embodiment, the concentration of the monovalent salt is between 0.6 M and at saturation. In another embodiment, the processive DNA modifying enzyme is isolated from a mesophile or a virus naturally infecting a mesophile. In another embodiment, the processive DNA modifying enzyme is isolated from a halophile or a virus naturally infecting a halophile. In another embodiment, the processive DNA modifying enzyme is isolated from an extreme halophile or a virus naturally infecting an extreme halophile.

In another embodiment, the processive DNA modifying enzyme is selected from a bacterium from the group consisting of *Haloferax, Halogeometricum, Halococcus, Haloterrigena, Halorubrum, Haloarcula, Halobacterium, Salinivibrio costicola, Halomonas elongata, Halomonas israelensis, Salinibacter rube, Dunaliella salina, Staphylococcus aureus, Actinopolyspora halophila, Marinococcus halophilus,* and *S. costicola.* In another embodiment, the processive DNA modifying enzyme is selected from the group consisting of phi29 DNA polymerase, T7 DNA polymerase, His 1 DNA polymerase, and His 2 DNA polymerase, Bacillus phage M2 DNA polymerase, Streptococcus phage CP1 DNA polymerase, enterobacter phage PRD1 DNA polymerase, and variants threof.

In a preferred embodiment, the processive DNA modifying enzyme is phi29 DNA polymerase.

In another embodiment, the processive DNA modifying enzyme is from a moderate halophile, wherein the moderate halophile is selected from the group consisting of *Pseudomonas, Flavobacterium, Spirochaeta, Salinivibrio, Arhodomonas,* and *Dichotomicrobium.*

In an yet other embodiment, the disclosure provides a device for determining the nucleotide sequence of a polynucleotide in a sample, the device comprising an electrical source, an anode, a cathode, a *cis* chamber, a *trans* chamber, wherein the *cis* and the *trans* chambers are separated by a thin film, the thin film having a plurality of apertures (pores), wherein each aperture (pore) is between about 0.25 nm and about 4 nm in diameter, a conducting solvent, a processive DNA modifying enzyme, a plurality of dNTP molecules, and a metal ion co-factor.

In one embodiment, the device further comprises at least one species of ddNTP molecule. In another embodiment, the device further comprises an ammeter. In one preferred embodiment, the aperture diameter is about 2 nm. In another embodiment, the conducting solvent is an aqueous solvent. In an alternative embodient the conducting solvent is a non-aqueous solvent. In another embodiment, the processive DNA modifying enzyme is a DNA polymerase. In another embodiment, the processive DNA modifying enzyme is tolerant to at least 0.6 M monovalent salt. In another embodiment, the concentration of the monovalent salt is at saturation. In another embodiment, the concentration of the monovalent salt is between 0.6 M and at saturation. In another embodiment, the processive DNA modifying enzyme is isolated from a mesophile or a virus naturally infecting a mesophile. In another embodiment, the processive DNA modifying enzyme is isolated from a halophile or a virus naturally infecting a halophile. In another embodiment, the processive DNA modifying enzyme is isolated from an extreme halophile or a virus naturally infecting an extreme halophile.

In another embodiment, the processive DNA modifying enzyme is selected from a bacterium from the group consisting of *Haloferax, Halogeometricum, Halococcus, Haloterrigena, Halorubrum, Haloarcula, Halobacterium, Salinivibrio costicola, Halomonas elongata, Halomonas israelensis, Salinibacter rube, Dunaliella salina, Staphylococcus aureus, Actinopolyspora halophila, Marinococcus halophilus,* and *S. costicola.* In another embodiment, the processive DNA modifying enzyme is selected from the group consisting of phi29 DNA polymerase, T7 DNA polymerase, His 1 DNA polymerase, and His 2 DNA polymerase, Bacillus phage M2 DNA polymerase, Streptococcus phage CP1 DNA polymerase, enterobacter phage PRD1 DNA polymerase, and variants threof.

In a preferred embodiment, the processive DNA modifying enzyme is phi29 DNA polymerase.

In another embodiment, the processive DNA modifying enzyme is from a moderate halophile, wherein the moderate halophile is selected from the group consisting of *Pseudomonas, Flavobacterium, Spirochaeta, Salinivibrio, Arhodomonas,* and *Dichotomicrobium.*

In another embodiment, the disclosure provides a method for determining the nucleotide sequence of a polynucleotide in a sample, the method comprising the steps of: providing two separate adjacent chambers comprising a liquid medium, an interface between the two chambers, the interface having an aperture (pore) so dimensioned as to allow sequential monomer-by-monomer passage from the cis-side of the channel to the *trans*-side of the channel of only one polynucleotide strand at a time; providing a processive DNA-modifying enzyme having binding activity for a polynucleotide; providing a polynucleotide in a sample, wherein a portion of the polynucleotide is double-stranded and a portion is single-stranded; introducing the polynucleotide into one of the two chambers; introducing the enzyme into the same chamber; allowing the processive DNA-modifying enzyme to bind to the polynucleotide; applying a potential difference between the two chambers, thereby creating a first polarity, the first polarity causing the single-stranded portion of the polynucleotide to transpose through the aperture (pore) to the *trans*-side; introducing the enzyme into the same chamber; allowing the enzyme to bind to the polynucleotide; measuring the electrical current through the channel thereby detecting a nucleotide base in the polynucleotide; decreasing the potential difference a first time; allowing the single-stranded portion of the polynucleotide to transpose through the aperture; measuring the change in electrical current; increasing the potential difference; measuring the electrical current through the channel, thereby detecting a particular nucleotide base positioned at the aperture (pore); repeating any one of the steps, thereby determining the nucleotide sequence of the polynucleotide. In one embodiment the method further comprises a step of adding at least one species of ddNTP molecule. In another embodiment the method further comprises wherein the aperture diameter is about 2 nm. In another embodiment the method further comprises wherein the liquid medium is an aqueous solvent. In another embodiment the method further comprises wherein the processive DNA modifying enzyme is a DNA polymerase. In another embodiment the method further comprises wherein the processive DNA modifying enzyme is tolerant to at least 0.6 M salt. In another embodiment, the concentration of the monovalent salt is at saturation. In another embodiment, the concentration of the monovalent salt is between 0.6 M and at saturation. In another embodiment the method further comprises wherein the processive DNA modifying enzyme is isolated from a mesophile or a virus naturally infecting a mesophile. In another embodiment the method further comprises wherein the processive DNA modifying enzyme is isolated from a mesophile, a halophile, or an extreme halophile or a virus naturally infecting a mesophile, a halophile, or an extreme halophile.

In another embodiment, the processive DNA modifying enzyme is selected from a bacterium from the group consisting of *Haloferax, Halogeometricum, Halococcus, Haloterrigena, Halorubrum, Haloarcula, Halobacterium, Salinivibrio costicola, Halomonas elongata, Halomonas israelensis, Salinibacter rube, Dunaliella salina, Staphylococcus aureus, Actinopolyspora halophila, Marinococcus halophilus,* and *S. costicola.* In another embodiment, the processive DNA modifying enzyme is selected from the group consisting of phi29 DNA polymerase, T7 DNA polymerase, His 1 DNA polymerase, and His 2 DNA polymerase, Bacillus phage M2 DNA polymerase, Streptococcus phage CP1 DNA polymerase, enterobacter phage PRD1 DNA polymerase, and variants threof.

In a preferred embodiment, the processive DNA modifying enzyme is phi29 DNA polymerase.

In another embodiment, the processive DNA modifying enzyme is from a moderate halophile, wherein the moderate halophile is selected from the group consisting of *Pseudomonas, Flavobacterium, Spirochaeta, Salinivibrio, Arhodomonas,* and *Dichotomicrobium.*

In another embodiment, the disclosure provides a method of sequencing a polynucleotide, the method comprising a step of including an oligomer, the oligomer comprising at least one abasic nucleotide species. In a preferred embodiment, the oligomer comprises more than one abaisc nucleotide. In a more preferrd embosdiment, the oligomer comprses at least five abasic nucleotides. In an alternative ebodiment, the method further comprises a step of including an oligomer comprising a C3 (CPG) spacer.

In another embodiment, the disclosure provides a method for sequencing a polynucleotide, the method further comprising a step of including a registry oligomer.

In another embodiment, the disclosure provides a method for sequencing a polynucleotide, the method further comprising a step of including a blocking oligomer. In a preferred embodiment, the blocking oligomer comprises at least 15 nucleotides. In another preferred embodiment, the blocking oligmer comprises at least 20 nucleotides. In another preferred embodiment, the blocking oligmer comprises at least 25 nucleotides. In another preferred embodiment, the blocking oligmer comprises at least 30 nucleotides. In another preferred embodiment, the blocking oligmer comprises at least 35 nucleotides. In another preferred embodiment, the blocking oligmer comprises at least 40 nucleotides. In another preferred embodiment, the blocking oligmer comprises at least 45 nucleotides. In another preferred embodiment, the blocking oligmer comprises at least 50 nucleotides. In an alterantive embodiment the blocking oligomer is selected from the group consisting of a 1 0-mer, a 15-mer, a 20-mer, a 25-mer, a 30-mer, a 31-mer, a 32-mer, a 33-mer, a 34-mer, a 35-mer, a 36-mer, a 37-mer, a 38-mer, a 39-mer, a 40-mer, a 50-mer, or any number of nucleotides therebetween. It may also be desirable to provide a blocking oligomer having more than 50 nucleotides.

In another embodiment the disclosure provides a method of sequencing a polyncleotide, wherein the polynucleotide has a size in the range of between 10 nucleotides to 50 thousand nucleotides. The number of nucleotides in the polyncuelotide can be 10, 15, 20, 25, 30, 35, 40, 45, 50, 75, 100, 150, 200, 250, 300, 350, 400, 450, 500, 750, 1,000, 1,500, 2,000, 2,500, 3,000, 3,500, 4,000, 4,500, 5,000, 10, 000, 15,000, 20, 000, 30, 000, 40,000, 50,000 or any number therebetween. It may also be desirable to sequence polynucleotides in excess of 50,000 nucleotides.

The disclosure provides thin film devices, systems, and methods for using the same. The subject devices or systems comprise *cis* and *trans* chambers connected by an electrical communication means. The *cis* and *trans* chambers are separated by a thin film comprising at least one pore or channel. In one preferred embodiment, the thin film comprises a compound having a hydrophobic domain and a hydrophilic domain. In a more preferred embodiment, the thin film comprises a phospholipid. The devices or systems further comprise a means for applying an electric field between the *cis* and the *trans* chambers. The pore or channel is shaped and sized having dimensions suitable for passaging a polymer. In one preferred embodiment the pore or channel accommodates a part but not all of the polymer. In one other preferred embodiment, the polymer is a polynucleotide. In an alternative preferred embodiment, the polymer is a polypeptide. Other polymers described herein include polypeptides, phospholipids, polysaccharides, and polyketides.

In one embodiment, the thin film further comprises a compound having a binding affinity for the polymer. In one preferred embodiment the binding affinity (Kₐ) is at least 10⁶ l/mole. In a more preferred embodiment the Kₐ is at least 10⁸ l/mole. In yet another preferred embodiment the compound is adjacent to at least one pore. In a more preferred embodiment the compound is a channel. In a yet more preferred embodiment the channel has biological activity. In a most preferred embodiment, the compound comprises the pore.

In another embodiment the pore is sized and shaped to allow passage of an activator, wherein the activator is selected from the group consisting of ATP, NAD⁺, NADP⁺, diacylglycerol, phosphatidylserine, eicosinoids, retinoic acid, calciferol, ascorbic acid, neuropeptides, enkephalins, endorphins, 4-aminobutyrate (GABA), 5-hydroxytryptamine (5-HT), catecholamines, acetyl CoA, S-adenosylmethionine, and any other biological activator.

In yet another embodiment the pore is sized and shaped to allow passage of a cofactor, wherein the cofactor is selected from the group consisting of Mg²⁺, Mn²⁺, Ca²⁺, ATP, NAD⁺, NADP⁺, and any other biological cofactor.

In a preferred embodiment the pore or channel is a pore molecule or a channel molecule and comprises a biological molecule, or a synthetic modified molecule, or altered biological molecule, or a combination thereof. Such biological molecules are, for example, but not limited to, an ion channel, a nucleoside channel, a peptide channel, a sugar transporter, a synaptic channel, a transmembrane receptor, such as GPCRs and the like, a nuclear pore, synthetic variants, chimeric variants, or the like. In one preferred embodiment the biological molecule is α-hemolysin.

In an alternative, the compound comprises non-enzyme biological activity. The compound having non-enzyme biological activity can be, for example, but not limited to, proteins, peptides, antibodies, antigens, nucleic acids, peptide nucleic acids (PNAs), locked nucleic acids (LNAs), morpholinos, sugars, lipids, glycophosphoinositols, lipopolysaccharides or the like. The compound can have antigenic activity. The compound can have selective binding properties whereby the polymer binds to the compound under a particular controlled environmental condition, but not when the environmental conditions are changed. Such conditions can be, for example, but not limited to, change in [H⁺], change in environmental temperature, change in stringency, change in hydrophobicity, change in hydrophilicity, or the like.

In another embodiment, the disclosure provides a compound, wherein the compound further comprises a linker molecule, the linker molecule selected from the group consisting of a thiol group, a sulfide group, a phosphate group, a sulfate group, a cyano group, a piperidine group, an Fmoc group, and a Boc group.

In one embodiment the thin film comprises a plurality of pores. In one embodiment the device comprises a plurality of electrodes.

### Polynucleotides

In another embodiment, the disclosure provides a method for controlling binding of an enzyme to a partially double-stranded polynucleotide complex, the method comprising: providing two separate, adjacent pools of a medium and an interface between the two pools, the interface having a channel so dimensioned as to allow sequential monomer-by-monomer passage from one pool to the other pool of only one polynucleotide at a time; providing an enzyme having binding activity to a partially double-stranded polynucleotide complex; providing a polynucleotide complex comprising a first polynucleotide and a second polynucleotide, wherein a portion of the polynucleotide complex is double-stranded, and wherein the first polynucleotide further comprises a moiety that is incompatible with the second polynucleotide; introducing the polynucleotide complex into one of the two pools; introducing the enzyme into one of the two pools; applying a potential difference between the two pools, thereby creating a first polarity; reversing the potential difference a first time, thereby creating a second polarity; reversing the potential difference a second time to create the first polarity, thereby controlling the binding of the enzyme to the partially double-stranded polynucleotide complex. In a preferred embodiment, the medium is electrically conductive. In a more preferred embodiment, the medium is an aqueous solution. In a preferred embodiment, the moiety is selected from the group consisting of a peptide nucleic acid, a 2'-O-methyl group, a fluorescent compound, a derivatized nucleotide, and a nucleotide isomer. In another preferred embodiment, the method further comprises the steps of measuring the electrical current between the two pools; comparing the electrical current value obtained at the first time the first polarity was induced with the electrical current value obtained at the time the second time the first polarity was induced. In another preferred embodiment the method further comprises the steps of measuring the electrical current between the two pools; comparing the electrical current value obtained at the first time the first polarity was induced with the electrical current value obtained at a later time. In a more preferred embodiment, the enzyme is selected from the group consisting of DNA polymerase, RNA polymerase, endonuclease, exonuclease, DNA ligase, DNase, uracil-DNA glycosidase, kinase, phosphatase, methylase, and acetylase. In another alternative embodiment, the method further comprises the steps of providing at least one reagent that initiates enzyme activity; introducing the reagent to the pool comprising the polynucleotide complex; and incubating the pool at a suitable temperature. In a more preferred embodiment, the reagent is selected from the group consisting of a deoxyribonucleotide and a cofactor. In a yet more preferred embodiment, the deoxyribonucleotide is introduced into the pool prior to introducing the cofactor. In another still more preferred embodiment, the cofactor is selected from the group consisting of Mg2+, Mn2+, Ca2+, ATP, NAD+, and NADP+. In one embodiment the enzyme is introduced into the same pool as the polynucleotide. In an alternative embodiment, the enzyme is introduced into the opposite pool.

Disclosed herein are devices and methods that can regulate the rate at which an individual polymer in a mixture is acted upon by another compound, for example, an enzyme. The devices and methods are also used to determine the nucleotide base sequence of a polynucleotide The disclosure is of particular use in the fields of molecular biology, structural biology, cell biology, molecular switches, molecular circuits, and molecular computational devices, and the manufacture thereof.

In one embodiment the nanopore system can control binding of a molecule to a polymer at a rate of between about 5 Hz and 2000 Hz. The nanopore system can control binding of a molecule to a polymer at, for example, about 5 Hz, at about 10 Hz, at about 15 Hz, at about 20 Hz, at about 25 Hz, at about 30 Hz, at about 35 Hz, at about 40 Hz, at about 45 Hz, at about 50 Hz, at about 55 Hz, at about 60 Hz, at about 65 Hz, at about 70 Hz, at about 75 Hz, at about 80 Hz, at about 85 Hz, at about 90 Hz, at about 95 Hz, at about 100 Hz, at about 110 Hz, at about 120 Hz, at about 125 Hz, at about 130 Hz, at about 140 Hz, at about 150 Hz, at about 160 Hz, at about 170 Hz, at about 175 Hz, at about 180 Hz, at about 190 Hz, at about 200 Hz, at about 250 Hz, at about 300 Hz, at about 350 Hz, at about 400 Hz, at about 450 Hz, at about 500 Hz, at about 550 Hz, at about 600 Hz, at about 700 Hz, at about 750 Hz, at about 800 Hz, at about 850 Hz, at about 900 Hz, at about 950 Hz, at about 1000 Hz, at about 1125 Hz, at about 1150 Hz, at about 1175 Hz, at about 1200 Hz, at about 1250 Hz, at about 1300 Hz, at about 1350 Hz, at about 1400 Hz, at about 1450 Hz, at about 1500 Hz, at about 1550 Hz, at about 1600 Hz, at about 1700 Hz, at about 1750 Hz, at about 1800 Hz, at about 1850 Hz, at about 1900 Hz, at about 950 Hz, and at about 2000 Hz. In a preferred embodiment, the nanopore system can control binding of a molecule to a polymer at a rate of between about 25 Hz and about 250 Hz. In a more preferred embodiment the nanopore system can control binding of a molecule to a polymer at a rate of between about 45 Hz and about 120 Hz. In a most preferred embodiment the nanopore system can control binding of a molecule to a polymer at a rate of about 50 Hz.

The disclosure also provides thin film devices and methods for using the same. The subject devices comprise *cis* and *trans* chambers connected by an electrical communication means. The *cis* and *trans* chambers are separated by a thin film comprising at least one pore or channel. In one preferred embodiment, the thin film comprises a first compound having a hydrophobic domain and a hydrophilic domain. In a more preferred embodiment, the thin film comprises a phospholipid. The devices further comprise a means for applying an electric field between the *cis* and the *trans* chambers. The pore or channel is shaped and sized having dimensions suitable for passaging a polymer. In one preferred embodiment the pore or channel accommodates a part but not all of the polymer. In another preferred embodiment the pore or channel accommodates a monomer part of the polymer but not a dimer part of the polymer. In one other preferred embodiment, the polymer is a polynucleotide. In an alternative preferred embodiment, the polymer is a polypeptide. Other polymers disclosed herein include polypeptides, phospholipids, polysaccharides, and polyketides.

In one embodiment, the thin film further comprises a second compound having a binding affinity for the polymer. In one preferred embodiment the binding affinity (Kₐ) is at least 10⁶ l/mole. In a more preferred embodiment the Kₐ is at least 10⁸ l/mole. In yet another preferred embodiment the compound is adjacent to at least one pore. In a more preferred embodiment the compound is a channel. In a yet more preferred embodiment the channel has biological activity. In a most preferred embodiment, the compound comprises the pore.

In one embodiment the second compound comprises enzyme activity. The enzyme activity can be, for example, but not limited to, enzyme activity of proteases, kinases, phosphatases, hydrolases, oxidoreductases, isomerases, transferases, methylases, acetylases, ligases, lyases, and the like. In a more preferred embodiment the enzyme activity can be enzyme activity of DNA polymerase, RNA polymerase, endonuclease, exonuclease, DNA ligase, DNase, uracil-DNA glycosidase, kinase, phosphatase, methylase, acetylase, or the like.

In one preferred embodiment, the DNA polymerase is isolated from a halophile microorganism. In an alternative preferred embodiment, the DNA polymerase is a naturally-occurring variant of the DNA polymerase isolated from a halophile microorganism. In an alternative preferred embodiment, the DNA polymerase is a synthetic variant of the DNA polymerase isolated from a halophile microorganism. In yet another alternative preferred embodiment, the DNA polymerase is a synthetic composition having the enzyme properties of the DNA polymerase isolated from a halophile microorganism or alternatively, a naturally-occurring variant of the DNA polymerase isolated from a halophile microorganism. In a more preferred embodiment, the halofile microorganism is an extreme halophile microorganism. In another more preferred embodiment the halophile microorganism is a moderate halophile microorganism.

In another preferred embodiment, the halophile microorganism thrives under environmental conditions selected from the group consisting of temperature equal to or greater than 50° C, pressure equal to or greater that 200 kPa, pH equal to or lower than 6.5, pH equal to or greater than 7.5, and salinity equal to or greater than 0.5M. For example, the temperature can be 50° C, 55° C, 60° C, 65° C, 70° C, 75° C, 80° C, 85° C, 90° C, 95° C, and 99° C. In another example, the pH can be 2.5, 3.0, 3.5, 4.0, 4.5, 5.0, 5.5, 6.0, 6.5, 7.5, 8.0,8.5, 9.0, and 9.5.

In an alternative preferred embodiment, the DNA polymerase is isolated from a virus that can infect a halophile microorganism. In an alternative preferred embodiment, the DNA polymerase is a naturally-occurring variant of the DNA polymerase isolated from a virus that can infect a halophile microorganism. In an alternative preferred embodiment, the DNA polymerase is a synthetic variant of the DNA polymerase isolated from a virus that can infect a halophile microorganism. In yet another alternative preferred embodiment, the DNA polymerase is a synthetic composition having the enzyme properties of the DNA polymerase isolated from a virus that can infect a halophile microorganism or alternatively, a naturally-occurring variant of the DNA polymerase isolated from a virus that can infect a halophile microorganism. In a more preferred embodiment, the halofile microorganism is an extreme halophile microorganism. In an alternative embodiment, the virus that can infect a halophile microorganism is infective under environmental conditions selected from the group consisting of temperature equal to or greater than 50° C, pressure equal to or greater that 200 kPa, pH equal to or lower than 6.5, pH equal to or greater than 7.5, and salinity equal to or greater than 0.5M. For example, the temperature can be 50° C, 55° C, 60° C, 65° C, 70° C, 75° C, 80° C, 85° C, 90° C, 95° C, and 99° C. In another example, the pH can be 2.5, 3.0, 3.5, 4.0, 4.5, 5.0, 5.5, 6.0, 6.5, 7.5, 8.0, 8.5, 9.0, and 9.5.

The second compound can have selective binding properties whereby the polymer binds to the second compound under a particular controlled environmental condition, but not when the environmental conditions are changed. Such conditions can be, for example, but not limited to, change in [H⁺], change in environmental temperature, change in stringency, change in hydrophobicity, change in hydrophilicity, or the like.

In another embodiment the pore is sized and shaped to allow passage of an activator, wherein the activator is selected from the group consisting of ATP, NAD⁺, NADP⁺, diacylglycerol, phosphatidylserine, eicosinoids, retinoic acid, calciferol, ascorbic acid, neuropeptides, enkephalins, endorphins, 4-aminobutyrate (GABA), 5-hydroxytryptamine (5-HT), catecholamines, acetyl CoA, S-adenosylmethionine, and any other biological activator.

In yet another embodiment the pore is sized and shaped to allow passage of a cofactor, wherein the cofactor is selected from the group consisting of Mg²⁺, Mn²⁺, Ca²⁺, ATP, NAD⁺, NADP⁺, and any other biological cofactor.

In a preferred embodiment the pore or channel comprises a biological molecule, or a synthetic modified or altered biological molecule. Such biological molecules are, for example, but not limited to, an ion channel, a nucleoside channel, a peptide channel, a sugar transporter, a synaptic channel, a transmembrane receptor, such as GPCRs and the like, a nuclear pore, or the like.

In an alternative, the second compound comprises non-enzyme biological activity. The second compound having non-enzyme biological activity can be, for example, but not limited to, proteins, peptides, antibodies, antigens, nucleic acids, peptide nucleic acids (PNAs), locked nucleic acids (LNAs), morpholinos, sugars, lipids, glycophosphoinositols, lipopolysaccharides or the like.

In another embodiment, the disclosure provides a third compound, wherein the third compound further comprises a linker molecule, the linker molecule selected from the group consisting of a thiol group, a sulfide group, a phosphate group, a sulfate group, a cyano group, a piperidine group, an Fmoc group, and a Boc group.

In one embodiment the thin film comprises a plurality of pores. In one embodiment the device comprises a plurality of electrodes.

The disclosure also contemplates a method of binding phi29 DNA polymerase (DNAP) to single-stranded DNA (ss-DNA) and thereby reducing the rate at which the ss-DNA traverses an α-Hemolysin nanopore under a 180 mV applied potential. In a preferred ebodiment, single-stranded DNA threads through the phi29 DNAP and α-Hemolysin nanopore at a rate near one nucleotide per 1-100 ms. In another embodiment, the rate is from between one nucleotide per 100-1000 ms.

The disclosure also contemplates a method of using the primer DNA 5' terminus to protect the template 3' terminus from digestion by DNA polymerases (DNAP).

The disclosure also contemplates a method of covalently bonding a C3 (CPG) spacer, followed by an abasic residue on the 3'-terminus and preventing exonucleolytic digestion of the DNA.

The disclosure also contemplates a method of protecting the primer DNA strand from phi29 DNAP function by binding a modified DNA oligomer adjacent to the primer template junction. In a preferred embodiment, phi29 binds at the oligomer 5'-terminus and capture of this complex on an α-Hemolysin nanopore with 180 mV applied potential removes the oligomer and places phi29 at the primer terminus, after which DMA replication can take place.

The disclosure also contemplates a method of using a registry oligomer, preferrably a modified DNA oligomer, to control where phi29 DNAP binds and sits on the ss-DNA. Capture of these DNAP-DNA complexes on an α-Hemolysin nanopore using a 180 mV applied potential removes the oligomer and allows the s-DNA to translocate through phi29 DNAP and the α-Hemolysin.

The disclosure also contemplates a method wherein phi29 DNAP-bound dsDNA unzips in a nanopore by applied voltage (180 mV). In a preferred embodiment, voltage reduction allows re-zipping of the DNA. Restoring the voltage unzips the DNA again and this allows movement of the DNA back and forth through the nanopore.

The disclosure also contemplates using a blocking oligomer binding at the DNA primer/transcript junction whereby the oligomer is stripped off when captured on a nanopore, and the DNA is subsequently activated for ratcheting through the nanopore.

The disclosure also contemplates using shorter blocking oligomers and decreasing the time required to strip the blocking oligomer off the DNA. In a preferred embodiment, this allows activation of DNA molecules for replication on the nanopore faster, and that this increases the throughput of the nanopore for sequencing applications.

The disclosure also contemplates a method of sewquencing a polynucleotide, the method comprising a step of determining the noise level in a signal, the noise level being representative of the identity of the nucleotide inducing the signal compared with the previous nucleotide inducing a signal and the subsequent nucleotide inducing a signal. In a preferred embodiment, the signal is a change in current measured between the two adjacent pools. In a more preferred embodiment, the noise level measured is greater for a nucleotide when the previous nucleotide and/or the subsequent nucleotide are a different nucleotide.

The disclosure also contemplates a method of sequecing a polynculeotide, the method conpridsing the step of including a dNTP at lower concentration that other dNTPs thereby reducing the rate of reaction of the DNAP. In one embodment, the dNTP is at about one order of magnitude lower in concentration that the other dNTPs. In a more preferred embodiment, the dNTP is at about two orders of magnitude lower in concentration that the other dNTPs.

### Brief Description of the Drawings

Figure 1 illustrates an embodiment of the disclosure whereby binary complexes between phi29 DNA polymerase (phi29 DNAP) and DNA can be retained on a nanopore almost indefinitely. Figure 1 also illustrates an embodiment of the disclosure whereby DNA duplexes can be systematically unzipped by an applied voltage across a nanopore.

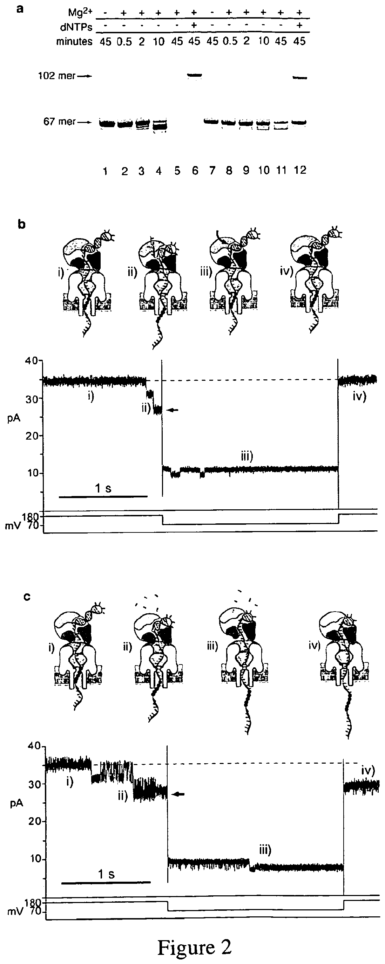

Figure 2 illustrates an embodiment of the disclosure whereby DNA duplexes bound to phi29 DNAP can be systematically unzipped by an applied voltage across a nanopore and then be re-annealed at lower potential difference. Figure 2 also illustrates an embodiment of the disclosure showing how the processive exonuclease of wild type phi29 DNAP can systematically ratchet DNA through a nanopore; the process may be controlled by dNTP concentration and by an applied voltage across the nanopore. Individual DNA templates may be moved back and forth across a nanopore by the polymerase domain and the exonuclease domain of a single bound phi29 DNAP; the competing motions may be regulated by voltage and dNTP concentration.

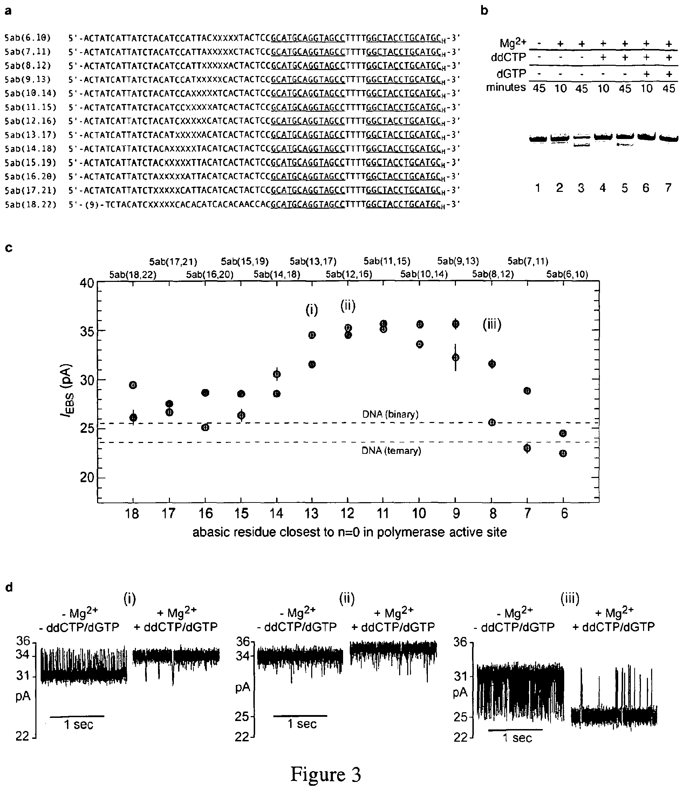

Figure 3 illustrates and embodiment of the disclosure whereby binding and dissociation of correct dNTPs in the polymerase catalytic site of phi29 DNAP may be measured by ionic current across a nanopore; the activity may be dependent upon the concentration of the correct dNTP in a buffer bathing the nanopore. In addition, the drawings show that ionic flicker can predict the ionic current caused by monomers immediately proximal to a given monomer on the DNA strand; this process may be dependent upon concentrations of the complementary dNTP to the templating nucleotide in the phi29 DNAP polymerase site. Figure 3 also illustrates how DNA template/primer pairs may be maintained at a fixed ssDNA/dsDNA junction in bulk phase using a ddNMP (3'-H) terminated primer strand along with the dNTP complementary to the templating base in the polymerase catalytic site of phi29 DNAP; protection of the ssDNA/dsDNA junction may be potentiated by including the ddNMP (3'-H) that is analogous to the ddNMP (3'-H) terminus.
Figure 4 illustrates how activation of the DNA for replication (as shown in Figure 3) can be potentiated by the nanopore voltage that causes excision of the ddNMP (3'-H) terminus.
Figure 5 illustrates an embodiment of the disclosure showing how processivity of the DNA replication may be influenced by dNTPs in solution causing pauses and distinct signature currents dependent upon their presence or concentration.
Figure 6 illustrates an embodiment of the disclosure showing that phi29 DNAP may replicate a DNA template against an applied voltage of at least 300 mV across a nanopore; the rate of DNA replication may be modulated by the applied voltage.
Figure 7 illustrates an embodiment of the disclosure showing that the length (number of bases) of the DNA template that may be transposed through the nanopore by phi29 DNAP is dependent upon the length of the DNA captured by the nanopore. The drawing also shows that phi29 DNAP can replicate DNA on the nanopore at 0.6 M KCl.

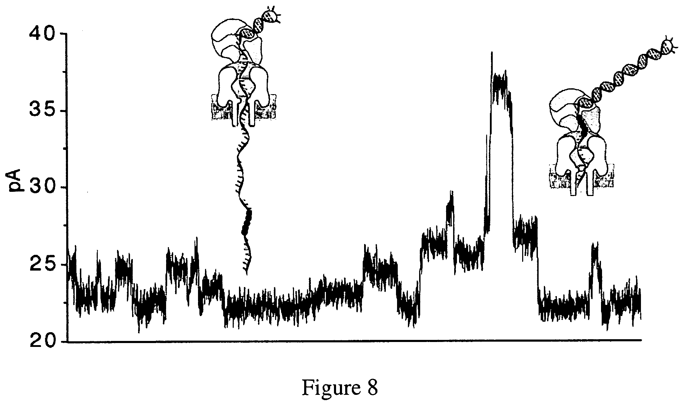

Figure 8 illustrates the same data as Figure 7b also showing the status of the polynucleotide and the DNAP at different stages during the passage of polynucleotide throught a nanopore. The abasic residues are shown in red.
Figure 9 illustrates an exemplary embodiment of the disclosure illustrating that noise (as current measured) can indicate prior and subsequent nucleotide monomer identity; the amplitude of the noise observed from an intermediary nucleotide on a first strand differs when the prior and subsequent nucleotides differ from those observed from a second orthologous strand when the intermediary nucleotide is identical to that of the first strand.
Figure 10 illustrates that binding phi29 DNA polymerase (DNAP) to single-stranded DNA (ss-DNA) dramatically reduces the rate at which the ss-DNA traverses an α-Hemolysin nanopore under a 180 mV applied potential.
Figure 11 illustrates that the primer DNA strand may be protected from phi29 DNAP function by binding a modified DNA oligomer adjacent to the primer template junction; phi29 binds at the oligomer 5'-terminus and capture of this complex on an α-Hemolysin nanopore with 180 mV applied potential removes the oligomer and places phi29 at the primer terminus, after which DMA replication can take place.
Figure 12 illustrates that phi29 DNAP can bind and move along ss-DNA; a registry oligomer - a modified DNA oligomer - is used to control where phi29 DNAP binds and sits on the ss-DNA.
Figure 13 illustrates that DNA polymerase enzymes with a 3' -5' exonuclease can digest the 3' terminus of template DNA; the method uses the primer DNA 5' terminus to protect the template 3' terminus from digestion by DNA polymerases (DNAP).
Figure 14 illustrates how the rate of synthesis of the DNA template can be controlled by using dilute dNTPs.
Figures 15 to 19 illustrate experimental resulults showing voltage-activated forward and reverse ratcheting of DNA in a nanopore.

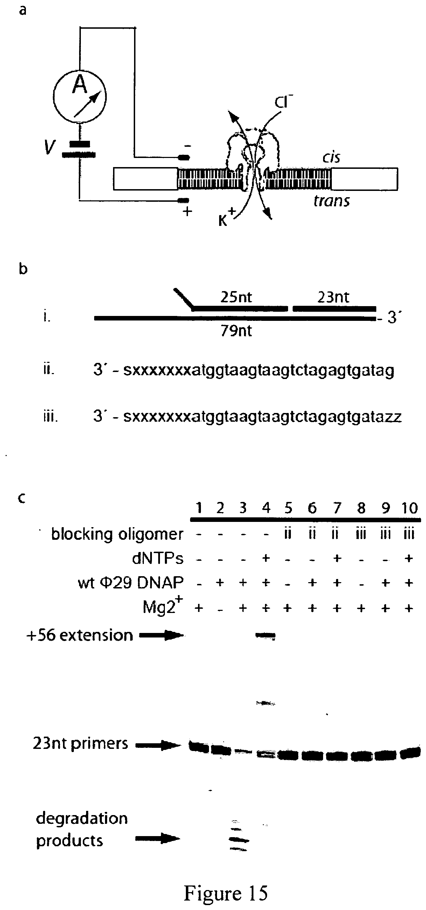

Figure 15. Components of the nanopore device, **a)** nanopore device. A single alpha-HL nanopore is inserted in a lipid bilayer that separates two wells, each containing 100 µl of a buffered KCl solution. Negatively charged single-stranded DNA (ssDNA) is added to the cis well. A voltage applied across the wells (trans side +) drives ionic current through the pore (for example, 60 pA at 0.3 M KCL, 180 mV), and causes the ssDNA to enter and translocate through the nanopore. **b)** Schematic of P/t DNA protected from phi29 DNAP-directed digestion and extension. The primer DNA strand needed to be protected against synthesis and digestion in bulk phase, but activated for synthesis on the nanopore. This was achieved by annealing modified blocking oligomers at the p/t junction, (i) Shows a p/t (23nt/79nt) substrate with a 25nt blocking oligomer binding site (bent red line). Blocking oligomers contain a 3'-C3 spacer (S) followed by six abasic residues (Xs) to protect against degradation and facilitate removal on the nanopore. Version iii contains two 5'-acridine residues (Zs). **c)** Protection of p/t DNA in bulk phase using blocking oligomers. The DNA p/t substrate in b.i) absent or present blocking oligomer ii or iii was incubated with phi29 DNAP, dNTPs, and Mg2+ where indicated. Absent blocking oligomer, Phi29 DNAP digested (-dNTPs, lane 3) and extended (+dNTPs, lane 4) the primer. Present blocking oligomer (i) or (ii), the primer strand was protected from digestion (-dNTPs, lanes 6, 9) and extension (+dNTPs, lanes 7, 10).

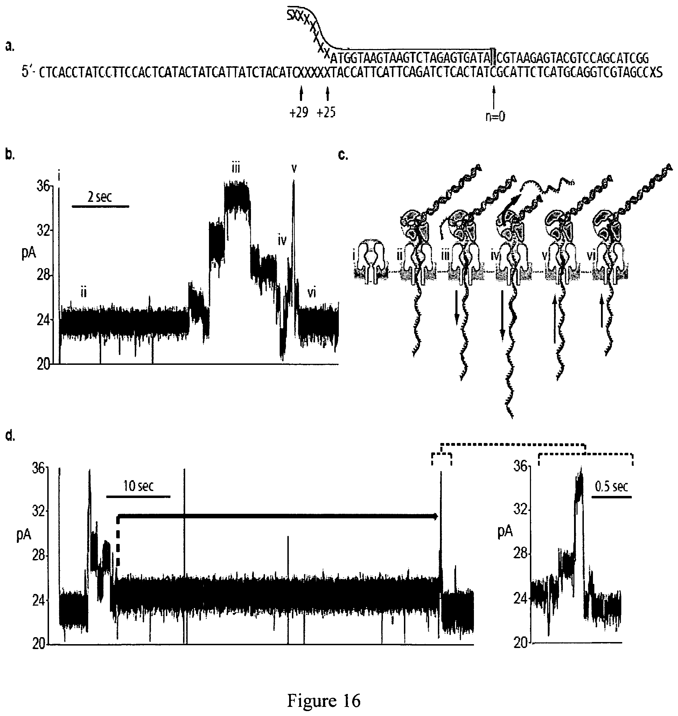

Figure 16. Forward and reverse ratcheting of DNA through the nanopore. **(a)** P/t DNA for ratcheting through the nanopore. Blocking oligomer iii from Figure 16b (red line) protects the primer from catalysis in bulk phase. Five abasic residues (Xs) at positions 25-to-29 on the template cause a peak in current as they traverse the nanopore (cite). **(b,c)** Forward and reverse ratcheting of DNA through the nanopore. The p/t DNA in (a) pre-loaded with phi29 DNAP is captured on the nanopore by an applied voltage (i). Capture initially places the abasic insert (red circles) above the nanopore. The applied voltage forces non-catalytic unzipping of the blocking oligomer/template duplex, which causes a 35pA peak in the current (ii) as the abasic insert ratchets forward through the nanopore. (iii) Further unzipping removes the blocking oligomer and places phi29 DNAP at the primer/template junction. In the presence of dNTPs and Mg2+, phi29 DNAP then processively replicates 25 DNA bases, causing the abasic insert to ratchet in reverse through the nanopore and a retrace of the 35pA current peak (iv). Replication stalls when the abasic insert reaches the polymerase active site (v). **(d)** Reverse DNA ratcheting is replication-dependent. Substitution of the primer 3'-deoxycytosine in a) for a 2',3'-dideoxycytosine delays the appearance of the second peak (red arrow). Phi29 DNAP eventually excises the terminal dideoxycytosine, which initiates DNA synthesis and causes traversal of the second 35pA peak.

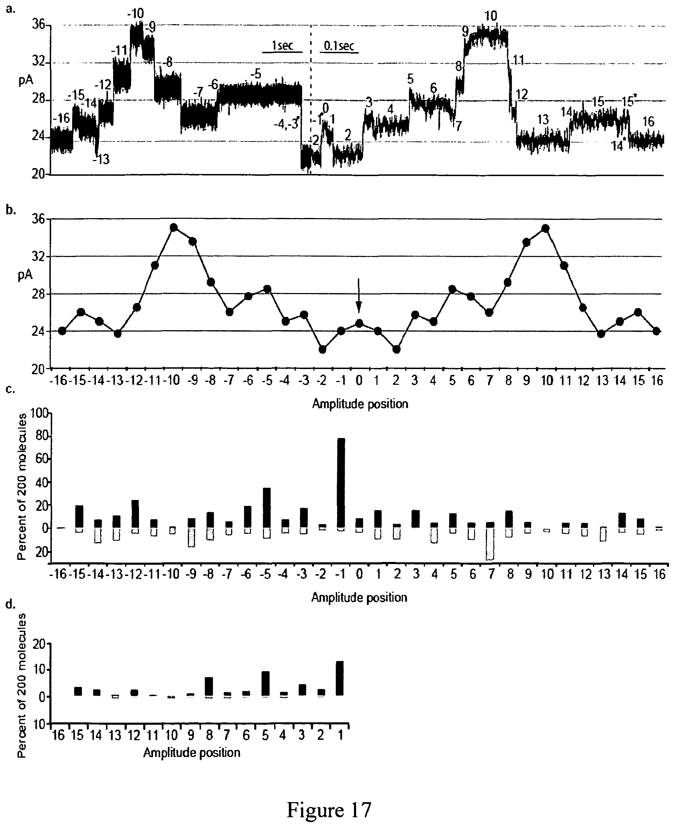

Figure 17.Analysis of the ionic current signal reporting forward and reverse DNA ratcheting. **(a)** Ionic current trace showing the discrete amplitudes detected from forward/reverse ratcheting a single molecule through the nanopore. A total of 33 discrete amplitudes were detected that were symmetric about a common 25pA amplitude (position 0). Amplitudes were randomly skipped (for example, positions -4 and -3) or repeated (for example, positions 14 and 15) from molecule to molecule, **(b)** Reference map of the current amplitudes shown in (a). **(c)** Percent of the time each amplitude position in (b) was skipped (black upward bars) or repeated (grey downward bars) for 200 molecules processed in a single 5 hr experiment, **(d)** Percent of the time both corresponding amplitudes (for example, positions -15 and 15) were skipped (black upward bars) or repeated (grey downward bars) for the molecules analyzed in (c).

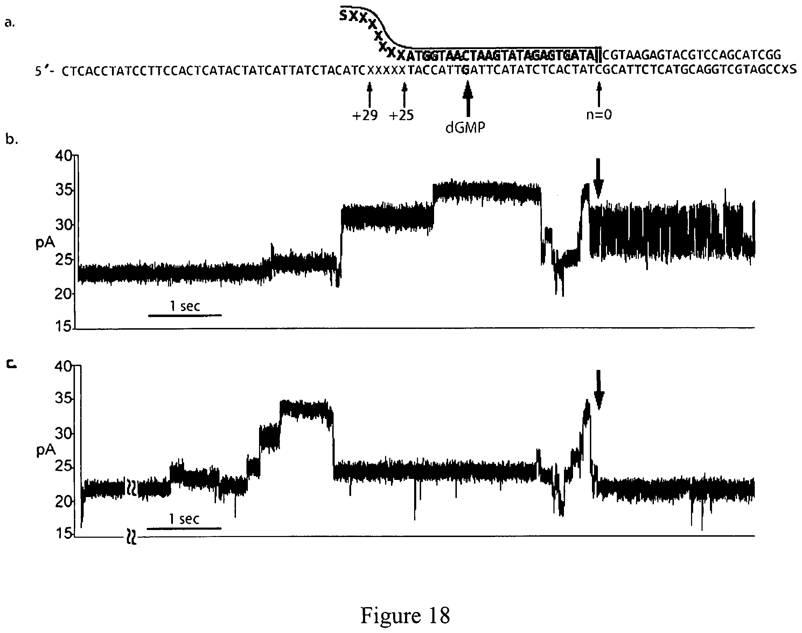

Figure 18. DNA replication dependence on key dNTP substrates, **(a)** P/t DNA for ratcheting through the nanopore. A single deoxyguanosine (red arrow) is at position +16 between the p/t junction and abasic insert. Absent dCTP, DNA synthesis will stall at the dGTP and place the abasic residues in positions +8 to +12 in the nanopore, which will yield a bifurcation in the ionic current between 25pA and 31pA (JACS) **(b)** Forward/reverse ratcheting of the DNA construct in (a) through the nanopore, absent dCTP. After the first 35pA is traversed, the ionic current stalls at the second peak and bifurcates between 25pA and 31pA. **(c)** Forward/reverse ratcheting of the DNA construct in (a) through the nanopore, present dCTP. After the first 35pA peak is traversed, the ionic current proceeds through the second 35pA peak without stalling. These data support our claim that the second 35pA peak is dependent on enzyme-directed DNA replication.

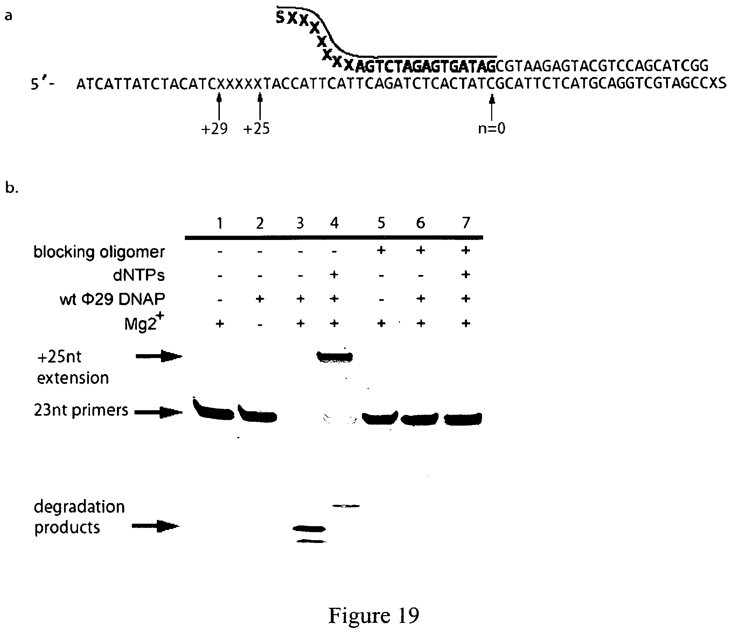

Figure 19. Blocking oligomer optimized for increased throughput on the nanopore. **(a)** optimized blocking oligomer (red line) bound to p/t DNA substrate. The complementary sequence to the template strand was shorted from 25nt to 15nt to facilitate faster removal of the blocking oligomer on the nanopore. **(b)** Protection of p/t DNA in bulk phase using the optimized blocking oligomer in (a). The p/t DNA substrate in a) absent or present blocking oligomer was incubated with phi29 DNAP, dNTPs, and Mg2+ where indicated. Absent blocking oligomer, Phi29 DNAP digested (-dNTPs, lane 3) and extended (+dNTPs, lane 4) the primer. Present blocking oligomer, the primer strand was protected from digestion (-dNTPs, lanes 6) and extension (+dNTPs, lanes 7). Reactions were run for 5 hr in nanopore buffer at 23°C.

### Description of the Sequence Listing

SEQ ID NO: 1 shows the polynucleotide sequence encoding one subunit of α-hemolysin-E111N/K147N (α-HL-NN; Stoddart et al., PNAS, 2009; 106(19): 7702-7707).
SEQ ID NO: 2 shows the amino acid sequence of one subunit of α-HL-NN.
SEQ ID NO: 3 shows the codon optimised polynucleotide sequence encoding the Phi29 DNA polymerase.
SEQ ID NO: 4 shows the amino acid sequence of the Phi29 DNA polymerase.
SEQ ID NOs.: 5 to 28 are the synthetic polynucleotide sequences (templates, oligomers, and blocking oligomers) used in the Examples.

### Detailed Description of the Invention

The embodiments disclosed in this document are illustrative and exemplary and are not meant to limit the invention. Other embodiments can be utilized and structural changes can be made without departing from the scope of the claims of the present invention.

As used herein and in the appended claims, the singular forms "a," "an," and "the" include plural reference unless the context clearly dictates otherwise. Thus, for example, a reference to "a nanopore" includes a plurality of such nanopores, and a reference to "a signal" is a reference to one or more signals and equivalents thereof, and so forth.

By "polynucleotide" is meant DNA or RNA, including any naturally occurring, synthetic, or modified nucleotide. Nucleotides include, but are not limited to, ATP, dATP, CTP, dCTP, GTP, dGTP, UTP, TTP, dUTP, 5-methyl-CTP, 5-methyl-dCTP, ITP, dITP, 2-amino-adenosine-TP, 2-amino-deoxyadenosine-TP, 2-thiothymidine triphosphate, pyrrolo-pyrimidine triphosphate, 2-thiocytidine as well as the alphathiotriphosphates for all of the above, and 2'-O-methyl-ribonucleotide triphosphates for all the above bases. Modified bases include, but are not limited to, 5-Br-UTP, 5-Br-dUTP, 5-F-UTP, 5-F-dUTP, 5-propynyl dCTP, and 5-propynyl-dUTP.

By "transport property" is meant a property measurable during polymer movement with respect to a nanopore. The transport property may be, for example, a function of the solvent, the polymer, a label on the polymer, other solutes (for example, ions), or an interaction between the nanopore and the solvent or polymer.

A "hairpin structure" is defined as an oligonucleotide having a nucleotide sequence that is about 6 to about 10,000 nucleotides in length, the first half of which nucleotide sequence is at least partially complementary to the second part thereof, thereby causing the polynucleotide to fold onto itself, forming a secondary hairpin structure.

"Identity" or "similarity" refers to sequence similarity between two polynucleotide sequences or between two polypeptide sequences, with identity being a more strict comparison. The phrases "percent identity" and "% identity" refer to the percentage of sequence similarity found in a comparison of two or more polynucleotide sequences or two or more polypeptide sequences. "Sequence similarity" refers to the percent similarity in base pair sequence (as determined by any suitable method) between two or more polynucleotide sequences. Two or more sequences can be anywhere from 0-100% similar, or any integer value therebetween. Identity or similarity can be determined by comparing a position in each sequence that may be aligned for purposes of comparison. When a position in the compared sequence is occupied by the same nucleotide base or amino acid, then the molecules are identical at that position. A degree of similarity or identity between polynucleotide sequences is a function of the number of identical or matching nucleotides at positions shared by the polynucleotide sequences. A degree of identity of polypeptide sequences is a function of the number of identical amino acids at positions shared by the polypeptide sequences. A degree of homology or similarity of polypeptide sequences is a function of the number of amino acids at positions shared by the polypeptide sequences.

The term "incompatible" refers to the chemical property of a molecule whereby two molecules or portions thereof cannot interact with one another, physically, chemically, or both. For example, a portion of a polymer comprising nucleotides can be incompatible with a portion of a polymer comprising nucleotides and another chemical moiety, such as for example, a peptide nucleic acid, a 2'-O-methyl group, a fluorescent compound, a derivatized nucleotide, a nucleotide isomer, or the like. In another example, a portion of a polymer comprising amino acid residues can be incompatible with a portion of a polymer comprising amino acid residues and another chemical moiety, such as, for example, a sulfate group, a phosphate group, an acetyl group, a cyano group, a piperidine group, a fluorescent group, a sialic acid group, a mannose group, or the like.

"Alignment" refers to a number of DNA or amino acid sequences aligned by lengthwise comparison so that components in common (such as nucleotide bases or amino acid residues) may be visually and readily identified. The fraction or percentage of components in common is related to the homology or identity between the sequences. Alignments may be used to identify conserved domains and relatedness within these domains. An alignment may suitably be determined by means of computer programs known in the art, such as MACVECTOR software (1999) (Accelrys, Inc., San Diego, CA).

The terms "highly stringent" or "highly stringent condition" refer to conditions that permit hybridization of DNA strands whose sequences are highly complementary, wherein these same conditions exclude hybridization of significantly mismatched DNAs. Polynucleotide sequences capable of hybridizing under stringent conditions with the polynucleotides disclosed herein may be, for example, variants of the disclosed polynucleotide sequences, including allelic or splice variants, or sequences that encode orthologs or paralogs of presently disclosed polypeptides. Polynucleotide hybridization methods are disclosed in detail by Kashima et al. (1985) Nature 313: 402-404, and Sambrook et al. (1989) Molecular Cloning: A Laboratory Manual, 2nd Ed., Cold Spring Harbor Laboratory, Cold Spring Harbor, N.Y. ("Sambrook"); and by Haymes et al., "Nucleic Acid Hybridization: A Practical Approach", IRL Press, Washington, D.C. (1985).

In general, stringency is determined by the incubation temperature, ionic strength of the solution, and concentration of denaturing agents (for example, formamide) used in a hybridization and washing procedure (for a more detailed description of establishing and determining stringency, see below). The degree to which two nucleic acids hybridize under various conditions of stringency is correlated with the extent of their similarity. Thus, similar polynucleotide sequences from a variety of sources, such as within an organism's genome (as in the case of paralogs) or from another organism (as in the case of orthologs) that may perform similar functions can be isolated on the basis of their ability to hybridize with known peptide-encoding sequences. Numerous variations are possible in the conditions and means by which polynucleotide hybridization can be performed to isolate sequences having similarity to sequences known in the art and are not limited to those explicitly disclosed herein. Such an approach may be used to isolate polynucleotide sequences having various degrees of similarity with disclosed sequences, such as, for example, sequences having 60% identity, or more preferably greater than about 70% identity, most preferably 72% or greater identity with disclosed sequences, the resulting sequence having biological activity.

### Methods of the invention

The disclosure provides a method of sequencing a target polynucleotide. The method comprises contacting the target polynucleotide with a pore and a Phi29 DNA polymerase such that the polymerase controls the movement of the target polynucleotide through the pore and a proportion of the nucleotides in the target polynucleotide interacts with the pore. The current passing through the pore during each interaction is measured and this determines the sequence of the target polynucleotide. Steps (a) and (b) are carried out with a voltage applied across the pore. The target polynucleotide is therefore sequenced using Strand Sequencing.

As discussed above, the Phi29 DNA polymerase acts like a molecular brake controlling the movement of the polynucleotide through the pore along the field resulting from the applied voltage. The method has several advantages. For instance, the target polynucleotide moves through the pore at a rate that is commercially viable yet allows effective sequencing. The method may also be carried out in one of three preferred ways based on the three modes of the Phi29 DNA polymerase. These are discussed in more detail below. Each way includes a method of proof reading the sequence.

The method disclosed herein is for sequencing a polynucleotide. A polynucleotide, such as a nucleic acid, is a macromolecule comprising two or more nucleotides. The polynucleotide or nucleic acid may comprise any combination of any nucleotides. The nucleotides can be naturally occurring or artificial. The nucleotide can be oxidized or methylated. A nucleotide typically contains a nucleobase, a sugar and at least one phosphate group. The nucleobase is typically heterocyclic. Nucleobases include, but are not limited to, purines and pyrimidines and more specifically adenine, guanine, thymine, uracil and cytosine. The sugar is typically a pentose sugar. Nucleotide sugars include, but are not limited to, ribose and deoxyribose. The nucleotide is typically a ribonucleotide or deoxyribonucleotide. The nucleotide typically contains a monophosphate, diphosphate or triphosphate. Phosphates may be attached on the 5' or 3' side of a nucleotide.

Nucleotides include, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP). The nucleotides are preferably selected from AMP, TMP, GMP, CMP, UMP, dAMP, dTMP, dGMP or dCMP.

A nucleotide may contain a sugar and at least one phosphate group (that is, lack a nucleobase).

The polynucleotide may be single stranded or double stranded. At least a portion of the polynucleotide is preferably double stranded.

The polynucleotide can be a nucleic acid, such as deoxyribonucleic acid (DNA) or ribonucleic acid (RNA). The target polynucleotide can comprise one strand of RNA hybridized to one strand of DNA. The polynucleotide may be any synthetic nucleic acid known in the art, such as peptide nucleic acid (PNA), glycerol nucleic acid (GNA), threose nucleic acid (TNA), locked nucleic acid (LNA) or other synthetic polymers with nucleotide side chains.

The whole or only part of the target nucleic acid sequence may be sequenced using this method. The target polynucleotide can be any length. For example, the polynucleotide can be at least 10, at least 50, at least 100, at least 150, at least 200, at least 250, at least 300, at least 400 or at least 500 nucleotide pairs in length. The polynucleotide can be 1000 or more nucleotide pairs, 5000 or more nucleotide pairs in length or 100000 or more nucleotide pairs in length.

The target polynucleotide is present in any suitable sample. The disclosure is typically carried out on a sample that is known to contain or suspected to contain the target polynucleotide. Alternatively, the disclosure may be carried out on a sample to confirm the identity of one or more target polynucleotides whose presence in the sample is known or expected.

The sample may be a biological sample. The disclosure may be carried out *in vitro* on a sample obtained from or extracted from any organism or microorganism. The organism or microorganism is typically prokaryotic or eukaryotic and typically belongs to one the five kingdoms: plantae, animalia, fungi, monera and protista. The disclosure may be carried out *in vitro* on a sample obtained from or extracted from any virus. The sample is preferably a fluid sample. The sample typically comprises a body fluid of the patient. The sample may be urine, lymph, saliva, mucus or amniotic fluid but is preferably blood, plasma or serum. Typically, the sample is human in origin, but alternatively it may be from another mammal animal such as from commercially farmed animals such as horses, cattle, sheep or pigs or may alternatively be pets such as cats or dogs. Alternatively a sample of plant origin is typically obtained from a commercial crop, such as a cereal, legume, fruit or vegetable, for example wheat, barley, oats, canola, maize, soya, rice, bananas, apples, tomatoes, potatoes, grapes, tobacco, beans, lentils, sugar cane, cocoa, cotton.

The sample may be a non-biological sample. The non-biological sample is preferably a fluid sample. Examples of a non-biological sample include surgical fluids, water such as drinking water, seawater or river water, and reagents for laboratory tests.

The sample is typically processed prior to being assayed, for example by centrifugation or by passage through a membrane that filters out unwanted molecules or cells, such as red blood cells. The sample may be measured immediately upon being taken. The sample may also be typically stored prior to assay, preferably below -70°C.

A transmembrane pore is a structure that permits hydrated ions driven by an applied potential to flow from one side of the membrane to the other side of the membrane.

Any membrane may be used in accordance with the disclosure. Suitable membranes are well-known in the art. The membrane is preferably an amphiphilic layer. An amphiphilic layer is a layer formed from amphiphilic molecules, such as phospholipids, which have both hydrophilic and lipophilic properties.

The membrane is preferably a lipid bilayer. Lipid bilayers are models of cell membranes and serve as excellent platforms for a range of experimental studies. For example, lipid bilayers can be used for *in vitro* investigation of membrane proteins by single-channel recording. Alternatively, lipid bilayers can be used as biosensors to detect the presence of a range of substances. The lipid bilayer may be any lipid bilayer. Suitable lipid bilayers include, but are not limited to, a planar lipid bilayer, a supported bilayer or a liposome. The lipid bilayer is preferably a planar lipid bilayer. Suitable lipid bilayers are disclosed in International Application No. PCT/GB08/000563 (published as WO 2008/102121), International Application No. PCT/GB08/004127 (published as WO 2009/077734) and International Application No. PCT/GB2006/001057 (published as WO 2006/100484).

Methods for forming lipid bilayers are known in the art. Suitable methods are disclosed in the Example. Lipid bilayers are commonly formed by the method of Montal and Mueller (Proc. Natl. Acad. Sci. USA., 1972; 69: 3561-3566), in which a lipid monolayer is carried on aqueous solution/air interface past either side of an aperture which is perpendicular to that interface.

The method of Montal & Mueller is popular because it is a cost-effective and relatively straightforward method of forming good quality lipid bilayers that are suitable for protein pore insertion. Other common methods of bilayer formation include tip-dipping, painting bilayers and patch-clamping of liposome bilayers.

In a preferred embodiment, the lipid bilayer is formed as described in International Application No. PCT/GB08/004127 (published as WO 2009/077734). Advantageously in this method, the lipid bilayer is formed from dried lipids. In a most preferred embodiment, the lipid bilayer is formed across an opening as described in WO2009/077734 (PCT/GB08/004127).

In another preferred embodiment, the membrane is a solid state layer. A solid-state layer is not of biological origin. In other words, a solid state layer is not derived from or isolated from a biological environment such as an organism or cell, or a synthetically manufactured version of a biologically available structure. Solid state layers can be formed from both organic and inorganic materials including, but not limited to, microelectronic materials, insulating materials such as Si3N4, A1203, and SiO, organic and inorganic polymers such as polyamide, plastics such as Teflon® or elastomers such as two-component addition-cure silicone rubber, and glasses. The solid state layer may be formed from graphene. Suitable graphene layers are disclosed in International Application No. PCT/US2008/010637 (published as WO 2009/035647). The solid state layer may be formed from silicon, silicon nitride, or graphene. The solid state layer may further comprise a solid state pore or a plurality of such pores. The solid state layer or pore may further comprise a linker group compound that is attached by covalent bond. A DNA Polymerase may be attached to a solid state layer or solid state pore using a suitable linker group.

The method is typically carried out using (i) an artificial bilayer comprising a pore, (ii) an isolated, naturally-occurring lipid bilayer comprising a pore, or (iii) a cell having a pore inserted therein. The method is preferably carried out using an artificial lipid bilayer. The bilayer may comprise other transmembrane and/or intramembrane proteins as well as other molecules in addition to the pore. Suitable apparatus and conditions are discussed below with reference to the sequencing embodiments of the disclosure. The method disclosed herein is typically carried out *in vitro.*

The transmembrane pore is preferably a transmembrane protein pore. A transmembrane protein pore is a polypeptide or a collection of polypeptides that permits hydrated ions, such as analyte, to flow from one side of a membrane to the other side of the membrane. In the present disclosure, the transmembrane protein pore is capable of forming a pore that permits hydrated ions driven by an applied potential to flow from one side of the membrane to the other. The transmembrane protein pore preferably permits analyte such as nucleotides to flow from one side of the membrane, such as a lipid bilayer, to the other. The transmembrane protein pore allows a polynucleotide, such as DNA or RNA, to be moved through the pore.

The transmembrane protein pore may be a monomer or an oligomer. The pore is preferably made up of several repeating subunits, such as 6, 7 or 8 subunits. The pore is more preferably a heptameric or octameric pore.

The transmembrane protein pore typically comprises a barrel or channel through which the ions may flow. The subunits of the pore typically surround a central axis and contribute strands to a transmembrane β barrel or channel or a transmembrane α-helix bundle or channel.

The barrel or channel of the transmembrane protein pore typically comprises amino acids that facilitate interaction with analyte, such as nucleotides, polynucleotides or nucleic acids. These amino acids are preferably located near a constriction of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids.

Transmembrane protein pores for use in accordance with the disclosure can be derived from β-barrel pores or α-helix bundle pores. β-barrel pores comprise a barrel or channel that is formed from β-strands. Suitable β-barrel pores include, but are not limited to, β-toxins, such as α-hemolysin, anthrax toxin and leukocidins, and outer membrane proteins/porins of bacteria, such as *Mycobacterium smegmatis* porin (Msp), for example MspA, outer membrane porin F (OmpF), outer membrane porin G (OmpG), outer membrane phospholipase A and *Neisseria* autotransporter lipoprotein (NalP). α-helix bundle pores comprise a barrel or channel that is formed from α-helices. Suitable α-helix bundle pores include, but are not limited to, inner membrane proteins and α outer membrane proteins, such as WZA and ClyA toxin. The transmembrane pore may be derived from Msp or from α-hemolysin (α-HL).

### Phi29 DNA Polymerase

To overcome the limitations disclosed above (low stability of the T7DNAP(exo)-DNA complex under load, diminished signal to noise ratio at 80 mV potential, and the high turnover rate of the polymerase), we examined other DNA-modifying enzymes whose structural and functional properties might facilitate processive catalysis when positioned at a nanopore orifice. An attractive candidate was the bacteriophage phi29 DNA polymerase (phi29 DNAP) (Blanco, L.; Salas, M. J. Biol. Chem. 1996, 271, 8509-8512; (19) Salas, M.; Blanco, L.; Lázaro, J. M.; de Vega, M. IUBMB. Life 2008, 60, 82-85). This DNA-dependent DNA replicase from the B family of DNA polymerases contains both 5'-3' polymerase and 3'-5' exonuclease functions within a single ∼ 66.5 kDa protein chain. Following an initial protein-primed stage that ensures the integrity of the ends of the bacteriophage phi29 linear chromosome, phi29 DNAP transitions to a DNA-primed stage and replicates the entire 19.2 kilobase bacteriophage genome without the need for accessory proteins such as sliding clamps or helicases (Salas, M. Annu. Rev. Biochem. 1991, 60, 39-71). This highly processive polymerase can catalyze the replication of at least 70 kilobases of DNA *in vitro* following a single binding event to a DNA-primed substrate (Blanco, L.; Bernad, A.; Lázaro, J. M.; Martin, G.; Garmendia, C.; Salas, M. J. Biol. Chem. 1989, 264, 8935-940).

Crystal structures of phi29 DNAP revealed the structural basis of this remarkable processivity. The polymerase domain of phi29 DNAP shares the conserved architecture of palm, fingers and thumb sub-domains that resembles a partially open right hand. In addition, a 32 amino acid beta-hairpin insert that is unique to protein-primed DNA polymerases, together with the palm and thumb sub-domains, encircles the primer-template DNA, suggesting that this structure enhances processivity in a manner similar to that achieved by sliding clamp proteins (Johnson, A.; O'Donnell, M. Annu. Rev. Biochem. 2005, 74, 283-315). This same beta-hairpin also forms part of a tunnel that surrounds the downstream template DNA. These features indicate that the beta hairpin insert contributes to both the strong DNA binding and processivity of phi29 DNAP. Consistent with this prediction, deletion of the beta-hairpin results in a mutant phi29 DNAP that displays distributive DNA synthesis activity rather than the processive activity of the wild-type enzyme and a markedly diminished binding affinity for primer-template duplex DNA (Rodriguez, I.; Lazaro, J. M.; Blanco, L.; Kamtekar, S.; Berman, A. J.; Wang, J.; Steitz, T. A.; Salas, M.; de Vega, M. Proc. Natl. Acad. Sci. U. S. A. 2005, 102, 6407-6412).

Experiments using optical tweezers have shown that phi29 DNAP can advance several hundred nucleotides along a template against applied loads of up to ∼ 37 pN, suggesting that this enzyme could replicate a DNA template held atop the nanopore. Here we show that phi29 DNAP-DNA complexes are three-to-four orders of magnitude more stable than KF(exo-)-DNA complexes when captured in an electric field across the α-HL nanopore. DNA substrates in captured complexes were activated for replication by exploiting the 3'-5' exonuclease activity of wild-type phi29 DNAP to excise a 3'-H terminal residue, yielding a primer strand 3'-OH. In the presence of deoxynucleoside triphosphates (dNTPs), DNA synthesis was initiated, allowing real time detection of numerous sequential nucleotide additions that was limited only by the length of the DNA template.

We have observed processive DNA synthesis on a nanopore in an electric field: phi29 DNAP-DNA complexes remained associated with the nanopore orifice and readily catalyzed sequential nucleotide additions under 180 mV applied potential. This is in sharp contrast to T7DNAP(exo-), which was difficult to retain atop the pore for sequential additions even at lower voltages (see Olasagasti et al. 2010 supra).

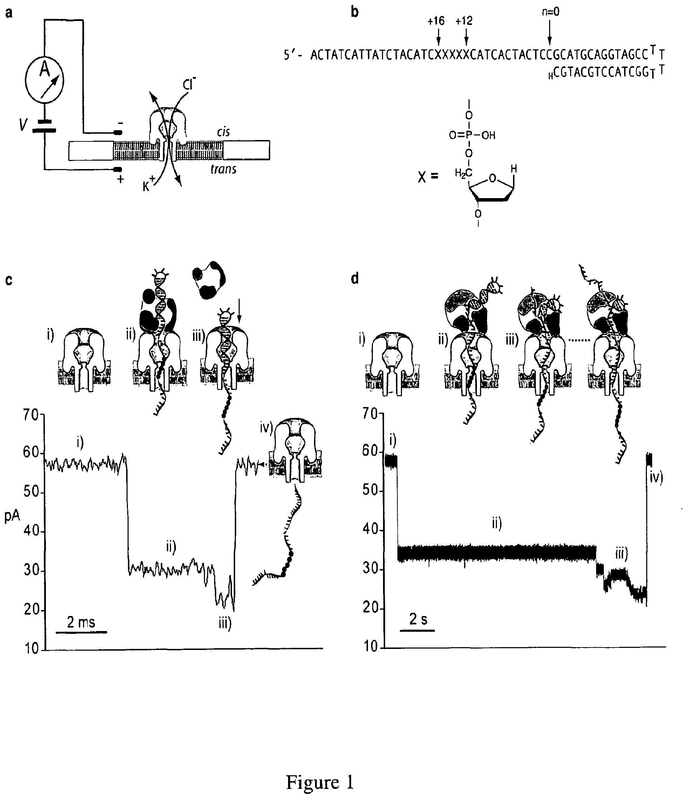

The tenacious binding of phi29 DNAP to DNA is highlighted by the different pathways by which this polymerase and KF(exo-) dissociate from DNA atop the nanopore, under conditions that do not permit exonucleolytic degradation of the DNA by phi29 DNAP. While the bond between KF and DNA can be pulled apart at 180 mV within a few milliseconds (Fig. 1c) with the hairpin duplex base-pairing remaining intact, dissociation from the tight binding of phi29 DNAP requires on average ∼ 20 seconds, and the force pulling on the template strand suspended through the pore must promote unzipping of base-pairs while the duplex is associated with the enzyme (Figs. 1d and 2b,c). The unzipped strand may then move away from the enzyme; the strand may move to a site exterior to the enzyme, to a site not associated with the enzyme, and/or through an aperture or exit pathway region of the enzyme, as shown in the Figures. The unzipped strand may also not be threaded through the enzyme or within a fold of the enzyme and may not even come into physical or chemical contact with the enzyme. The unzipped strand may be threaded over or around the enzyme.

We exploited three features of the phi29 DNAP 3'-5' exonuclease in this study. First, we found that a 3'-H terminated DNA substrate was degraded more slowly in bulk phase than a 3'-OH terminated substrate (Fig. 2a).

To our knowledge this is the first demonstration of discrimination against 3'-H terminated DNA substrates by the 3'-5' exonuclease activity of phi29 DNAP.

This feature provided protection in the bulk phase against both degradation and ddNMP excision-dependent initiation of primer extension of DNA substrate molecules. This protection in turn afforded a window following the addition of Mg²⁺ to the nanopore chamber during which we could capture numerous phi29 DNAP-DNA complexes in series in which the primer terminus was intact.

Second, we used the phi29 DNAP exonuclease activity to excise the ddNMP terminus of the DNA substrate in complexes while they were held atop the pore in an electric field. In the presence of dNTPs, the polymerization reaction is highly favored over processive degradation. Therefore excision of the ddCMP residue to yield a primer strand 3'-OH permitted the subsequent initiation of synthesis from a defined DNA template position.

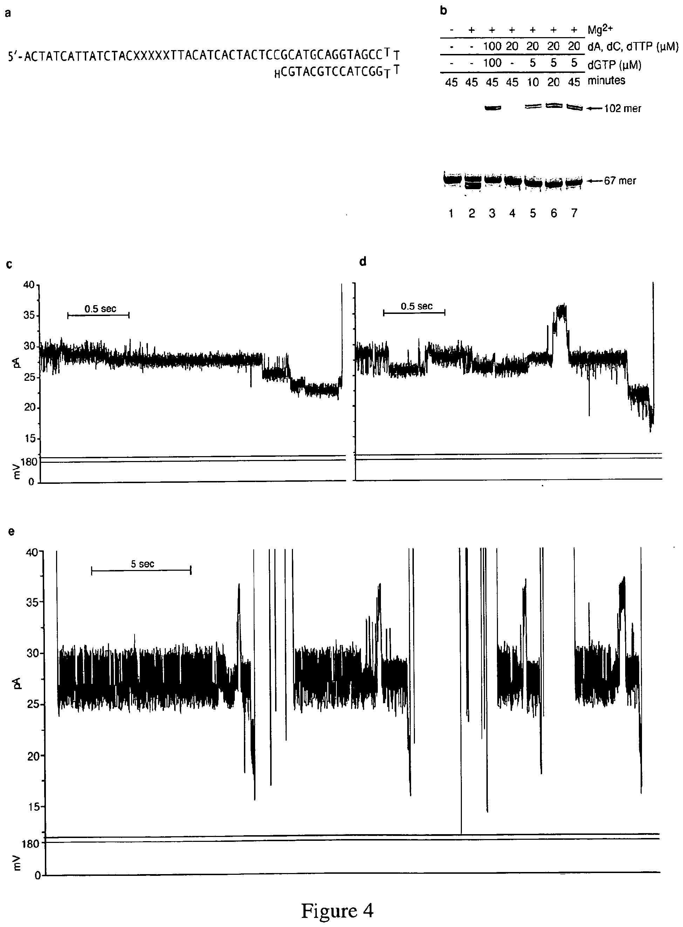

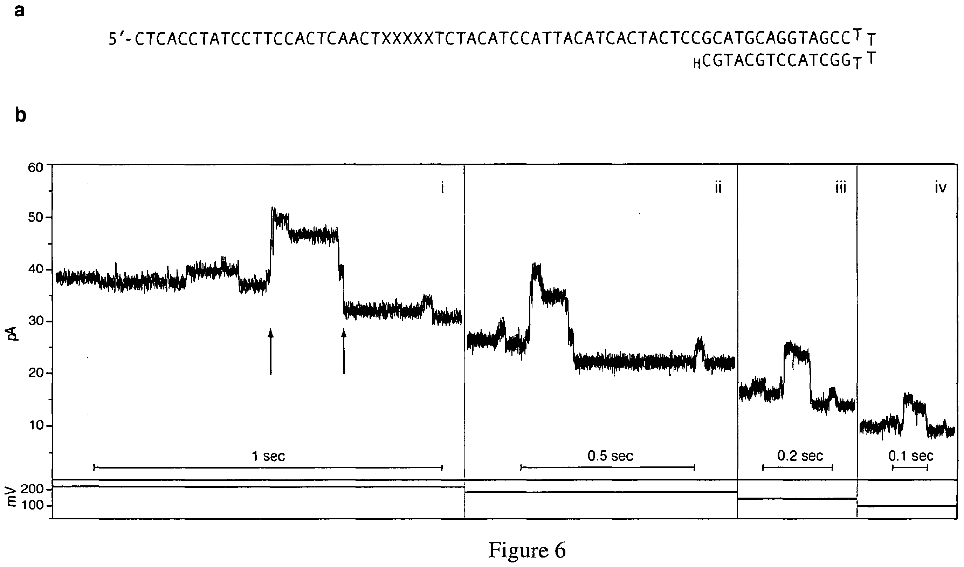

The excision of the ddNMP terminus may be accelerated in complexes by the electric field force atop the pore, as we observed that the time from complex capture to the initiation of synthesis decreased when voltage was increased (not shown). This voltage-promoted excision would nonetheless differ from the processive exonucleolytic regime induced under conditions of high template tension in optical tweezers experiments, in which processive exonucleolytic cleavage dominated even in presence of dNTPs. In contrast, while the initiation of synthesis required excision of the ddCMP residue, the polymerization reaction dominated in the nanopore experiments (Figs. 4, 5, 6 and 7) even at 220 mV applied potential (Fig. 6).

Maintenance of a significant pool of intact, unextended DNA substrate in the bulk phase due to the slow exoncleolytic removal of a ddNMP primer terminus allowed us to examine phi29 DNA-catalyzed synthesis in the nanopore under relatively simple conditions. Nonetheless, due to concerns regarding the slow change in the state of the DNA molecules and potential dNTP substrate depletion in the bulk phase over time, this strategy puts constraints on the time frame in which experiments can be conducted. The use of a more robust means of protecting DNA substrate molecules in the bulk phase, such as the blocking oligomers recently employed with KF(exo-) and T7DNAP(exo-), will extend the utility of this enzyme for both DNA sequencing applications and mechanistic studies of polymerase function using the nanopore.

Third, we used the exonulease domain to systematically move the DNA strand through the nanopore by excising nucleotides at the 3 prime end of the priming strand (Fig. 2c).

This arrangement and set of biochemical reactions is particularly useful for the field of polynucleotide sequencing as the sequence reads of individual nucleotide can be repeated to confirm the base-call as well as having the ability to perform the reactions in a time-frame whereby useful data are generated.

The results of this study demonstrate that phi29 DNAP has properties ideally suited for moving long strands of DNA through nanoscale pores at a rate that is compatible with reliable base detection and identification. In this study we used only chemically synthesized DNA templates, yet the number of sequential nucleotide additions catalyzed by a single enzyme molecule that could be observed was limited only by DNA template length. Features within current traces, such as the ionic current flicker within binary complex events that can predict ternary complex amplitude (Fig. 3), and the oscillation between two amplitudes upon complex capture that precedes replication reactions (Figs. 4, 5, and 7), suggest that biochemical processes such as the fingers opening-closing transition and dNTP binding may have discernible signatures. The ability to observe dynamics in complexes under defined substrate conditions and to resolve individual catalytic cycles (Figs. 4, 5, 6, and 7) in real time at high bandwidth offers the opportunity to quantify biochemical transformations as a function of applied voltage and dNTP concentration.

Here, we describe nanopore analysis of up to 500 DNA templates in single file order using modifications of a blocking oligomer strategy herein described. These optimized blocking oligomers promote pre-loading of phi29 DNAP onto target DNA, while simultaneously protecting the DNA substrate against replication and exonucleolysis in bulk phase for at least five hours. These DNA molecules are activated for replication only when captured on the nanopore.

We have used blocking oligomers to regulate ssDNA movement through the nanopore catalyzed by phi29 DNAP. Improvements were 1) increased protection of p/t DNA from replication and digestion in bulk phase, 2) faster activation of p/t DNA for replication on the nanopore, and 3) forward and reverse ratcheting of DNA through the nanopore. Overall, these improvements increased the throughput of the nanopore for sequencing application to ∼130 analyzed DNA molecules per hour on a single nanopore, and increased the allowable nanopore experiment time to at least 5 hours. In addition, each molecule was analyzed twice by forward and reverse ratcheting through the nanopore. Coupling this method of strand control with a nanopore that can resolve individual nucleotides could potentially allow for sequencing and re-sequencing of the same DNA strands in a nanopore.

Single-channel thin film devices and methods for using the same are provided. The subject devices comprise *cis* and *trans* chambers connected by an electrical communication means. At the *cis* end of the electrical communication means is a horizontal conical aperture sealed with a thin film that includes a single nanopore or channel. The devices further include a means for applying an electric field between the *cis* and *trans* chambers. The subject devices find use in applications in which the ionic current through a nanopore or channel is monitored, where such applications include the characterization of naturally occurring ion channels, the characterization of polymeric compounds, and the like.

In particular, the disclosure provides a novel system comprising a nanopore positioned between the *cis* and *trans* chambers and a DNA polymerase isolated from a mesophile, a halophile, or an extreme halophile microorganism. In one preferred embodiment, the DNA polymerase isolated from the mesophile prokaryote is phi29 DNAP protein. In another preferred embodiment, the DNA polymerase comprises a 5'-3' polymerase and a 3'-5' exonuclease. In a more preferred embodiment, the halophile microorganism is an extreme halophile microorganism. In the alternative, the DNA polymerase is isolated from a virus that can infect a mesophile, a halophile, or an extreme halophile microorganism.

The DNA polymerase may be active in low salt concentrations, for example less than 0.5M salt, or under high-salt concentrations, for example, at least about 0.5 M, at least about 0.6 M, at least about 1 M, at least about 1.5 M, at least about 2 M, at least about 2.5 M, at least about 3 M, at least about 3.5 M, at least about 4 M, at least about 4.5 M, at least about 5 M, at least about 5.5 M, and at saturation.

The disclosure also provides a DNA polymerase that may also be active for significantly longer time than that of a Klenow (exo-) fragment under similar conditions. In one example, the DNA polymerase can be active for up to 40 seconds compared with a few milliseconds using Klenow (exo-) fragment. This ∼10,000-fold increase in activity is clearly an unexpectedly superior result that would not have been predicted by the prior art in any combination, including T7 DNA polymerase which is known to be highly processive in bulk phase when bound to thioredoxin but which rapidly dissociates when captured on a nanopore (Olasagasti, F.; Lieberman, K. R.; Benner, S.; Cherf, G. M.; Dahl, J. M.; Deamer, D. W.; Akeson, M., Nat. Nanotechnol. 2010, advance online publication, doi:10.103 8/nnano.2010.177. The disclosure provides a DNA polymerase that may be active for 40 seconds, for 60 seconds, for 120 seconds, for 5 minutes, for 10 minutes, for 15 minutes, for 20 minutes, for 30 minutes, for 45 minutes, for 60 minutes, for 1.5 hours, for 2 hours, for 4 hours, for 8 hours, for 12 hours, for 16 hours, for 20 hours, for 24 hours, for several days, or for several weeks, including more than one month, or even indefinitely. One additional advantage is that in some instances or circumstances, it is not necessary to provide a step of waiting for a reaction to occur.

In one embodiment, the DNA polymerase activity results in a terminal cascade, a series of discrete ionic current steps.

### Exemplary Uses of the Invention

(1) A nanopore device can be used to monitor the turnover of enzymes such as exonucleases and polymerases, which have important applications in DNA sequencing.
(2) A nanopore device can function as a biosensor to monitor the interaction between soluble substances such as enzyme substrates or signaling molecules. Examples include blood components such as glucose, uric acid and urea, hormones such as steroids and cytokines, and pharmaceutical agents that exert their function by binding to receptor molecules.
(3) A nanopore device can monitor in real time the function of important biological structures such as ribosomes, and perform this operation with a single functional unit.
(4) Various scientific and industrial applications exist in which it would be advantageous to use a DNA polymerase that function efficiently at high salt concentrations. In sequencing, GC compressions can be resolved by using high salt concentrations. In nanopore sequencing high salt concentration boosts the signal to noise ratio for ionic-current-based nanopore measurements. Salt tolerant DNA polymerases may be found among members of the extreme halophiles, in which salt tolerance is achieved not by exclusion of monovalent ions from the cytosol, but by adapting intracellular machinery function in elevated salt. As an example of salt tolerance among members of the extreme halophiles, malate dehydrogenase from the archaeal halophile Haloarcula marismortui incorporates a salt-adaptive strategy where the high ionic concentration from the environment is not only tolerated but is incorporated within the protein. Sodium (or potassium) and chloride ions are found incorporated within the molecule itself. When considering viruses that infect extreme halophiles, not only are proteins of the viral capsid exposed directly to the environment, but the proteins of the replication machinery must operate effectively within the elevated salt environment of its archaeal host.

The high salt tolerance of these DNA polymerases may be very useful for various applications in which high salt concentration is an advantage. For example, the polymerases are useful for sequencing in which they provide better resolution of GC-rich compressions. Additionally the polymerases are useful for nanopore sequencing where a high salt concentration will boost the signal to noise ratio for ionic-current-based nanopore measurements.

### Additional Embodiments

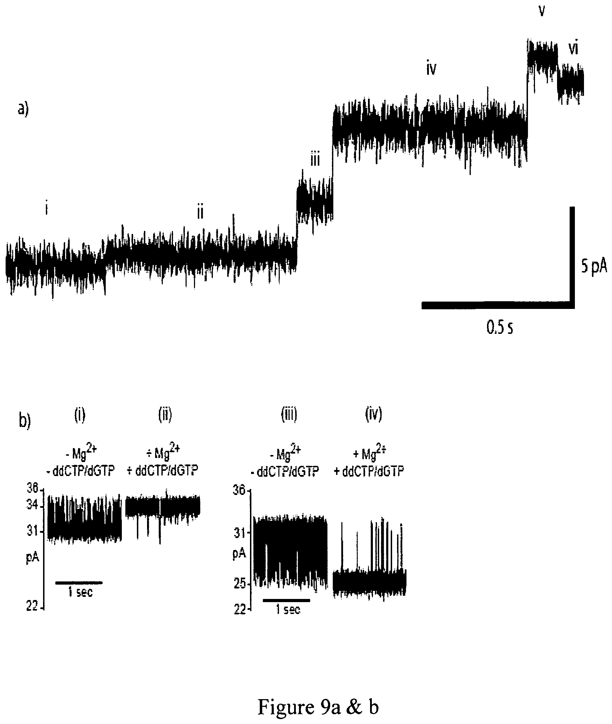

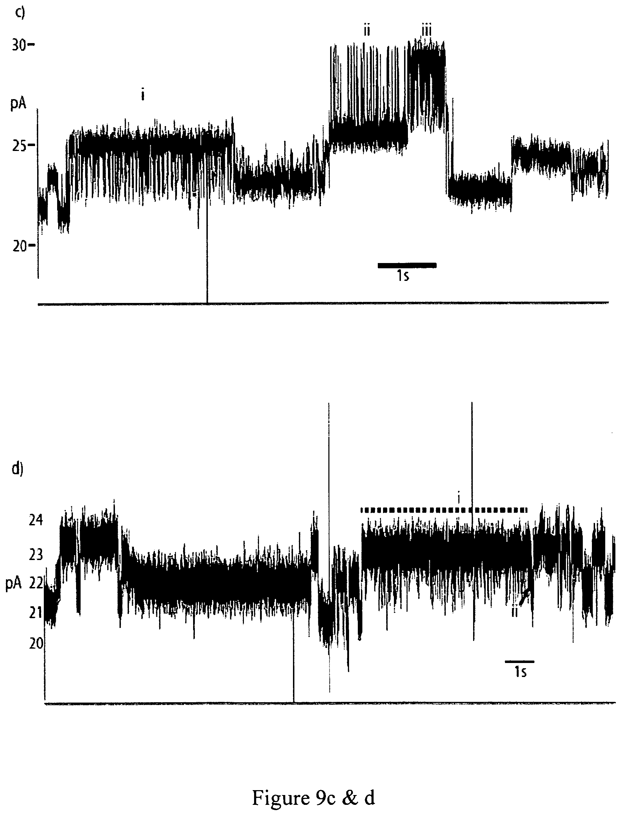

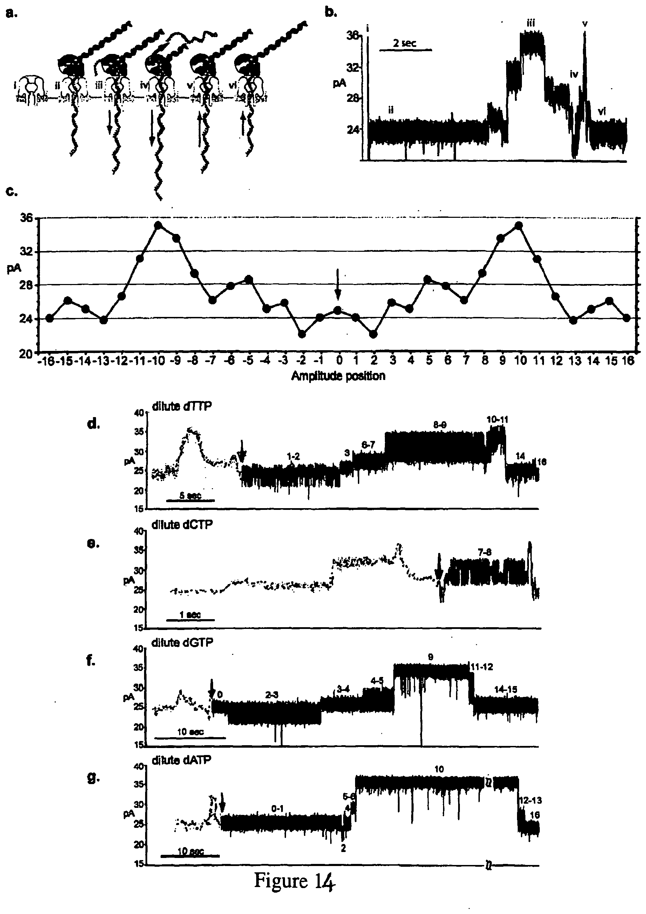

(A) We have also found that DNA polymerase enzymes with a 3'-5' exonuclease can digest DNA from the 3'->5' terminus. We have found that covalently bonding a C3 (CPG) spacer, followed by an abasic residue on the 3'-terminus prevents exonucleolytic digestion of the DNA.
(B) Phi29 DNAP-bound dsDNA unzips in a nanopore by applied voltage (180 mV). Voltage reduction allows re-zipping of the DNA. Restoring the voltage unzips the DNA again and this allows movement of the DNA back and forth through the nanopore.
(C) We have found that when a blocking oligomer binds at the DNA p/t junction the oligomer is stripped off when captured on a nanopore, and the DNA is subsequently activated for ratcheting through the nanopore. Using shorter blocking oligomers decrease the time required to strip the blocking oligomer off the DNA. This allows us to activate DNA molecules for replication on the nanopore faster, and that this increases the throughput of the nanopore for sequencing applications.
(D) Noise in a current trace can help identify neighboring monomers along a polymer strand. Figure 9 shows that as a polymer traverses the nanopore, a monomer or monomers within the polymer determine the average ionic current read by the sensor. In addition, motion of the polymer in the pore can super-impose current fluctuations (noise) on the average current. This noise is dictated in part by the identity of the neighboring monomer (or monomers) and the ionic current associated with those monomers. This would not have been predicted and is therefore an unexpectedly superior result.
(E) Controlled DNA delivery through a nanopore: this is expemplified in Example XVI and in Fig. 14, where we show how we exploit the effect of dNTP concentrations on the rate of DNA synthesis through a nanopore. This would not have been predicted and is therefore an unexpectedly superior result.

### Manufacture Of Single Channel Thin Film Devices

Single-channel thin film devices and methods for using the same are provided. The subject devices comprise a mixed-signal semiconductor wafer, at least one electrochemical layer, the electrochemical layer comprising a semiconductor material, such as silicon dioxide or the like, wherein the semiconductor material further comprises a surface modifier, such as a hydrocarbon, wherein the electrochemical layer defines a plurality of orifices, the orifices comprising a chamber and a neck and wherein the chamber of the orifices co-localize with a first metal composition of the mixed-signal semiconductor wafer, wherein a portion of the orifice is plugged with a second metal, for example, silver, wherein the second metal is in electronic communication with the first metal, and wherein the orifice further comprises a thin film, such as a phospholipid bilayer, the thin film forming a solvent-impermeable seal at the neck of the orifice, the thin film further comprising a pore, and wherein the orifice encloses an aqueous phase and a gas phase. In a preferred embodiment the metallization layer comprises a metal, or metal alloy, such as, but not limited to, nickel, gold, copper, and aluminum.

Pores for use in accordance with the disclosure can be β-barrel pores or α-helix bundle pores. β-barrel pores comprise a barrel or channel that is formed from β-sheets. Suitable β-barrel pores include, but are not limited to, β-toxins, such as α-hemolysin and leukocidins, and outer membrane proteins/porins of bacteria, such as Mycobacterium smegmatis porin A (MspA), MspB, MspC, MspD, outer membrane porin F (OmpF), outer membrane porin G (OmpG), outer membrane phospholipase A and Neisseria autotransporter lipoprotein (NalP). α-helix bundle pores comprise a barrel or channel that is formed from α-helices. Suitable α-helix bundle pores include, but are not limited to, inner membrane proteins and outer membrane proteins, such as E. coli Wza and ClyA toxin. Other useful pore proteins may include the NNN-RRK mutant of the MspA monomer that includes the following mutations: D90N, D91N, D93N, D118R, D134R and E139K.

Methods are known in the art for inserting subunits into membranes, such as lipid bilayers. For example, subunits may be suspended in a purified form in a solution containing a lipid bilayer such that it diffuses to the lipid bilayer and is inserted by binding to the lipid bilayer and assembling into a functional state. Alternatively, subunits may be directly inserted into the membrane using the "pick and place" method described in M.A. Holden, H. Bayley. J. Am. Chem. Soc. 2005, 127, 6502-6503 and International Application No. PCT/GB2006/001057 (published as WO 2006/100484).

The concentration of pore molecule or channel molecule is sufficient to form a single channel in any of the thin films or bilayers in approximately, for example, fifteen minutes. The time to form such channels can be for example, between one-half minute and one hour, for example, about one-half minute, one minute, two minutes, three minutes, four minutes, five minutes, seven minutes, ten minutes, fifteen minutes, twenty minutes, twenty five minutes, thirty minutes, thirty five minutes, forty minutes, forty five minutes, fifty minutes, fifty five minutes, sixty minutes, or any time therebetween. The time for formation can be altered by an operator by several factors or parameters, for example, increasing or decreasing the ambient or incubation temperature, increasing or decreasing the concentration of salt in second solution or first solution, placing a potential difference between the first solution and the second solution that attracts the pore or channel molecule towards the thin film or bilayer, or other methods know to those of skill in the art. The finite state machine can detect and/or sense formation of a single channel in its corresponding bilayer by reacting to the flow of current (ions) through the circuit, the circuit comprising the macroscopic electrode, the second solution, the single nanopore or channel molecule, first solution, and the metal electrode for any given array element.

Formation of biological channels is a stochastic process. Once a single channel has formed in a given array element bilayer, it is preferred that the chance that a second channel so forming therein is reduced or preferably, eliminated. The probability of second channel insertion can be modulated with applied potential, that is potential difference, across the bilayer. Upon sensing a single channel, the finite state machine adjusts the potential on the metal electrode to decrease the possibility of second channel insertion into the same bilayer.

In an alternative embodiment, each array element may comprise a gold electrode surrounding the orifice. This gold electrode may serve to activate chemical reagents using reduction or oxidation reactions and that can act specifically at the location of a specific orifice.

The nanopore system can be created using state-of-the-art commercially available 65nm process technology, for example from Taiwan Semiconductor Manufacturing Company, Taiwan). A 600 x 600 array of nanopores can perform 360,000 biochemical reaction and detection/sensing steps at a rate of 1000 Hz. This may enable sequencing of polynucleotides, for example, to proceed at a rate of 360 million baser per second per 1 cm x 1 cm die cut from the semiconductor wafer.

Exemplary means for applying an electric field between the *cis-* and *trans*-chambers are, for example, electrodes comprising an immersed anode and an immersed cathode, that are connected to a voltage source. Such electrodes can be made from, for example silver chloride, or any other compound having similar physical and/or chemical properties.

### Detection

Time-dependent transport properties of the nanopore aperture may be measured by any suitable technique. The transport properties may be a function of the medium used to transport the polynucleotide, solutes (for example, ions) in the liquid, the polynucleotide (for example, chemical structure of the monomers), or labels on the polynucleotide. Exemplary transport properties include current, conductance, resistance, capacitance, charge, concentration, optical properties (for example, fluorescence and Raman scattering), and chemical structure. Desirably, the transport property is current.

Exemplary means for detecting the current between the *cis* and the *trans* chambers have been described in WO 00/79257, U.S. Patent Nos. 6,46,594, 6,673 6,673,615, 6,627,067, 6,464,842, 6,362,002, 6,267,872, 6,015,714, and 5,795,782 and U.S. Publication Nos. 2004/0121525, 2003/0104428, and 2003/0104428, and can include, but are not limited to, electrodes directly associated with the channel or pore at or near the pore aperture, electrodes placed within the *cis* and the *trans* chambers, ad insulated glass micro-electrodes. The electrodes may be capable of, but not limited to, detecting ionic current differences across the two chambers or electron tunneling currents across the pore aperture or channel aperture. In another embodiment, the transport property is electron flow across the diameter of the aperture, which may be monitored by electrodes disposed adjacent to or abutting on the nanopore circumference. Such electrodes can be attached to an Axopatch 200B amplifier for amplifying a signal.

Applications and/or uses disclosed herein may include, but not be limited to the following:
1. Assay of relative or absolute gene expression levels as indicated by mRNA, rRNA, and tRNA. This includes natural, mutated, and pathogenic nucleic acids and polynucleotides.
2. Assay of allelic expressions.
3. Haplotype assays and phasing of multiple SNPs within chromosomes.
4. Assay of DNA methylation state.
5. Assay of mRNA alternate splicing and level of splice variants.
6. Assay of RNA transport.
7. Assay of protein-nucleic acid complexes in mRNA, rRNA, and DNA.
8. Assay of the presence of microbe or viral content in food and environmental samples via DNA, rRNA, or mRNA.
9. Identification of microbe or viral content in food and environmental samples via DNA, rRNA, or mRNA.
10. Identification of pathologies via DNA, rRNA, or mRNA in plants, human, microbes, and animals.
11. Assay of nucleic acids in medical diagnosis.
12. Quantitative nuclear run off assays.
13. Assay of gene rearrangements at DNA and RNA levels, including, but not limited to those found in immune responses.
14. Assay of gene transfer in microbes, viruses and mitochondria.
15. Assay of genetic evolution.
16. Forensic assays.
17. Paternity assays.
18. Geneological assays.

Polynucleotides homologous to other polynucleotides may be identified by hybridization to each other under stringent or under highly stringent conditions. Single-stranded polynucleotides hybridize when they associate based on a variety of well characterized physical-chemical forces, such as hydrogen bonding, solvent exclusion, base stacking and the like. The stringency of a hybridization reflects the degree of sequence identity of the nucleic acids involved, such that the higher the stringency, the more similar are the two polynucleotide strands. Stringency is influenced by a variety of factors, including temperature, salt concentration and composition, organic and non-organic additives, solvents, etc. present in both the hybridization and wash solutions and incubations (and number thereof), as described in more detail in the references cited above.

Encompassed by the disclosure are polynucleotide sequences that are capable of hybridizing to polynucleotides and fragments thereof under various conditions of stringency (for example, in Wahl and Berger (1987) Methods Enzymol. 152: 399-407, and Kimmel (1987) Methods Enzymol. 152: 507-511). Estimates of homology are provided by either DNA-DNA or DNA-RNA hybridization under conditions of stringency as is well understood by those skilled in the art (Hames and Higgins, Editors (1985) Nucleic Acid Hybridisation: A Practical Approach, IRL Press, Oxford, U.K.). Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

### Characterization and Uses of the Invention

### Sequencing

In one embodiment, the disclosure may be used to perform sequence analysis of polynucleotides. The analyses have an advantage over the prior art and the current art in that a single analysis may be performed at a single site, thereby resulting in considerable cost savings for reagents, substrates, reporter molecules, and the like. Of additional import is the rapidity of the sequencing reaction and the signal generated, thereby resulting in an improvement over the prior art.

Other methods for sequencing nucleic acids are well known in the art and may be used to practice any of the embodiments of the disclosure. These methods employ enzymes such as the Klenow fragment of DNA polymerase I, SEQUENASE, Taq DNA polymerase and thermostable T7 DNA polymerase (Amersham Pharmacia Biotech, Piscataway NJ), or combinations of polymerases and proofreading exonucleases such as those found in the ELONGASE amplification system (Life Technologies, Gaithersburg MD). Preferably, sequence preparation is automated with machines such as the HYDRA microdispenser (Robbins Scientific, Sunnyvale CA), MICROLAB 2200 system (Hamilton, Reno NV), and the DNA ENGINE thermal cycler (PTC200; MJ Research, Watertown MA). Machines used for sequencing include the ABI PRISM 3700, 377 or 373 DNA sequencing systems (PE Biosystems), the MEGABACE 1000 DNA sequencing system (Amersham Pharmacia Biotech), and the like. The sequences may be analyzed using a variety of algorithms that are well known in the art and described in Ausubel et al. (1997; Short Protocols in Molecular Biology, John Wiley & Sons, New York N.Y., unit 7.7) and Meyers (1995; Molecular Biology and Biotechnology, Wiley VCH, New York N.Y., pp. 856-853).

Shotgun sequencing is used to generate more sequence from cloned inserts derived from multiple sources. Shotgun sequencing methods are well known in the art and use thermostable DNA polymerases, heat-labile DNA polymerases, and primers chosen from representative regions flanking the polynucleotide molecules of interest. Incomplete assembled sequences are inspected for identity using various algorithms or programs such as CONSED (Gordon (1998) Genome Res. 8: 195-202) that are well known in the art. Contaminating sequences including vector or chimeric sequences or deleted sequences can be removed or restored, respectively, organizing the incomplete assembled sequences into finished sequences.

### Extension of a Polynucleotide Sequence

The sequences disclosed herein may be extended using various PCR-based methods known in the art. For example, the XL-PCR kit (PE Biosystems), nested primers, and commercially available cDNA or genomic DNA libraries may be used to extend the polynucleotide sequence. For all PCR-based methods, primers may be designed using commercially available software, such as OLIGO 4.06 primer analysis software (National Biosciences, Plymouth MN) to be about 22 to 30 nucleotides in length, to have a GC content of about 50% or more, and to anneal to a target molecule at temperatures from about 55° C to about 68° C. When extending a sequence to recover regulatory elements, it is preferable to use genomic, rather than cDNA libraries.

### Use of Polynucleotides with the Invention

### Labeling of Molecules for Assay

A wide variety of labels and conjugation techniques are known by those skilled in the art and may be used in various nucleic acid, amino acid, and antibody assays. Synthesis of labeled molecules may be achieved using Promega (Madison WI) or Amersham Pharmacia Biotech kits for incorporation of a labeled nucleotide such as ³²P-dCTP, Cy3-dCTP or Cy5-dCTP or amino acid such as ³⁵S-methionine. Nucleotides and amino acids may be directly labeled with a variety of substances including fluorescent, chemiluminescent, or chromogenic agents, and the like, by chemical conjugation to amines, thiols and other groups present in the molecules using reagents such as BIODIPY or FITC (Molecular Probes, Eugene OR).

### Diagnostics

The polynucleotides, fragments, oligonucleotides, complementary RNA and DNA molecules, and PNAs may be used to detect and quantify altered gene expression, absence/presence versus excess, expression of mRNAs or to monitor mRNA levels during therapeutic intervention. Conditions, diseases or disorders associated with altered expression include idiopathic pulmonary arterial hypertension, secondary pulmonary hypertension, a cell proliferative disorder, particularly anaplastic oligodendroglioma, astrocytoma, oligoastrocytoma, glioblastoma, meningioma, ganglioneuroma, neuronal neoplasm, multiple sclerosis, Huntington's disease, breast adenocarcinoma, prostate adenocarcinoma, stomach adenocarcinoma, metastasizing neuroendocrine carcinoma, nonproliferative fibrocystic and proliferative fibrocystic breast disease, gallbladder cholecystitis and cholelithiasis, osteoarthritis, and rheumatoid arthritis; acquired immunodeficiency syndrome (AIDS), Addison's disease, adult respiratory distress syndrome, allergies, ankylosing spondylitis, amyloidosis, anemia, asthma, atherosclerosis, autoimmune hemolytic anemia, autoimmune thyroiditis, benign prostatic hyperplasia, bronchitis, Chediak-Higashi syndrome, cholecystitis, Crohn's disease, atopic dermatitis, dermatomyositis, diabetes mellitus, emphysema, erythroblastosis fetalis, erythema nodosum, atrophic gastritis, glomerulonephritis, Goodpasture's syndrome, gout, chronic granulomatous diseases, Graves' disease, Hashimoto's thyroiditis, hypereosinophilia, irritable bowel syndrome, multiple sclerosis, myasthenia gravis, myocardial or pericardial inflammation, osteoarthritis, osteoporosis, pancreatitis, polycystic ovary syndrome, polymyositis, psoriasis, Reiter's syndrome, rheumatoid arthritis, scleroderma, severe combined immunodeficiency disease (SCID), Sjogren's syndrome, systemic anaphylaxis, systemic lupus erythematosus, systemic sclerosis, thrombocytopenic purpura, ulcerative colitis, uveitis, Werner syndrome, hemodialysis, extracorporeal circulation, viral, bacterial, fungal, parasitic, protozoal, and helminthic infection; a disorder of prolactin production, infertility, including tubal disease, ovulatory defects, and endometriosis, a disruption of the estrous cycle, a disruption of the menstrual cycle, polycystic ovary syndrome, ovarian hyperstimulation syndrome, an endometrial or ovarian tumor, a uterine fibroid, autoimmune disorders, an ectopic pregnancy, and teratogenesis; cancer of the breast, fibrocystic breast disease, and galactorrhea; a disruption of spermatogenesis, abnormal sperm physiology, benign prostatic hyperplasia, prostatitis, Peyronie's disease, impotence, gynecomastia; actinic keratosis, arteriosclerosis, bursitis, cirrhosis, hepatitis, mixed connective tissue disease (MCTD), myelofibrosis, paroxysmal nocturnal hemoglobinuria, polycythemia vera, primary thrombocythemia, complications of cancer, cancers including adenocarcinoma, leukemia, lymphoma, melanoma, myeloma, sarcoma, teratocarcinoma, and, in particular, cancers of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus

The polynucleotides, fragments, oligonucleotides, complementary RNA and DNA molecules, and PNAs, or fragments thereof, may be used to detect and quantify altered gene expression; absence, presence, or excess expression of mRNAs; or to monitor mRNA levels during therapeutic intervention. Disorders associated with altered expression include akathesia, Alzheimer's disease, amnesia, amyotrophic lateral sclerosis, ataxias, bipolar disorder, catatonia, cerebral palsy, cerebrovascular disease Creutzfeldt-Jakob disease, dementia, depression, Down's syndrome, tardive dyskinesia, dystonias, epilepsy, Huntington's disease, multiple sclerosis, muscular dystrophy, neuralgias, neurofibromatosis, neuropathies, Parkinson's disease, Pick's disease, retinitis pigmentosa, schizophrenia, seasonal affective disorder, senile dementia, stroke, Tourette's syndrome and cancers including adenocarcinomas, melanomas, and teratocarcinomas, particularly of the brain. These cDNAs can also be utilized as markers of treatment efficacy against the diseases noted above and other brain disorders, conditions, and diseases over a period ranging from several days to months. The diagnostic assay may use hybridization or amplification technology to compare gene expression in a biological sample from a patient to standard samples in order to detect altered gene expression. Qualitative or quantitative methods for this comparison are well known in the art.

The diagnostic assay may use hybridization or amplification technology to compare gene expression in a biological sample from a patient to standard samples in order to detect altered gene expression. Qualitative or quantitative methods for this comparison are well known in the art.

For example, the polynucleotide or probe may be labeled by standard methods and added to a biological sample from a patient under conditions for the formation of hybridization complexes. After an incubation period, the sample is washed and the amount of label (or signal) associated with hybridization complexes, is quantified and compared with a standard value. If the amount of label in the patient sample is significantly altered in comparison to the standard value, then the presence of the associated condition, disease or disorder is indicated.

In order to provide a basis for the diagnosis of a condition, disease or disorder associated with gene expression, a normal or standard expression profile is established. This may be accomplished by combining a biological sample taken from normal subjects, either animal or human, with a probe under conditions for hybridization or amplification. Standard hybridization may be quantified by comparing the values obtained using normal subjects with values from an experiment in which a known amount of a substantially purified target sequence is used. Standard values obtained in this manner may be compared with values obtained from samples from patients who are symptomatic for a particular condition, disease, or disorder. Deviation from standard values toward those associated with a particular condition is used to diagnose that condition.

Such assays may also be used to evaluate the efficacy of a particular therapeutic treatment regimen in animal studies and in clinical trial or to monitor the treatment of an individual patient. Once the presence of a condition is established and a treatment protocol is initiated, diagnostic assays may be repeated on a regular basis to determine if the level of expression in the patient begins to approximate the level that is observed in a normal subject. The results obtained from successive assays may be used to show the efficacy of treatment over a period ranging from several days to months.

### Purification of Ligand

The polynucleotide or a fragment thereof may be used to purify a ligand from a sample. A method for using a polynucleotide or a fragment thereof to purify a ligand would involve combining the polynucleotide or a fragment thereof with a sample under conditions to allow specific binding, detecting specific binding, recovering the bound protein, and using an appropriate agent to separate the polynucleotide from the purified ligand.

In additional embodiments, the polynucleotides may be used in any molecular biology techniques that have yet to be developed, provided the new techniques rely on properties of polynucleotides that are currently known, including, but not limited to, such properties as the triplet genetic code and specific base pair interactions.

### Composition of the DNA Polymerase

The disclosure also contemplates variants of the processory DNA polymerase. Such variants may have increased or decreased binding affinity for DNA. Such variants may also have increased or decreased rates of reaction. For example, in the KF, the reactive tyrosine residue may be substituted by, for example, tryptophan.

Amino acid substitutions may be made to an peptide sequence, for example up to 1, 2, 3, 4, 5, 10, 20 or 30 substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in Table 1 below. Where amino acids have similar polarity, this can also be determined by reference to the hydropathy scale for amino acid side chains in Table 2.

**Table 1 - Chemical properties of amino acids**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

**Table 2- Hydropathy scale**

| Side Chain | Hydropathy |
|---|---|
| Ile | 4.5 |
| Val | 4.2 |
| Leu | 3.8 |
| Phe | 2.8 |
| Cys | 2.5 |
| Met | 1.9 |
| Ala | 1.8 |
| Gly | -0.4 |
| Thr | -0.7 |
| Ser | -0.8 |
| Trp | -0.9 |
| Tyr | -1.3 |
| Pro | -1.6 |
| His | -3.2 |
| Glu | -3.5 |
| Gln | -3.5 |
| Asp | -3.5 |
| Asn | -3.5 |
| Lys | -3.9 |
| Arg | -4.5 |

Conservative substitutions are those in which at least one residue in the amino acid sequence has been removed and a different residue inserted in its place. Such substitutions generally are made in accordance with the Table 3 when it is desired to maintain the activity of the protein. Table 2 shows amino acids which can be substituted for an amino acid in a protein and which are typically regarded as conservative substitutions.

**TABLE 3**

| **Residue** | **Conservative Substitutions** |
|---|---|
| Ala | Ser |
| Arg | Lys |
| Asn | Gln; His |
| Asp | Glu |
| Gln | Asn |
| Cys | Ser |
| Glu | Asp |
| Gly | Pro |
| His | Asn; Gln |
| Ile | Leu; Val |
| Leu | Ile; Val |
| Lys | Arg; Gln |
| Met | Leu; Ile |
| Phe | Met; Leu; Tyr |
| Ser | Thr; Gly |
| Thr | Ser; Val |
| Trp | Tyr |
| Tyr | Trp; Phe |
| Val | Ile; Leu |

Similar substitutions are those in which at least one residue in the amino acid sequence has been removed and a different residue inserted in its place. Such substitutions generally are made in accordance with the Table 4 when it is desired to maintain the activity of the protein. Table 4 shows amino acids which can be substituted for an amino acid in a protein and which are typically regarded as structural and functional substitutions. For example, a residue in column 1 of Table 4 may be substituted with a residue in column 2; in addition, a residue in column 2 of Table 4 may be substituted with the residue of column 1.

**TABLE 4**

| **Residue** | **Similar Substitutions** |
|---|---|
| Ala | Ser; Thr; Gly; Val; Leu; Ile |
| Arg | Lys; His; Gly |
| Asn | Gln; His; Gly; Ser; Thr |
| Asp | Glu, Ser; Thr |
| Gln | Asn; Ala |
| Cyc | Ser; Gly |
| Glu | Asp |
| Gly | Pro; Arg |
| His | Asn; Gln; Tyr; Phe; Lys; Arg |
| Ile | Ala; Leu; Val; Gly; Met |
| Leu | Ala; Ile; Val; Gly; Met |
| Lys | Arg; His; Gln; Gly; Pro |
| Met | Leu; Ile; Phe |
| Phe | Met; Leu; Tyr; Trp; His; Val; Ala |
| Ser | Thr; Gly; Asp; Ala; Val; Ile; His |
| Thr | Ser; Val; Ala; Gly |
| Trp | Tyr; Phe; His |
| Tyr | Trp; Phe; His |
| Val | Ala; Ile; Leu; Gly; Thr; Ser; Glu |

Substitutions that are less conservative than those in Table 2 can be selected by picking residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, for example, seryl or threonyl, is substituted for (or by) a hydrophobic residue, for example, leucyl, isoleucyl, phenylalanyl, valyl or alanyl; (b) a cysteine or proline is substituted for (or by) any other residue; (c) a residue having an electropositive side chain, for example, lysyl, arginyl, or histidyl, is substituted for (or by) an electronegative residue, for example, glutamyl or aspartyl; or (d) a residue having a bulky side chain, for example, phenylalanine, is substituted for (or by) one not having a side chain, for example, glycine.

The transmembrane protein pore is also preferably derived from α-hemolysin (α-HL). The wild type α-HL pore is formed of seven identical monomers or subunits (i.e. it is heptameric). The sequence of one monomer or subunit of α-hemolysin-NN is shown in SEQ ID NO: 2. The transmembrane protein pore preferably comprises seven monomers each comprising the sequence shown in SEQ ID NO: 2 or a variant thereof. Amino acids 1, 7 to 21, 31 to 34, 45 to 51, 63 to 66, 72, 92 to 97, 104 to 111, 124 to 136, 149 to 153, 160 to 164, 173 to 206, 210 to 213, 217, 218, 223 to 228, 236 to 242, 262 to 265, 272 to 274, 287 to 290 and 294 of SEQ ID NO: 2 form loop regions. Residues 113 and 147 of SEQ ID NO: 2 form part of a constriction of the barrel or channel of α-HL.

In such embodiments, a pore comprising seven proteins or monomers each comprising the sequence shown in SEQ ID NO: 2 or a variant thereof are preferably used in the method disclosed herein. The seven proteins may be the same (homoheptamer) or different (heteroheptamer).

A variant of SEQ ID NO: 2 is a protein that has an amino acid sequence which varies from that of SEQ ID NO: 2 and which retains its pore forming ability. The ability of a variant to form a pore can be assayed using any method known in the art. For instance, the variant may be inserted into a lipid bilayer along with other appropriate subunits and its ability to oligomerise to form a pore may be determined. Methods are known in the art for inserting subunits into membranes, such as lipid bilayers. Suitable methods are discussed above.

The variant may include modifications that facilitate covalent attachment to or interaction with the Phi29 DNA polymerase. The variant preferably comprises one or more reactive cysteine residues that facilitate attachment to the nucleic acid binding protein. For instance, the variant may include a cysteine at one or more of positions 8, 9, 17, 18, 19, 44, 45, 50, 51, 237, 239 and 287 and/or on the amino or carboxy terminus of SEQ ID NO: 2. Preferred variants comprise a substitution of the residue at position 8, 9, 17, 237, 239 and 287 of SEQ ID NO: 2 with cysteine (A8C, T9C, N17C, K237C, S239C or E287C). The variant is preferably any one of the variants described in International Application No. PCT/GB09/001690 (published as WO 2010/004273), PCT/GB09/001679 (published as WO 2010/004265) or PCT/GB10/000133 (published as WO 2010/086603).

The variant may also include modifications that facilitate any interaction with nucleotides.

The variant may be a naturally occurring variant which is expressed naturally by an organism, for instance by a *Staphylococcus* bacterium. Alternatively, the variant may be expressed *in vitro* or recombinantly by a bacterium such as *Escherichia coli.* Variants also include non-naturally occurring variants produced by recombinant technology. Over the entire length of the amino acid sequence of SEQ ID NO: 2, a variant will preferably be at least 50% homologous to that sequence based on amino acid identity. More preferably, the variant polypeptide may be at least 55%, at least 60%, at least 65%, at least 70%, at least 75%, at least 80%, at least 85%, at least 90% and more preferably at least 95%, 97% or 99% homologous based on amino acid identity to the amino acid sequence of SEQ ID NO: 2 over the entire sequence. There may be at least 80%, for example at least 85%, 90% or 95%, amino acid identity over a stretch of 200 or more, for example 230, 250, 270 or 280 or more, contiguous amino acids ("hard homology"). Homology can be determined as discussed above.

Amino acid substitutions may be made to the amino acid sequence of SEQ ID NO: 2 in addition to those discussed above, for example up to 1, 2, 3, 4, 5, 10, 20 or 30 substitutions. Conservative substitutions may be made as discussed above.

One or more amino acid residues of the amino acid sequence of SEQ ID NO: 2 may additionally be deleted from the polypeptides described above. Up to 1, 2, 3, 4, 5, 10, 20 or 30 residues may be deleted, or more.

Variants may fragments of SEQ ID NO: 2. Such fragments retain pore-forming activity. Fragments may be at least 50, 100, 200 or 250 amino acids in length. A fragment preferably comprises the pore-forming domain of SEQ ID NO: 2. Fragments typically include residues 119, 121, 135. 113 and 139 of SEQ ID NO: 2.

One or more amino acids may be alternatively or additionally added to the polypeptides described above. An extension may be provided at the amino terminus or carboxy terminus of the amino acid sequence of SEQ ID NO: 2 or a variant or fragment thereof. The extension may be quite short, for example from 1 to 10 amino acids in length. Alternatively, the extension may be longer, for example up to 50 or 100 amino acids. A carrier protein may be fused to a pore or variant.

As discussed above, a variant of SEQ ID NO: 2 is a subunit that has an amino acid sequence which varies from that of SEQ ID NO: 2 and which retains its ability to form a pore. A variant typically contains the regions of SEQ ID NO: 2 that are responsible for pore formation. The pore forming ability of α -HL, which contains a β-barrel, is provided by β-strands in each subunit. A variant of SEQ ID NO: 2 typically comprises the regions in SEQ ID NO: 2 that form β-strands. The amino acids of SEQ ID NO: 2 that form β-strands are discussed above. One or more modifications can be made to the regions of SEQ ID NO: 2 that form β-strands as long as the resulting variant retains its ability to form a pore. Specific modifications that can be made to the β-strand regions of SEQ ID NO: 2 are discussed above.

A variant of SEQ ID NO: 2 preferably includes one or more modifications, such as substitutions, additions or deletions, within its α-helices and/or loop regions. Amino acids that form α-helices and loops are discussed above.

The variant may be modified to assist its identification or purification as discussed above.

In some embodiments, the transmembrane protein pore is chemically modified. The pore can be chemically modified in any way and at any site. The transmembrane protein pore is preferably chemically modified by attachment of a molecule to one or more cysteines (cysteine linkage), attachment of a molecule to one or more lysines, attachment of a molecule to one or more non-natural amino acids, enzyme modification of an epitope or modification of a terminus. Suitable methods for carrying out such modifications are well-known in the art. The transmembrane protein pore may be chemically modified by the attachment of any molecule. For instance, the pore may be chemically modified by attachment of a dye or a fluorophore.

Any number of the monomers in the pore may be chemically modified. One or more, such as 2, 3, 4, 5, 6, 7, 8, 9 or 10, of the monomers is preferably chemically modified as discussed above.

The reactivity of cysteine residues may be enhanced by modification of the adjacent residues. For instance, the basic groups of flanking arginine, histidine or lysine residues will change the pKa of the cysteines thiol group to that of the more reactive S⁻ group. The reactivity of cysteine residues may be protected by thiol protective groups such as dTNB. These may be reacted with one or more cysteine residues of the pore before a linker is attached.

The molecule (with which the pore is chemically modified) may be attached directly to the pore or attached via a linker as disclosed in International Application Nos. PCT/GB09/001690 (published as WO 2010/004273), PCT/GB09/001679 (published as WO 2010/004265) or PCT/GB10/000133 (published as WO 2010/086603).

Any Phi29 DNA polymerase may be used in accordance with the disclosure. The Phi29 DNA polymerase preferably comprises the sequence shown in SEQ ID NO: 4 or a variant thereof. Wild-type Phi29 DNA polymerase has polymerase and exonuclease activity. It may also unzip double stranded polynucleotides under the correct conditions. Hence, the enzyme may work in three modes. This is discussed in more detail below. A variant of SEQ ID NO: 4 is an enzyme that has an amino acid sequence which varies from that of SEQ ID NO: 4 and which retains polynucleotide binding activity. The variant must work in at least one of the three modes discussed below. Preferably, the variant works in all three modes. The variant may include modifications that facilitate handling of the polynucleotide and/or facilitate its activity at high salt concentrations and/or room temperature. The variant may include Fidelity Systems' TOPO modification, which improves enzyme salt tolerance.

Over the entire length of the amino acid sequence of SEQ ID NO: 4, a variant will preferably be at least 40% homologous to that sequence based on amino acid identity. More preferably, the variant polypeptide may be at least 50%, at least 55%, at least 60%, at least 65%, at least 70%, at least 75%, at least 80%, at least 85%, at least 90% and more preferably at least 95%, 97% or 99% homologous based on amino acid identity to the amino acid sequence of SEQ ID NO: 4 over the entire sequence. There may be at least 80%, for example at least 85%, 90% or 95%, amino acid identity over a stretch of 200 or more, for example 230, 250, 270 or 280 or more, contiguous amino acids ("hard homology"). Homology is determined as described below. The variant may differ from the wild-type sequence in any of the ways discussed below with reference to SEQ ID NO: 2. The polymerase may be covalently attached to the pore.

These methods are possible because transmembrane protein pores can be used to differentiate nucleotides of similar structure on the basis of the different effects they have on the current passing through the pore. Individual nucleotides can be identified at the single molecule level from their current amplitude when they interact with the pore. The nucleotide is present in the pore if the current flows through the pore in a manner specific for the nucleotide (i.e. if a distinctive current associated with the nucleotide is detected flowing through the pore). Successive identification of the nucleotides in a target polynucleotide allows the sequence of the polynucleotide to be determined. As discussed above, this is Strand Sequencing.

During the interaction between a nucleotide in the single stranded polynucleotide and the pore, the nucleotide affects the current flowing through the pore in a manner specific for that nucleotide. For example, a particular nucleotide will reduce the current flowing through the pore for a particular mean time period and to a particular extent. In other words, the current flowing through the pore is distinctive for a particular nucleotide. Control experiments may be carried out to determine the effect a particular nucleotide has on the current flowing through the pore. Results from carrying out the method of the disclosure on a test sample can then be compared with those derived from such a control experiment in order to determine the sequence of the target polynucleotide.

The sequencing methods may be carried out using any apparatus that is suitable for investigating a membrane/pore system in which a pore is inserted into a membrane. The method may be carried out using any apparatus that is suitable for transmembrane pore sensing. For example, the apparatus comprises a chamber comprising an aqueous solution and a barrier that separates the chamber into two sections. The barrier has an aperture in which the membrane containing the pore is formed.

The sequencing methods may be carried out using the apparatus described in International Application No. PCT/GB08/000562.

The methods disclosed herein involve measuring the current passing through the pore during interaction with the nucleotide(s). Therefore the apparatus also comprises an electrical circuit capable of applying a potential and measuring an electrical signal across the membrane and pore. The methods may be carried out using a patch clamp or a voltage clamp. The methods preferably involve the use of a voltage clamp.

The sequencing methods disclosed herein involve the measuring of a current passing through the pore during interaction with the nucleotide. Suitable conditions for measuring ionic currents through transmembrane protein pores are known in the art and disclosed in the Example. The method is typically carried out with a voltage applied across the membrane and pore. The voltage used is typically from -400mV to +400mV. The voltage used is preferably in a range having a lower limit selected from -400 mV, -300mV, -200 mV, -150 mV, -100 mV, -50 mV,-20mV and 0 mV and an upper limit independently selected from +10 mV, + 20 mV, +50 mV, +100 mV, +150 mV, +200 mV, +300 mV and +400 mV. The voltage used is more preferably in the range 100mV to 240mV and most preferably in the range of 160mV to 240mV. It is possible to increase discrimination between different nucleotides by a pore by using an increased applied potential.

The sequencing methods are typically carried out in the presence of any alkali metal chloride salt. In the exemplary apparatus discussed above, the salt is present in the aqueous solution in the chamber. Potassium chloride (KCl), sodium chloride (NaCl) or caesium chloride (CsCl) is typically used. KCl is preferred. The salt concentration is typically from 0.1 to 2.5M, from 0.3 to 1.9M, from 0.5 to 1.8M, from 0.7 to 1.7M, from 0.9 to 1.6M or from 1M to 1.4M. The salt concentration is preferably from 150mM to 1M. In some alternative embodiments, it may be desirable to include salt at saturating concentrations. Phi29 DNA polymerase surprisingly works under high salt concentrations. The salt concentration is preferably at least 0.3M, such as at least 0.4M or 0.5 M. High salt concentrations provide a high signal to noise ratio and allow for currents indicative of the presence of a nucleotide to be identified against the background of normal current fluctuations. Lower salt concentrations may be used if nucleotide detection is carried out in the presence of an enzyme.

The methods are typically carried out in the presence of a buffer. In the exemplary apparatus discussed above, the buffer is present in the aqueous solution in the chamber. Any buffer may be used in the method of the disclosure. Typically, the buffer is HEPES. Another suitable buffer is Tris-HCl buffer. The methods are typically carried out at a pH of from 4.0 to 12.0, from 4.5 to 10.0, from 5.0 to 9.0, from 5.5 to 8.8, from 6.0 to 8.7 or from 7.0 to 8.8 or 7.5 to 8.5. The pH used is preferably about 7.5.

The methods may be carried out at from 0°C to 100°C, from 15°C to 95°C, from 16°C to 90°C, from 17°C to 85°C, from 18°C to 80°C, 19°C to 70°C, or from 20°C to 60°C. The methods are typically carried out at room temperature. The methods are optionally carried out at a temperature that supports enzyme function, such as about 37°C.

As mentioned above, good nucleotide discrimination can be achieved at low salt concentrations if the temperature is increased. In addition to increasing the solution temperature, there are a number of other strategies that can be employed to increase the conductance of the solution, while maintaining conditions that are suitable for enzyme activity. One such strategy is to use the lipid bilayer to divide two different concentrations of salt solution, a low salt concentration of salt on the enzyme side and a higher concentration on the opposite side. One example of this approach is to use 200 mM of KCl on the *cis* side of the membrane and 500 mM KCl in the *trans* chamber. At these conditions, the conductance through the pore is expected to be roughly equivalent to 400 mM KCl under normal conditions, and the enzyme only experiences 200 mM if placed on the *cis* side. Another possible benefit of using asymmetric salt conditions is the osmotic gradient induced across the pore. This net flow of water could be used to pull nucleotides into the pore for detection. A similar effect can be achieved using a neutral osmolyte, such as sucrose, glycerol or PEG. Another possibility is to use a solution with relatively low levels of KCl and rely on an additional charge carrying species that is less disruptive to enzyme activity.

The target polynucleotide being analysed can be combined with known protecting chemistries to protect the polynucleotide from being acted upon by the binding protein while in the bulk solution. The pore can then be used to remove the protecting chemistry. This can be achieved either by using protecting groups that are unhybridised by the pore, binding protein or enzyme under an applied potential (WO 2008/124107) or by using protecting chemistries that are removed by the binding protein or enzyme when held in close proximity to the pore (J Am Chem Soc. 2010 Dec 22;132(50):17961-72).

When the target polynucleotide is contacted with a Phi29 DNA polymerase and pore, the target polynucleotide firstly forms a complex with the Phi29 DNA polymerase. When the voltage is applied across the pore, the target polynucleotide/Phi29 DNA polymerase complex forms a complex with the pore and controls the movement of the polynucleotide through the pore.

As discussed above, wild-type Phi29 DNA polymerase has polymerase and exonuclease activity. It may also unzip double stranded polynucleotides under the correct conditions. Hence, the enzyme may work in three modes. The method may be carried out in one of three preferred ways based on the three modes of the Phi29 DNA polymerase. Each way includes a method of proof reading the sequence. First, the method is preferably carried out using the Phi29 DNA polymerase as a polymerase. In this embodiment, steps (a) and (b) are carried out in the presence of free nucleotides and an enzyme cofactor such that the polymerase moves the target sequence through the pore against the field resulting from the applied voltage. The target sequence moves in the 5' to 3' direction. The free nucleotides may be one or more of any of the individual nucleotides discussed above. The enzyme cofactor is a factor that allows the Phi29 DNA polymerase to function either as a polymerase or an exonuclease. The enzyme cofactor is preferably a divalent metal cation. The divalent metal cation is preferably Mg²⁺, Mn²⁺, Ca²⁺ or Co²⁺. The enzyme cofactor is most preferably Mg²⁺. The method preferably further comprises (c) removing the free nucleotides such that the polymerase moves the target sequence through the pore with the field resulting from the applied voltage (i.e. in the 3' and 5' direction) and a proportion of the nucleotides in the target sequence interacts with the pore and (d) measuring the current passing through the pore during each interaction and thereby proof reading the sequence of the target sequence obtained in step (b), wherein steps (c) and (d) are also carried out with a voltage applied across the pore.

Second, the method is preferably carried out using the Phi29 DNA polymerase as an exonuclease. In this embodiment, wherein steps (a) and (b) are carried out in the absence of free nucleotides and the presence of an enzyme cofactor such that the polymerase moves the target sequence through the pore with the field resulting from the applied voltage. The target sequence moves in the 3' to 5' direction. The method preferably further comprises (c) adding free nucleotides such that the polymerase moves the target sequence through the pore against the field resulting from the applied voltage (i.e. in the 5' to 3' direction) and a proportion of the nucleotides in the target sequence interacts with the pore and (d) measuring the current passing through the pore during each interaction and thereby proof reading the sequence of the target sequence obtained in step (b), wherein steps (c) and (d) are also carried out with a voltage applied across the pore.

Third, the method is preferably carried out using the Phi29 DNA polymerase in unzipping mode. In this embodiment, steps (a) and (b) are carried out in the absence of free nucleotides and the absence of an enzyme cofactor such that the polymerase controls the movement of the target sequence through the pore with the field resulting from the applied voltage (as it is unzipped). In this embodiment, the polymerase acts like a brake preventing the target sequence from moving through the pore too quickly under the influence of the applied voltage. The method preferably further comprises (c) lowering the voltage applied across the pore such that the target sequence moves through the pore in the opposite direction to that in steps (a) and (b) (i.e. as it re-anneals) and a proportion of the nucleotides in the target sequence interacts with the pore and (d) measuring the current passing through the pore during each interaction and thereby proof reading the sequence of the target sequence obtained in step (b), wherein steps (c) and (d) are also carried out with a voltage applied across the pore.

The method of the disclosure preferably involves a pore derived from MspA and a Phi29 DNA polymerase. The Phi29 DNA polymerase preferably separates a double stranded target polynucleotide and controls the movement of the resulting single stranded polynucleotide through the pore. This embodiment has three unexpected advantages. First, the target polynucleotide moves through the pore at a rate that is commercially viable yet allows effective sequencing. The target polynucleotide moves through the Msp pore more quickly than it does through a hemolysin pore. Second, an increased current range is observed as the polynucleotide moves through the pore allowing the sequence to be determined more easily. Third, a decreased current variance is observed when the specific pore and polymerase are used together thereby increasing the signal-to-noise ratio.

### Other methods

Disclosed herein is also a method of forming a sensor for sequencing a target polynucleotide. The method comprises contacting a pore with a Phi29 DNA polymerase in the presence of the target polynucleotide. A voltage is then applied across the pore to form a complex between the pore and the polymerase. This complex is a sensor for sequencing the target polynucleotide. The method preferably comprises contacting a pore derived from Msp with a Phi29 DNA polymerase in the presence of the target nucleic acid sequence and applying a voltage across the pore to form a complex between the pore and the polymerase. Any of the embodiments discussed above with reference to the sequencing method of the disclosure equally apply to this method.

Further disclosed is a method of increasing the rate of activity of a Phi29 DNA polymerase. The method comprises contacting the Phi29 DNA polymerase with a pore in the presence of a polynucleotide. A voltage is applied across the pore to form a complex between the pore and the polymerase and this increases the rate of activity of a Phi29 DNA polymerase. The method preferably comprising contacting the Phi29 DNA polymerase with a pore derived from Msp in the presence of a nucleic acid sequence and applying a voltage across the pore to form a complex between the pore and the polymerase. Any of the embodiments discussed above with reference to the sequencing method of the invention equally apply to this method.

### Kits

The present disclosure also provides kits for sequencing a target polynucleotide. The kits comprise (a) a pore and (b) a Phi29 DNA polymerase. Any of the embodiments discussed above with reference to the sequencing method of the disclosure equally apply to the kits.

The kit may further comprise the components of a membrane, such as the phospholipids needed to form a lipid bilayer.

The kits disclosed herein may additionally comprise one or more other reagents or instruments which enable any of the embodiments mentioned above to be carried out. Such reagents or instruments include one or more of the following: suitable buffer(s) (aqueous solutions), means to obtain a sample from a subject (such as a vessel or an instrument comprising a needle), means to amplify and/or express polynucleotides, a membrane as defined above or voltage or patch clamp apparatus. Reagents may be present in the kit in a dry state such that a fluid sample resuspends the reagents. The kit may also, optionally, comprise instructions to enable the kit to be used in the method of the disclosure or details regarding which patients the method may be used for. The kit may, optionally, comprise nucleotides.

### Apparatus

Also disclosed herein is an apparatus for sequencing a target polynucleotide. The apparatus comprises a plurality of pores and a plurality of Phi29 DNA polymerases. The apparatus preferably further comprises instructions for carrying out the sequencing method of the disclosure. The apparatus may be any conventional apparatus for polynucleotide analysis, such as an array or a chip. Any of the embodiments discussed above with reference to the methods of the disclosure are equally applicable to the apparatus .

The apparatus is preferably set up to carry out the sequencing method of the disclosure.

The apparatus preferably comprises:
a. a sensor device that is capable of supporting the membrane and plurality of pores and being operable to perform polynucleotide sequencing using the pores and proteins;
b. at least one reservoir for holding material for performing the sequencing;a fluidics system configured to controllably supply material from the at least one reservoir to the sensor device; and
c. a plurality of containers for receiving respective samples, the fluidics system being configured to supply the samples selectively from the containers to the sensor device.
The apparatus may be any of those described in International Application No. No. PCT/GB08/004127 (published as WO 2009/077734), PCT/GB10/000789 (published as WO 2010/122293), International Application No. PCT/GB10/002206 (not yet published) or International Application No. PCT/US99/25679 (published as WO 00/28312).

The disclosure will be more readily understood by reference to the following examples, which are included merely for purposes of illustration of certain aspects and embodiments of the present disclosure and not as limitations.

### Examples

Herein are described several examples to demonstrate the capability of measuring macromolecules and polanions or polycations.

### Example I: Enzymes and DNA oligonucleotides Enzyme binding is prevented by a blocking primer.

The D355A, E357A exonuclease-deficient KF (100,000 U ml⁻¹; specific activity 20,000 U mg⁻¹) was from New England Biolabs. Wild-type phi29 DNAP (833,000 U ml⁻¹; specific activity 83,000 U mg⁻¹) was from Enzymatics. DNA oligonucleotides were synthesized at Stanford University Protein and Nucleic Acid Facility and purified by denaturing PAGE.

### Example II: Primer extension and excision assays

A 67 mer, 14 base-pair hairpin DNA substrate labeled with 6-FAM at its 5' end was self-annealed by incubation at 90°C for four minutes, followed by rapid cooling in ice water. Reactions were conducted with 1 µM annealed hairpin and 0.75 µM phi 29 DNAP(exo+) in 10 mM K-Hepes, pH 8.0, 0.3 M KCl, 1 mM EDTA, 1 mM DTT with MgCl₂ added to 10 mM when indicated, and dNTPs added at the concentrations indicated. Reactions were incubated at room temperature for the indicated times and were terminated by the addition of buffer-saturated phenol. Following extraction and ethanol precipitation, reaction products were dissolved in 7 M urea, 0.1X TBE and resolved by denaturing electrophoresis on gels containing 18% acrylamide:bisacrylamide (19:1), 7 M urea, IX TBE. Extension products were visualized on a UVP Gel Documentation system using a Sybr Gold filter. Band intensities were quantified using ImageJ software (NIH).

### Example III: Nanopore experiments.

The nanopore device and insertion of a single α-HL nanopore into a lipid bilayer have been described. Ionic current flux through the α-HL nanopore was measured using an integrating patch clamp amplifier (Axopatch 200B, Molecular Devices) in voltage clamp mode. Data were sampled using an analog-to-digital converter (Digidata 1440A, Molecular Devices) at 100 kHz in whole-cell configuration and filtered at 5 kHz using a low-pass Bessel filter. For voltage clamped experiments, current blockades were measured at the voltages specified in each figure (*trans-*positive). Experiments were conducted at 23 ± 0.2 °C in buffer containing 10 mM K-Hepes pH 8.0, 1 mM EDTA, 1 mM DTT, 0.3 M or 0.6 M KCl as indicated, and 10 mM MgCl₂ where indicated. DNA hairpin substrates were annealed prior to each experiment by heating at 95°C for 3 minutes and rapidly cooling in an ice bath to prevent intermolecular hybridization.

### Example IV: Active voltage control experiments.

Active voltage control of DNAP-DNA complexes atop the nanopore was achieved using finite state machine (FSM) logic, which was programmed with LabVIEW software (Version 8, National Instruments) and implemented on a FPGA system (PCI-7831R, National Instruments), as described previously (Benner et al. 2000 supra; Wilson et al. 2009 supra). Details of the FSM logic applied in the experiments shown in Figures 2 and S2 are given in the figure legends.

### Example V: Nanopore data analysis.

Dwell time and amplitudes for KF(exo-)-DNA binary complexes were quantified using software developed in our laboratory that detects and quantifies the dwell time and amplitude of EBS and terminal current steps of capture events. Current blockades for phi29 DNAP complexes were quantified using Clampfit 10.2 software (Axon Instruments). Dominant *I*_{EBS} values for phi29 binary and ternary complexes were obtained by using Clampfit software to determine the peaks of all-points amplitude histograms measured for 1 to 5 second windows in the initial segment of capture events.

### Example VI: Relative stability of phi29 DNAP-DNA binary complexes and KF-DNA binary complexes.

To perform nanopore experiments, a single α-HL nanopore is inserted in a lipid bilayer separating two chambers (termed *cis* and *trans*) containing buffer solution, and a patch-clamp amplifier applies voltage and measures ionic current (Fig. 1a). To examine binary complexes formed between phi29 DNAP and DNA, we used a 14 base-pair DNA hairpin substrate (Fig. 1b). As demonstrated previously (Benner et al. 2000 supra; Hurt et al. 2009 supra), when a KF-DNA binary complex formed with this substrate is captured in the α-HL pore, the resulting ionic current signature is characterized by an initial enzyme bound state (EBS). This occurs when KF resides atop the pore, holding the double-strand/single-strand junction of the DNA substrate within the confines of the polymerase active site (Fig. 1c, ii). In this KF-bound state, the DNA template strand is suspended through the nanopore lumen, which is wide enough to accommodate single-stranded but not duplex DNA. The amplitude of this state (*I*_{EBS}) can be selectively augmented by an insert of abasic (1',2'-H) residues within the template strand positioned so that it resides in the nanopore lumen when the polymerase-DNA complex is perched atop the pore, such as the 5 abasic residues between template positions +12 to +16 in the DNA hairpin shown in Figure 1b. For KF-DNA binary complexes, the EBS typically lasts a few milliseconds at 180 mV applied potential (Fig. 1c, ii). It is followed by a shorter lower amplitude state (Fig 1c, iii), which occurs when the force pulling on the template strand causes dissociation of KF from the DNA, and the duplex DNA drops into the nanopore vestibule. When this occurs the abasic block that was positioned in the pore lumen during the EBS is displaced to the *trans* side of the pore, where it has negligible effect on the amplitude of this terminal current step (∼20 pA at 180 mV). Unzipping of the DNA hairpin within the vestibule followed by electrophoresis of the strand to the *trans* compartment restores the open channel current (Fig. 1c, iv).

Binary complexes between phi29 DNAP and DNA substrates can be formed in the absence of the divalent cations required for both 5'-3' polymerase and 3'-5' exonuclease activity. When phi29 DNAP-DNA binary complexes were formed with the hairpin substrate in Figure 1b and captured in the α-HL pore at 180 mV (Fig. 1d, ii), the ∼ 35 pA *I*_{EBS} typically lasted tens of seconds (median = 17.6 s, IQR = 25.6, n = 62). This is approximately 10,000 times longer than KF-DNA binary complexes under the same conditions (median = 1.9 ms, IQR = 2.4 ms, n = 199). In contrast to capture events for KF-DNA complexes, these phi29 DNAP-DNA events did not end in a single terminal step, but instead ended in a series of discrete ionic current steps (Fig 1d, iii) that we termed a "terminal cascade". The 3'-5' exonuclease of wild type phi29 DNAP is inhibited under the conditions of the experiment (1 mM EDTA, absent added Mg²⁺) and thus these current steps are not due to digestion of the primer strand. Therefore we reasoned that the DNA duplex may be unzipping while bound within the confines of the enzyme (Fig. 1d, iii). In this scenario, as the template threads out of the complex under tension, the abasic block is drawn out of the lumen in single nucleotide increments that give rise to the sequence of discrete amplitude steps in the terminal cascade (Fig 1d, iii).

Figure 1 illustrates capture of polymerase-DNA binary complexes in the α-HL nanopore. (a) Schematic of the nanopore device. A single α-HL nanopore is inserted in a 30 µm-diameter lipid bilayer that separates two 100 µL wells containing 10 mM K-Hepes, pH 8.0, 300 mM KCl, 1 mM DTT and 1 mM EDTA at 23 °C. The nanopore buffer contained no added MgCl₂. A membrane potential across the bilayer is determined by AgCl electrodes in series with an Axon 200B amplifier. (b) DNA hairpin substrate used in this experiment. The DNA strand is designed to fold back onto itself forming a 14 bp duplex stem joined by a four dTMP residue loop. The 3' residue of the primer strand is ddCMP. The red Xs indicate the five abasic (1 ',22-H) residues that span positions +12 to +16 of the DNA template strand (indicated by numbered arrows above the sequence). Template strand numbering is relative to the first unpaired residue (dCMP) residue at position n = 0 (indicated in blue). The chemical structure of an abasic monomer is shown below the DNA sequence, (c) Ionic current signature for capture of a KF(exo-)-DNA complex at 180 mV applied potential. (i) is the open channel current; (ii) is the enzyme bound state current (*I*_{EBS}); (iii) is the current caused when voltage-promoted dissociation of KF(exo-) from the DNA causes the duplex segment of the hairpin to drop into the pore vestibule; (iv) is the return to open channel current caused by unzipping of the DNA hairpin while it is within the nanopore vestibule followed by electrophoresis to the *trans* compartment. Median EBS dwell time for the KF(exo-) binary complexes was 1.9 ms (n = 199), identical to the dwell time for binary complexes formed with the same hairpin substrate in the presence of 5 mM MgCl₂ . (d) Ionic current signature upon capture of a phi29 DNAP-DNA complex at 180 mV potential. (i) is the open channel current; (ii) is *I*_{EBS} for the phi29 DNAP-DNA binary complex; (iii) is a terminal cascade of the current caused by putative unzipping of the DNA duplex while it is bound to phi29 DNAP, and the consequent ratcheting of the DNA through the pore; and (iv) is the restoration of the open channel current following electrophoresis of the unzipped DNA to the *trans* compartment. The concentrations of KF(exo-) in panel (c) and phi29 DNAP in panel (d) were 0.75 µM; in both panels the DNA concentration was 1.0 µM. Note the difference in time scale between panels (c) and (d).

This model suggests that the interaction between phi29 DNA and the DNA is strong enough that the DNA secondary structure unzips due to the force pulling on the template strand before the bond between phi29 DNAP and DNA can be broken. It furthermore predicts that reducing the applied voltage during the terminal cascade could allow the DNA duplex to re-anneal while associated with the enzyme and thus reset the phi29 DNAP-DNA complex to its original position on the DNA template strand, indicated by a return to the ~ 35 pA state. To test this prediction, we compared the ability of complexes captured in the presence or absence of Mg²⁺ to recover their original EBS amplitude at 180 mV following a controlled voltage drop. A prerequisite for this comparison is a means to ensure that DNA molecules captured in the presence of Mg²⁺ are intact, so that the nanopore assay compares their fate only after capture. Thus exonucleolytic cleavage of the primer strand in the bulk phase must be miminized during the course of the experiment.

We tested whether a 3'-H terminus on the DNA substrate inhibited the rate of 3'-5' exonucleolytic cleavage by phi29 DNAP, in a gel assay comparing degradation of two 67 mer 5'-6-FAM labeled hairpin substrates (Fig. 2a) bearing either a dCMP (lanes 1-6) or ddCMP (lanes 7-12) terminus. Consistent with the requirement for divalent cations for phi29 DNAP 3'-5' exonuclease function, no cleavage of either DNA substrate was observed after 45 minutes incubation in nanopore buffer containing 1 mM EDTA absent added Mg²⁺ (Fig. 2a, lanes 1 and 7). With 10 mM Mg²⁺ present, the extent of DNA digestion for the 3'-H substrate was discernably less than for the 3'-OH substrate. After 10 minutes, while only 24.5% full-length DNA molecules remained for the dCMP-terminated hairpin, 90.5% of the ddCMP-terminated substrate remained intact (Fig. 2a, lanes 4 and 10). After 45 minutes, 4% of the dCMP-terminated substrate and 45% of the ddCMP-terminated substrate remained intact (Fig. 2a, lanes 5 and 11). The protection against excision afforded by a 3'-H terminus is further evidenced by the extent of primer extension in the presence of all four dNTPs. For the 3'-H terminated substrate, the onset of DNA synthesis requires that the ddCMP residue first be excised. Thus while with the 3'-OH terminated hairpin > 80% of the molecules were extended to the full-length 102 mer product in 45 minutes (Fig. 2a, lane 6), with the 3'-H terminated hairpin, 79.8% of the DNA substrate remained intact, with only 20.1% full-length extension product (Fig. 2a, lane 12). Thus 3'-H terminated DNA substrates afforded a window following the addition of Mg²⁺ during which phi29 DNAP-DNA complexes could be captured with the DNA substrate intact. We therefore used the ddCMP terminated hairpin shown in Figure 1b in a nanopore experiment designed to assess the potential for hairpin refolding following initiation of the phi29 DNAP terminal cascade.

In this experiment, upon capture of a phi29 DNAP-DNA complex at 180 mV, a finite state machine (FSM, see Example IV) monitored ionic current in real time until the downward current steps of the terminal cascade were detected (Fig 2b, ii). When the ionic current dropped below 31 pA for at least 0.5 ms (red arrow in Figure 2b), the FSM reduced the applied potential to 70 mV (Fig 2b, iii). After two seconds at 70 mV, the applied potential was restored to 180 mV and the amplitude of the phi29 DNAP-DNA complex was remeasured. In the absence of Mg²⁺, the *I*_{EBS} level was reproducibly reset to the original 35 pA level in each of 11 molecules tested. This EBS amplitude is indicative of the initial state in which phi29 DNAP is bound to the base-paired duplex with the n = 0 template residue positioned in the polymerase active site (Fig 2b, iv), and is consistent with re-annealing of the DNA template with an intact primer strand.

Importantly, the dominant amplitude during the 70 mV intervals was ∼ 10.2 pA, with occasional deflections to ∼ 8.5 pA, measurably above the 6.8 pA value determined for unbound DNA at 70 mV in a control experiment (Figure S2). This indicates that the phi29 DNAP complex remained atop the nanopore orifice without dissociating throughout the lower voltage interval, consistent with a model in which hairpin unzipping at 180 mV and refolding at 70 mV occurs when associated with phi29 DNAP atop the pore.

When the refolding experiment was performed in the presence of 10 mM Mg²⁺, 16 complexes out of 24 captured in the first 12.5 minutes after the addition of Mg²⁺ had the ∼ 35 pA *I*_{EBS} level indicating they were formed with intact DNA substrate molecules (Fig. 2c, i). This 35 pA state was maintained for several seconds (median = 10.2 s, IQR = 12.7 s, n = 16), before ending with a drop in amplitude (Fig. 2c, ii). The features of the steps that occurred following the 35 pA state differed from those that characterized the terminal cascade in the absence of Mg²⁺ (compare Fig. 2b, ii to 2c, ii). For these complexes, when the voltage was reduced to 70 mV for two seconds and then restored to 180 mV, the 35 pA *I*_{EBS} level did not reset for any of the complexes tested (Fig. 2c). This is in contrast to the phi29 DNAP-DNA complexes captured in the absence of Mg²⁺ and it indicates that the DNA substrates, which had been captured intact, were modified by exonucleolytic cleavage while they were held atop the pore. This process of systematic non-catalytic unzipping followed by re-annealing of DNA on the nanopore bound to phi29 DNAP could be repeated numerous times under active voltage control.

Figure 2 illustrates the duplex unzipping during DNA hairpin dissociation from phi29 DNAP at 180 mV applied potential is reversed at 70 mV. (a) Protection in the bulk phase of a 14 bp DNA hairpin substrate from phi29 DNAP-catalyzed 3'-5' exonucleolytic degradation by a ddNMP (3'-H) terminated primer strand. Hairpin substrates (1 µM) labeled with 5'-6-FAM bearing either a 3'-OH (lanes 1-6) or 3'-H (lanes 7-12) terminus were incubated at room temperature with 0.75 µM phi29 DNAP in buffer containing 10 mM K-Hepes, pH 8.0, 300 mM KCl, 1 mM DTT and 1 mM EDTA for the times indicated. The reactions in lanes 1 and 7 contained no added MgCl₂; those in lanes 2-6 and 8-12 contained 10 mM MgCl₂. The reactions in lanes 6 and 12 also contained 200 µM each dATP, dCTP, dGTP and dTTP. Reaction products were resolved on an 18% denaturing polyacrylamide gel. Positions of the gel bands corresponding to the intact 67 mer starting substrates and the 102 mer full-length extension products are indicated with arrows on the side of the gel. Sequences of the 5'-6-FAM labeled DNA hairpins are shown in Figure S1. (b) Steps in the pathway of voltage-promoted phi29 DNAP-DNA complex dissociation are reversible. In this experiment, the buffer contained ImM EDTA and no added MgCl₂ in order to prevent phi29 DNAP 3'-5' exonucleolytic activity. (i) Capture of a phi29 DNAP-DNA binary complex formed with the hairpin substrate shown in Figure 1b. This positions the abasic insert, located between positions +12 to +16 of the template strand, in the limiting aperture of the nanopore lumen, yielding an *I_{EBS}* of 35 pA; (ii) after several seconds in this 35 pA state, a step-wise reduction in current through the nanopore ensues, as the 180 mV applied potential promotes unzipping of the DNA duplex and progressive movement of the five abasic block out of the limiting aperture; (iii) when the current amplitude dropped below 31 pA for at least 0.5 ms, a finite state machine (FSM) reduced the voltage to 70 mV (red arrow in the current trace) for 2 seconds to allow re-annealing of the DNA duplex to its original state (indicated by the curved red arrow in the cartoon) while retaining the phi29 DNAP-DNA complex on the α-HL nanopore; (iv) after 2 seconds at 70 mV, the FSM restored the applied potential to 180 mV. Recovery of the original 35 pA current level (dashed red line) indicates that the phi29 DNAP-DNA complex has reset to its original captured state. (c) phi29 DNAP-DNA complex dissociation under conditions that permit 3'-5' exonucleolytic excision of nucleotides from the DNA primer strand. In this experiment, 10mM MgCl₂ was added to the buffer described in panel b. (i) Capture of a phi29 DNAP-DNA complex in the α-HL nanopore positions the 5 abasic block in the limiting aperture of the nanopore lumen, yielding an *I_{EBS}* of 35 pA that is diagnostic for a complex bearing a DNA substrate with an intact ddCMP terminus; (ii) movement of the 5 abasic block out of the limiting aperture results in a reduction in current through the nanopore, which can be caused by 1) unzipping of the DNA duplex, or 2) phi29 DNAP-catalyzed 3'-5' exonucleolytic degradation of the primer strand while the complex is retained atop the pore; (iii) as in panel b(iii), when the current amplitude dropped below 31 pA for at least 0.5 ms, the FSM reduced the voltage to 70 mV for 2 seconds to allow for re-annealing of the DNA duplex (red arrow in the current trace), while retaining the phi29 DNAP-DNA complex on the nanopore; (iv) in contrast to panel b(iv), restoration of 180 mV applied potential after 2 seconds by the FSM does not recover the original 35 pA *I_{EBS}* (dashed red line), indicating that under conditions that permit catalysis of 3'-5' exonucleolytic excision in phi29 DNAP-DNA complexes atop the pore, the original captured state is not recovered.

### Example VII: Mapping the effect of template abasic insert position on I_{EBS} for DNA substrates bound to phi29 DNAP.

Our strategy for detecting DNA synthesis catalyzed by polymerase-DNA complexes held atop the nanopore employs monitoring changes in ionic current as a block of abasic residues in the template strand is drawn into and through the nanopore lumen in single nucleotide increments when the polymerase advances along the template. This approach permits the recognition of sequential Angstrom-scale movements driven by the enzyme.

As a prelude to DNA replication experiments with phi29 DNAP, we established a reference map that related *I*_{EBS} to the position of a 5 abasic block within the template strand of DNA hairpin substrates (Fig. 3). To construct this map, phi29 DNAP was bound to each of a series of substrates that contained a block of 5 consecutive abasic residues, sequentially displaced by one nucleotide (Fig. 3a). We measured the *I*_{EBS} in buffer containing 0.3M KCl for captured complexes under two conditions: i) 1 mM EDTA with no added Mg²⁺, which permits formation of binary complexes without supporting nucleotide excision or addition (Fig. 3b, lane 1); and ii) 10 mM Mg²⁺, 400 µM ddCTP, and 100 µM dGTP. These latter conditions maintained the intact status of 98.2 and 96% of 3'-H terminated hairpin molecules in the bulk phase for 10 and 45 minutes, respectively (Fig 3b, lanes 6 and 7). Protection was afforded by ddCTP, which permitted the polymerase function of phi29 DNAP to restore the ddCMP terminus of molecules if it was excised by the exonuclease function (Fig. 3b, lanes 3 and 4). Protection was enhanced by the presence of dGTP, which is complementary to the template residue at n = 0 and can form a phi29 DNAP-DNA-dGTP ternary complex in the presence of the 3'-H terminated DNA substrate that can increase the proportion of time the primer terminus resides in the polymerase domain rather than in the exonuclease domain (Fig. 3b, lanes 6 and 7; Fig. S3). The complex formed in the presence of Mg²⁺, ddCTP, and dGTP is therefore operationally defined as a *ternary complex* in this study.

The *I*_{EBS} maps for phi29 DNAP binary complexes (blue dots) and ternary complexes (red dots) are shown in Figure 3c. Both maps were similar to a map determined for KF(exo-)-DNA-dNTP ternary complexes at 80 mV using a six abasic template insert. In 0.3 M KCl at 180 mV, *I*_{EBS} ranged from 22.3 pA for the ternary complex formed with the 5ab(6,10) substrate (abasic block spanning template positions +6 to +10 measured from n = 0 in the polymerase catalytic site), to 35.4 pA for the binary complexes formed with the 5ab(11,15) and 5ab(9,13) substrates (abasic blocks spanning template positions +11 to +15, and +9 to +13, respectively). This gives a dynamic amplitude range of at least 13 pA for the detection of enzyme movements during polymerization or exonucleolytic reactions.

At all positions within the map, *I*_{EBS} for the binary and ternary complexes were offset from one another. The direction and the scale of the offset depended in part on the position along the map. For example, at position (i) (Fig. 3c), the change from a binary complex to a ternary complex caused an *I*_{EBS} increase from 31.5 pA to 34.5 pA. By comparison, at position (ii) (Fig. 3c) the binary to ternary change resulted in a relatively small current increase from 34.4 to 35.2 pA, and at position (iii) (Fig 3c) the binary to ternary transition caused a large *I*_{EBS} current *decrease* from 31.5 pA to 25.5 pA. Interestingly, the direction and magnitude of an ionic current flicker within the binary state often predicted the dominant amplitude observed for the ternary complex formed with the same substrate (Fig. 3d).

Figure 3 illustrates EBS amplitudes at 180 mV of phi29 DNAP-DNA complexes as a function of abasic insert position in DNA template strands, (a) DNA hairpins used in phi29 DNAP mapping experiments. In each sequence, red Xs indicate the positions of the abasic (1 ',2'-H) residues. Abasic configuration is denoted as 5ab(x,y), where 5 is the number of abasic residues in the insert, and x and y indicate the distance (in nucleotides) of the first and last abasic residues of the insert, measured from the template strand dNMP at n = 0 in the polymerase catalytic site. The self-complementary sequence blocks that form the 14 base pair hairpin are underlined. The abasic configuration for each hairpin is indicated to the left of each sequence, (b) State of hairpin substrates in the bulk phase during nanopore experiments to map the amplitude of phi29 DNAP-DNA complexes. A5'-6-FAM, 3'- H 14 bp hairpin (1 µM) was incubated at room temperature with 0.75 µM phi29 DNAP in buffer containing 1 mM EDTA, absent (lane 1) or present (lanes 2-7) 10 mM MgCl₂ for the times indicated. Reactions included 400 µM ddCTP (lanes 4 and 5) or 400 µM ddCTP and 100 µM dGTP (lanes 6 and 7). The conditions in lane 1 are those employed to map the amplitude of the phi29 DNAP-DNA binary complexes. Conditions in lanes 6 and 7 are those used to map the amplitude of phi29 DNAP-DNA-dGTP ternary complexes, (c) Map of dominant amplitude values in buffer containing 0.3 M KCl for the EBS of phi29 DNAP-DNA binary (blue circles) or phi29 DNAP-DNA-dGTP ternary (red circles) complexes. Each point represents the average *I*_{EBS} determined from three separate experiments +/- the standard error. The blue and red dashed lines indicate the amplitudes for phi29 DNAP binary and ternary complexes, respectively, formed with a DNA hairpin substrate composed of normal DNA residues bearing no abasic insert. (d) Current traces showing representative segments of events for complexes captured under binary (labeled as -Mg²⁺, -ddCTP/dGTP) or ternary (labeled as +Mg²⁺, +ddCTP/dGTP) mapping conditions, formed with DNA hairpin substrates with the abasic configurations (i) 5ab(13,17), (ii) 5ab(12,16), or (iii) 5ab(8,12). The positions on the map for complexes formed with these substrates are indicated by corresponding lower case Roman numerals in panel 3c.

The results of the mapping experiments permit a prediction based upon the model proposed for the molecular events that give rise to the terminal cascade (Figs. 1 and 2): the sequence of current steps in the terminal cascade of binary complex capture events should vary in a manner that is dependent on the initial position of the abasic block in the complex. This was found to be the case. For example, when the duplex segment of the 5ab(6,10) substrate was unzipped during the terminal cascade, the abasic block was drawn from its position proximal to the enzyme towards the *trans* chamber. This resulted in a series of current steps with a ∼ 36 pA peak as the abasic block traversed the pore lumen (Fig. S4, a). In contrast, for binary complexes formed with the 5ab(18,22) substrate, the initial position of the abasic block is distal from the enzyme. When this substrate is unzipped in the terminal cascade, no amplitude peak is observed (Fig. S4, b).

### Example VIII: Controlled translocation of DNA templates in the nanopore catalyzed by phi29 DNAP.

Results from our laboratory have shown that advance of a DNA template in the α-HL nanopore could be detected at single nucleotide precision during replication by T7DNAP(exo-). However, for the majority of complexes with this enzyme only one or two nucleotide addition cycles could be monitored. To determine if phi29 DNAP was more efficient at catalyzing sequential nucleotide additions on the nanopore, we measured phi29 DNAP-driven displacement of synthetic DNA substrates molecules bearing 5 abasic inserts in their template strands. The map in Figure 3 was used to interpret changes in *I*_{EBS} as single nucleotides were enzymatically added to or removed from the DNA 3' terminus.

The experiment in Figure 2c showed that the slow excision of a ddNMP residue in the bulk phase could be exploited to capture complexes in the presence of Mg²⁺ in which the primer strand was intact. Importantly, this experiment also showed that excision of the ddNMP residue could be achieved on the pore, exposing the 3'-OH of the -1 residue and thus yielding a substrate that is potentially competent for synthesis reactions atop the pore in the presence of dNTPs. Consistent with previous findings, the gel assay in Figure 2a showed that in the presence of dNTPs the polymerization reaction dominated over the exonuclease reaction in bulk phase. These findings were essental to our strategy for DNA replication experiments: capture phi29 DNAP complexes bearing intact 3'-H terminated substrates in the presence of dNTPs, allow the excision reaction to occur on the pore, and use an abasic block marker in the template strand to determine unambiguously whether the polymerization reaction can be observed for complexes held atop the pore. Using this strategy, the majority of complexes captured in the nanopore should initiate replication at the same template position (-1 relative to the original n = 0 position of the starting substrate).

Because dGTP can slow the rate of ddCMP excision due to formation of ternary complexes (Fig. 3b, Fig. S3) we chose to conduct initial nanopore synthesis experiments using 20 µM each of dATP, dCTP, dTTP and 5 µM dGTP. We determined the effect of these conditions on the state of the DNA substrate molecules in bulk phase in a gel assay using the 5'-6-FAM, 3'-H hairpin substrate (Fig. 4b). After 10 minutes, 82.5% of the 67 mer starting substrate remained intact, and 13.6% was extended to the 102 mer product. After 20 minutes, these proportions were 69.4% and 26.1% extension product, and by 45 minutes almost 30% of the fluorescein labeled hairpin had been extended. We therefore confined our measurements in the nanopore experiments to the first 10 minutes following the addition of Mg²⁺ and dNTP substrates to the *cis* chamber.

In initial nanopore replication experiments under these conditions (Fig. 4), we used a DNA substrate with the starting abasic configuration 5ab(15,19) bearing a 3' ddCMP terminus (Fig. 4a). Typical ionic current traces for capture of phi 29 DNAP-DNA complexes at 180 mV with this substrate in the presence of 10 mM Mg²⁺, with or without dNTPs, are shown in Figure 4c and 4d, respectively. The dominant initial *I*_{EBS} upon capture was ∼ 29 pA under both conditions, with deflections to ∼ 26 pA consistent with an oscillation between the map values for 5ab(15,19) binary and ternary complexes (Fig. 3c). Under both conditions, there was a delay at this starting *I*_{EBS} level, afforded by the slow excision of the 3' ddCMP terminus, after which a series of current changes ensued. We interpret the current changes in the experiment conducted in the absence of dNTPs (Fig. 4c) as follows: upon ddCMP excision, the phi29 DNAP exonuclease continued to sequentially cleave nucleotides from the primer terminus, resulting in a progressively shorter duplex segment and greater distance between the enzyme and the abasic insert. The abasic segment was thus moved through the pore toward the *trans* compartment, causing a progressive ionic current decrease. Eventually, the ionic current returned to the open channel state, consistent with dissociation of the DNA molecule from phi29 DNAP and its subsequent electrophoresis into the *trans* compartment.

In contrast, when the experiment was conducted in the presence of 20 µM each dATP, dCTP, dTTP and 5 µM dGTP a different ionic current pattern resulted, characterized by a peak at 35.4 pA (Fig. 4d). We hypothesized that these current changes occurred because, following phi29 DNAP excision of the ddCMP residue protecting the DNA 3' terminus, the presence of dNTPs favored nucleotide additions catalyzed by phi29 DNAP while atop the pore. The duplex DNA segment was lengthened as phi29 DNAP moved progressively closer to the abasic insert within the DNA template, drawing it through the nanopore lumen with the attendant traversal of the major ionic current peak between abasic configurations 5ab(15,19) to 5ab(6,10) in the map in Figure 3b. Several DNA template replication reactions, catalyzed by phi29 DNAP-DNA complexes captured in series during this experiment are shown in Figure 4e.

In the gel experiment shown in Figure 4b, in addition to the starting 67 mer hairpin substrate and the full length extension products, intermediate bands corresponding to partial extension products accumulated with time (Fig. 4b, lanes 6 and 7). These products could arise due to depletion of dNTP pools in the bulk phase, as an increasing fraction of the DNA substrate molecules that are present at 1 µM in both the gel and nanopore assays are replicated. Because this has the potential to affect the extent and rate of synthesis catalyzed by phi29 DNAP complexes atop the pore, we examined whether this could be minimized by using a higher concentration of dNTPs.

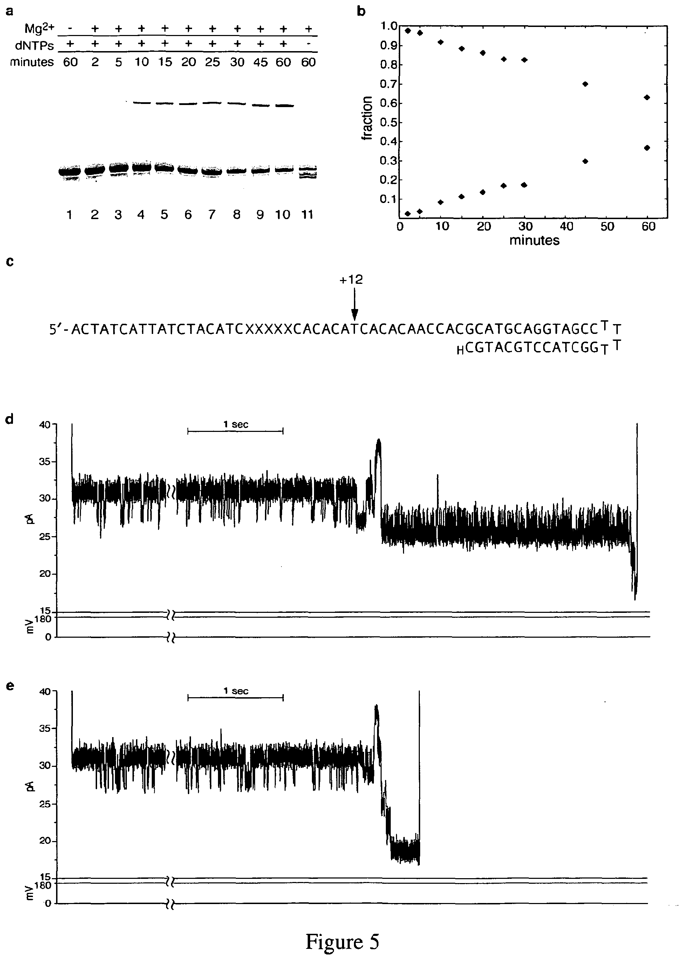

We measured the extent of primer extension for the 5'-6-FAM, 3'-H terminated hairpin in the presence of 100 µM each of dGTP, dCTP, dTTP and dATP as a function of time (Fig. 5a and b). Under these conditions the rate of accumulation of the full-length product was slower than in the experiment in Figure 4b (using 20 µM each of dCTP, dTTP, dATP and 5 µM dGTP), likely due to the more efficient inhibition of excision of the ddCMP terminus afforded by the higher dGTP concentration. After 20 minutes, 86.3% of the starting DNA substrate remained intact, and 13.6% was fully extended (Fig. 5a, lane 6, and 5b), compared to 69.4% and 26.1% for these species, respectively, in reactions conducted for the same amount of time with the lower concentrations of dNTPs (Fig. 4b, lane 6). Importantly, even after 30 minutes, accumulation of shorter extension products was below the limit of detection of the assay. We therefore used dNTP substrates at a concentration of 100 µM each in subsequent replication experiments.

To test the model proposed for the ionic current signatures observed in the replication experiment in Figures 4d and 4e, we used a DNA hairpin substrate in which the first template dTMP residue was at a defined position relative to the abasic insert (Fig. 5). When DNA synthesis reactions are conducted with this substrate in the presence of 100 µM each of dGTP, dCTP, dTTP and ddATP, 12 nucleotides can be added, during which the abasic block will be drawn from its starting position of 5ab(18,22), across the 35.4 pA peak at 5ab(11,15), to position 5ab(6,10). After reaching the dTMP residue at position +12, replication is predicted to stall. In contrast, replication reactions conducted in the presence of 100 µM each dGTP, dCTP, dTTP and dATP should proceed past the +12 position.

Figure 4 illustrates DNA replication catalyzed by phi29 DNAP on the nanopore. (a) DNA hairpin substrate for nanopore replication experiments. The starting abasic configuration for this substrate is 5ab(15,19). The onset of primer extension requires exonucleolytic excision of the terminal ddCMP residue, after which fifteen nucleotides can be added before the enzyme reaches the abasic block. As replication proceeds, the 5 abasic residue block will be drawn through and past abasic configurations 5ab(15,19) to 5ab(6,10), which comprise the major peak in the map in Figure 3. (b) Phi29 DNAP-catalyzed primer extension of a DNA hairpin substrate in bulk phase under nanopore experiment conditions. A 67 mer, 5'-6-FAM, 3'-H 14 bp hairpin (1 µM) was incubated at room temperature for the indicated times with 0.75 µM phi29 DNAP in buffer containing 10 mM K-Hepes, pH 8.0, 0.3 M KCl, 1 mM DTT, and 1 mM EDTA, absent (lane 1) or present (lanes 2-7) 10 mM MgCl₂, with dNTPs added as indicated. Reaction products were resolved on an 18% denaturing polyacrylamide gel. Lanes 5-7 show the extent of primer extension at 10, 20, and 45 minutes in bulk phase under the dNTP substrate conditions of the nanopore experiments in panels d and e (5 µM dGTP, 20 µM each dATP, dCTP, and dTTP). (c) Representative capture event for a phi29 DNAP-DNA complex formed with the 5ab(15,19) hairpin shown in panel a, in the presence of 1 mM EDTA and 11 mM MgCl₂, absent dNTPs. (d) Representative capture event for a phi29 DNAP-DNA complex formed with the 5ab(15,19) hairpin shown in panel a in the presence of 1 mM EDTA, 11 mM MgCl₂, and 5 µM dGTP, 20 µM each dATP, dCTP, and dTTP. (e) Phi29 DNAP-catalyzed replication of individual DNA substrate molecules captured in series. The current trace is shown in real time; the first event in the series of four is the event shown expanded in panel d. Current traces shown in panels c-e were collected within the first 10 minutes of the addition of MgCl₂ (c) or MgCl₂ and dNTPs (d, e) to minimize dNTP depletion due to bulk phase reactions.

When phi29 DNAP complexes formed with this DNA substrate were captured under both of these conditions, an initial period of several seconds occurred during which the dominant current amplitude was ∼ 31 A, with oscillations to ∼ 27 pA (Fig. 5d and e), similar to the map values for the ternary and binary complexes for this 5ab(18,22) configuration (Fig. 3c). After this state ended, the 35.4 pA ionic current peak was rapidly traversed, indicative of the abasic block being drawn through the lumen. If dGTP, dCTP, dTTP and ddATP were present in the *cis* chamber, after traversing the peak the polymerase stalled in a state in which the current oscillated between a dominant amplitude of ∼25 pA to 28 pA for several seconds (Fig 5d). In contrast, in the presence of dATP rather than ddATP, the polymerase advanced without stalling through and beyond the 25 pA state (Fig. 5e). This establishes that the stalled state observed in the presence of ddATP (which indicates replicating complexes have reached the dTMP residue) is attained *after* the template segment that causes the amplitude peak traverses the lumen. Because reaching this dTMP template residue requires the nucleotide incorporations necessary to traverse the 5ab(17,21) to 5ab(7,11) abasic configurations, these experiments verify that the characteristic amplitude peak is due to replication that ensues following ddCMP excision on the pore.

Figure 5 illustrates phi29 DNAP-catalyzed replication up to or through a specific template position. (a) Time course of primer extension for a DNA hairpin substrate in bulk phase, in the presence of phi29 DNAP and 100 µM each dGTP, dCTP, dTTP and dATP. A 67 mer, 5'-6-FAM, 3'-H 14 bp hairpin (1 µM) was incubated at room temperature with 0.75 µM phi29 DNAP in buffer containing 1 mM EDTA, absent (lane 1) or present (lanes 2-7) 10 mM MgCl₂ and 100 µM each of all four dNTPs (lanes 1-10) for the times indicated. The onset of primer extension requires exonucleolytic excision of the terminal ddCMP residue preceding processive dNTP additions. Reaction products were resolved on an 18% denaturing polyacrylamide gel. (b) The fluorescence intensity of bands in the gel in panel a corresponding to the intact, unextended hairpin (blue diamonds) and the extension product (red diamonds) were quantified using ImageJ software (NIH). For each lane, the fraction of the total fluorescence for these two bands was plotted as a function of reaction time. (c) DNA hairpin substrate for nanopore replication experiments. The starting abasic configuration is 5ab(18,22). In the presence of dGTP, dCTP, dTTP and ddATP, 12 nucleotides can be added up to ddATP addition in response to the first template dTMP residue (blue). This dTMP residue is positioned such that reaching this endpoint requires replication of a segment of template during which the abasic block (red Xs) is drawn into and through the nanopore lumen. After ddATP incorporation, a phi29 DNAP-DNA-dTTP ternary complex can be formed with abasic configuration 5ab(6,10). In the presence of dGTP, dCTP, dTTP and dATP, replication can proceed past the +12 position up to the abasic block. (d) phi29 DNAP-catalyzed replication on the hairpin substrate shown in panel c in the presence of 100 µM each dGTP, dCTP, dTTP and ddATP, in buffer containing 0.3 M KCl and 10 mM MgCl₂. (e) phi29 DNAP-catalyzed replication after 200 µM dATP was added to the experiment shown in panel (d). Events shown in panels d and e are representative of dozens of complexes captured. Events in a control experiment in which 100 µM each dGTP, dCTP, dTTP and dATP were added absent ddATP were identical to the representative event shown in panel e. Complexes were captured within the first 10 minutes after the addition of MgCl₂ to the nanopore chamber.

### Example IX: The rate of phi29 DNAP catalyzed DNA replication is influenced by applied voltage across the nanopore.

Experiments using optical tweezers have shown that the rate of replication catalyzed by phi29 DNAP is slowed by tension on the template at forces between ∼ 20 and ∼ 37 pN. This result predicts that the rate of phi29 DNAP replication would be influenced by the voltage applied across the nanopore. However, the voltage regime where this would occur is not known.

Figure 6 shows representative events during phi29 DNAP replication reactions along a 25 nt template segment of a DNA hairpin substrate (Fig. 6a), for experiments in which the applied potential was varied in 40 mV increments in the range between 220 mV and 100 mV. The starting abasic configuration for this substrate was 5ab(25,29); therefore during DNA synthesis, the 5 abasic insert will be drawn through the limiting aperture of the nanopore lumen, spanning abasic configurations 5ab(18,22) to 5ab(6,10) and thus the amplitudes mapped in Figure 3c. These peaks were traversed at each voltage, at rates that appeared to increase as applied voltage was decreased (Fig. 6b). We measured the time required to advance between two readily discernible current amplitudes corresponding to positions flanking the major current peak (blue arrows in Figure 6b, i), separated by approximately five nucleotides. At 220 mV, the median time required for replication over this distance was 227 ms (IQR = 174 ms, n = 45); at 100 mV, the median time for replication was 67 ms (IQR = 41 ms, n = 59).

Figure 6 illustrates phi29 DNAP-catalyzed replication by complexes held atop the nanopore at different voltages, (a) DNA hairpin substrate for nanopore replication experiments. The starting abasic configuration for this substrate is 5ab(25,29). After the exonucleolytic excision of the terminal ddCMP residue that is required for initiation of DNA synthesis, 25 nucleotides can be added before the enzyme reaches the abasic block. During DNA synthesis, the 5 abasic insert will be drawn through and past abasic configurations 5ab(18,22) to 5ab(6,10), which spans the positions mapped in Figure 3. (b) Representative current traces showing phi29 DNAP replication of the hairpin substrate shown in panel a, in buffer containing 0.3 M KCl, 10 mM MgCl₂, in the presence of 100 µM each dGTP, dCTP, dTTP and dATP. Traces are shown for synthesis at (i) 220 mV, (ii) 180 mV, (iii) 140 mV, and (iv) 100 mV applied potential. Synthesis was examined within the first 10 minutes after the addition of MgCl₂ to the nanopore chamber. The blue arrows below the 220 mV trace indicate the starting and end states used to quantify the synthesis rate at 220 and 100 mV.

### Example X: Replication of longer DNA templates by phi29 DNAP on the nanopore.

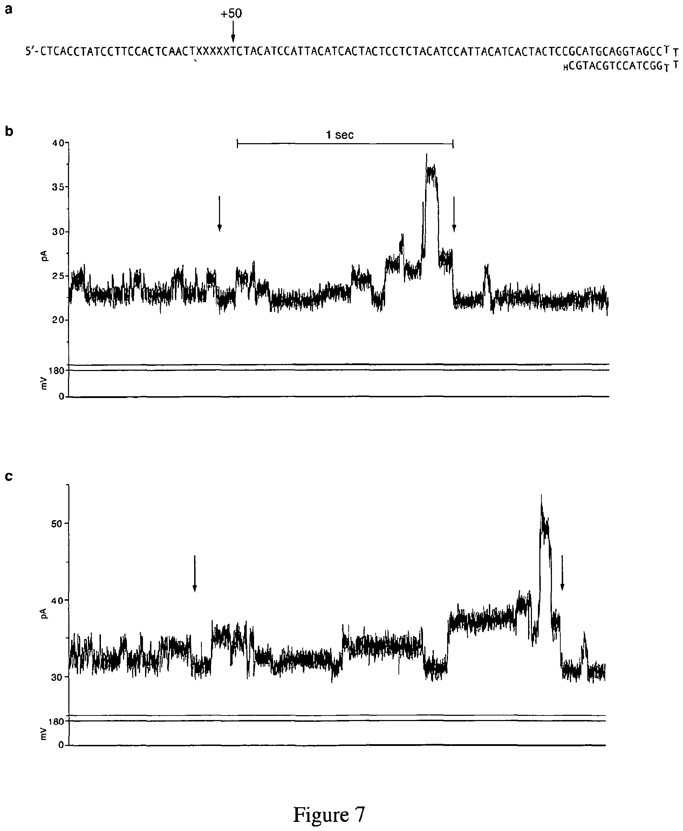

In anticipation of replicating natural DNA templates in the nanopore, we measured phi29 DNAP-dependent replication of a longer segment within a synthetic DNA hairpin substrate. This hairpin substrate had a starting abasic configuration of 5ab(50,54), and up to 50 nucleotides can be added before the enzyme reaches the abasic block (Fig. 7a). When phi29 DNAP-DNA complexes formed with this substrate were captured at 180 mV in buffer containing 0.3 M KCl, there was an initial interval of several seconds during which the current oscillated between a dominant amplitude of ∼ 23 pA, with transitions to ∼ 25 pA. In 27 out of 47 captured complexes that started with this oscillation, when this period ended, the polymerase proceeded to traverse the mapped amplitude peak (Fig.7b).

We speculated that this oscillating signature corresponds to complexes captured with the ddCMP terminus intact, prior to the ddCMP excision reaction that permits synthesis to ensue, because (i) a similar pattern invariably occurred between capture and synthesis for each successful replication reaction that subsequently traversed the abasic 35.4 pA peak in the experiments shown in Figures 4, 5, 6, and 7; (ii) the upper and lower amplitude levels of the oscillation differ among those experiments in a manner that depends upon the starting abasic configuration of the DNA substrate; (iii) those levels closely approximated the amplitudes for the binary and ternary complexes mapped for the abasic configuration for each substrate; and, (iv) the proportion of time spent in the upper or lower amplitude state can be modulated as a function of dGTP concentration (data not shown).

We therefore used the end of this oscillating state as a start point to approximate the time required for phi29 DNAP to traverse the ∼ 50 nt template segment. We measured from a small but reproducible current dip that occurred just after the oscillation ended (left blue arrow in Figure 7b) to a discernible amplitude state on the distal side of the major map peak (right blue arrow in Figure 7b). The median time required to replicate across this distance in buffer containing 0.3 M KCl was 1.39 s (IQR = 0.57 s; n = 27).

Surprisingly for this mesophilic polymerase, replication of the 5ab(50,54) substrate by phi29 DNAP was also detectable in buffer containing 0.6 M KCl (Fig. 7c). Like the replication reactions in 0.3 M KCl, these events began with a state in which the current oscillated between two levels for several seconds before the onset of synthesis (Fig. 7c). Under these higher ionic strength conditions, the current oscillated between a dominant level of ∼ 32 pA, with transitions to ∼ 34 pA. Replication that drew the abasic segment through the nanopore lumen, causing the abasic block to traverse the mapped amplitude peak, ensued in 25 out of 41 events that began with this current oscillation. In 0.6 M KCl, the median time required to traverse the distance between the end of the oscillation period (left blue arrow in Figure 7c) and the distal side of the major abasic amplitude peak (right blue arrow in Figure 7c) was 2.41 s (IQR = 1.13 s; n = 25).

Figure 7 illustrates processive DNA replication catalyzed by phi29 DNAP on the nanopore. (a) DNA hairpin substrate for nanopore replication experiments. The starting abasic configuration for this substrate is 5ab(50,54). After the exonucleolytic excision of the terminal ddCMP residue that is required prior to DNA synthesis, 50 nucleotides can be added before the enzyme reaches the abasic block (indicated by the blue arrow above the template strand sequence). During DNA synthesis, the 5 abasic insert is drawn toward the pore lumen as the first 32 nucleotides are incorporated and the abasic configuration 5ab(18,22) is reached; subsequent nucleotide additions then draw the block up to and past configuration 5ab(6,10). Thus the abasic configurations in the amplitude map in Figure 3 are spanned, (b) Representative current trace at 180 mV applied potential showing phi29 DNAP replication of the hairpin substrate shown in panel a, in buffer containing 0.3 M KCl. (c) Representative current trace at 180 mV applied potential showing phi29 DNAP replication of the hairpin substrate shown in panel a, in buffer containing 0.6 M KCl. In panels b and c, the left and right blue arrows indicate the start and end points, respectively, used to approximate the time required to replicate ∼ 50 nts along this template. Synthesis reactions were carried out in the presence of 100 µM each dGTP, dCTP, dTTP and dATP, and were examined within the first 10 minutes after the addition of MgCl₂ to the nanopore chamber.
These results were unexpectedly superior to those expected considering the prior art.

### Example XI: Noise in a current trace can help identify neighboring monomers along a polymer strand

Fig. 9a: This trace shows six average current levels (i-vi) associated with movement of a DNA strand bearing abasic residues through the alpha-HL pore controlled by phi29 DNA polymerase . The peak-to-peak noise in current level iv is significantly greater than noise in all other levels. This is caused by motion of the template around position iv which probes neighboring positions iii and v. The current associated with positions iii and v are much different than position iv, thus the noise around iv is greater predicting the identity of its neighbors.

Fig 9b: This trace shows current differences due to strand displacement by ∼3-5 angstrom as a DNA template bearing abasic residues is displaced within phi29 DNA polymerase. In panel (i) absent substrates, the dominant current is 31 pA with current deflections (noise) to about 34 pA, i.e. predicting that the next dominant state caused by strand displacement relative to the sensor will be 34 pA. In panel (ii), the next position (about 3-5 angstrom away from the first) is stabilized by substrates at the predicted 34 pA level. Occasional downward noise spikes to 31 pA confirm the identity of the monomer or monomers that previously occupied the sensor. iii) At a different position along the template strand bound to phi29 DNA polymerase, the dominant current (absent substrates) is 31 pA. Noise deflections to ∼25 pA predict the current that will dominate when the strand is stabilized one nucleotide (∼3-5 angstrom). In the presence of substrates (panel iv), the -25 pA level is stabilized confirming the prediction in (iii). Occasional noise spikes from 25 pA to 31 pA in (iv) confirm the identity of the prior monomer or monomers in the sensor.

Fig 9c: This trace shows replication and attendant 1nt movement of a DNA template in the nanopore catalyzed by phi29DNA polymerase. A single abasic reporter in the DNA template causes a large current dynamic range. Here catalysis occurred in the presence of 100 uM each of dATP, dCTP, dTTP, but only 1 uM dGTP. Distinct flicker between some states is due to 3-5 angstrom (1nt) displacement of the template strand as a dC monomer within the template reaches the catalytic domain of phi29 DNA polymerase but fails to incorporate a dG nucleotide thus returning to the prior state. As in (b), flicker predicts the next stable amplitude. This is highlighted at positions i, ii, and iii. Note at these positions the flicker is asymmetric around the current mean.

Fig 9d: This trace shows that our ability to predict subsequent ionic current amplitudes is valid for an all DNA template. In this case catalysis occurred in the presence of 100 uM each of dATP, dCTP, dGTP, but only 1 uM dTTP. Flicker from 23 pA to 22 pA at (i) occurs as phi29 DNA polymerase attempts to add dT opposite a templating dA in the catalytic domain. Failure to add dT causes the template to regress to its prior state (1 nt away) under a 180 mV load. Eventually (ii, red arrow) the dT is added, stabilizing the current at 22 pA thus allowing the template to advance further.

### Example XII: Decrease in Rate of DNA Passing through a Nanopore

We have found that binding phi29 DNA polymerase (DNAP) to single-stranded DNA (ss-DNA) dramatically reduces the rate at which the ss-DNA traverses an α-Hemolysin nanopore under a 180 mV applied potential. Single-stranded DNA threads through the phi29 DNAP and α-Hemolysin nanopore at a rate near one nucleotide per 1-100 ms.

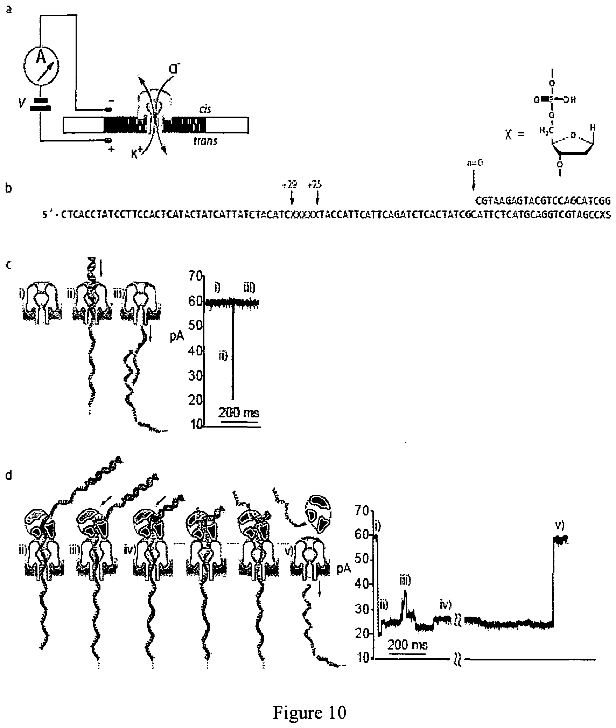

Fig. 10a shows a typical nanopore having a potential difference across the membrane. Fig. 10b shows an experimental polynucleotide ss-DNA hybridized to a short oligonucleotide probe. In this case 'X' represents an abasic nucleotide. Fig. 10c illustrates a typical cycle in the absence of phi29 DNAP representing the behavior of the oligonucleotide partially hybridized to the ss-DNA and showing the concomitant change in current across the film or membrane. In this case both stands pass through the α-Hemolysin. Fig. 10d illustrates that in the presence of phi29 DNAP, the ss-DNA target sequence is sequentially passed through the α-Hemolysin nanopore but that the oligonucleotide is iteratively un-hybridized from the ss-DNA, the ss-DNA remaining at the same position wihin the nanopore, until all the oligomer is unbound, whereupon the ss-DANA is then free to pass through. The relevant current plot showing the peaks in current at the relevant steps (steps (ii) through (v)) are shown.

### Example XIII: Protection of Primer DNA from phi29 DNAP activity

We have found that we can protect the primer DNA strand from phi29 DNAP function by binding a modified DNA oligomer adjacent to the primer template junction. Phi29 binds at the oligomer 5'-terminus and capture of this complex on an α-Hemolysin nanopore with 180 mV applied potential removes the oligomer and places phi29 at the primer terminus, after which DMA replication can take place.

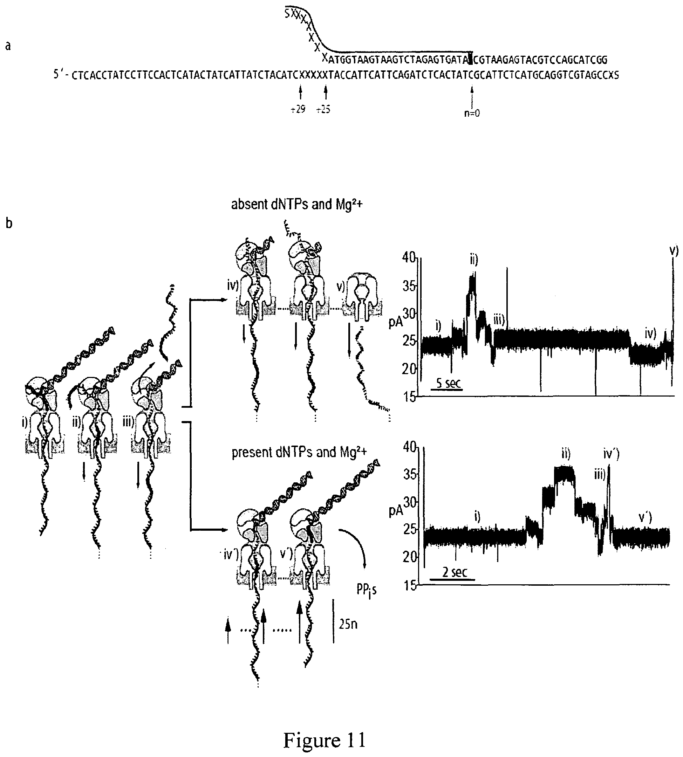

Fig. 11a illustrates a typical oligonucleotide binding to the target DNA; 'X' represents abasic nucleotides, 'S' represents the C3 (CPG) spacer. Fig. 11b illustrates contrasting signal currents in the absence of dNTPs and Mg²⁺ (upper section) or in the presence of dNTPs and Mg²⁺ (lower section).

### Example XIV: Control of Phi29 DNAP Binding Location Along a ss-DNA Substrate Using a Registry Oligomer

We have found that phi29 DNAP can bind and move along ss-DNA. We use a registry oligomer - a modified DNA oligomer - to control where phi29 DNAP binds and sits on the ss-DNA. Capture of these DNAP-DNA complexes on an α-Hemolysin nanopore using a 180 mV applied potential removes the oligomer and allows the s-DNA to translocate through phi29 DNAP and the α-Hemolysin.

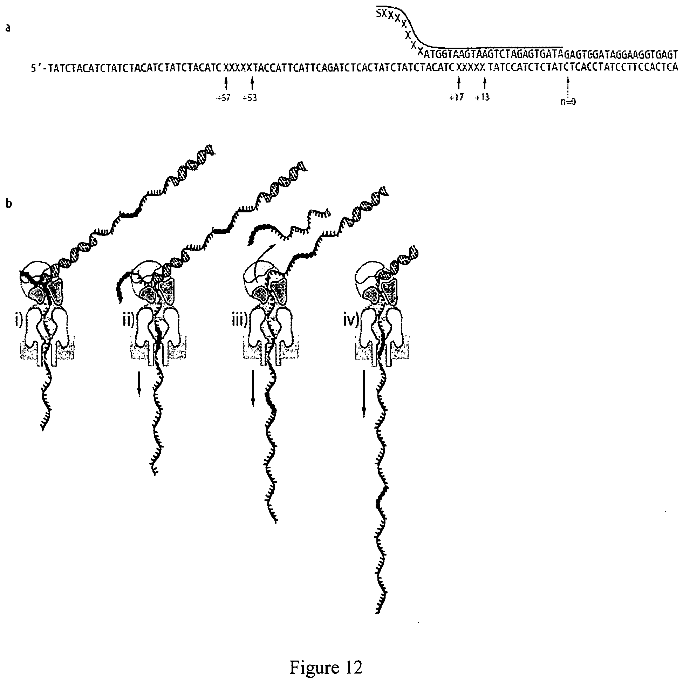

Fig. 12a illustrates a typical registry oligonucleotide binding to the target DNA; 'X' represents abasic nucleotides. Fig. 12b illustrates a typical cycle of the polynucleotide complex acting at an α-Hemolysin nanopore in the presence of phi29 DNAP.

### Example XV: Protection of Template 3' terminus from Digestion

We have found that DNA polymerase enzymes with a 3' -5' exonuclease can digest the 3' terminus of template DNA. The method uses the primer DNA 5' terminus to protect the template 3' terminus from digestion by DNA polymerases (DNAP).

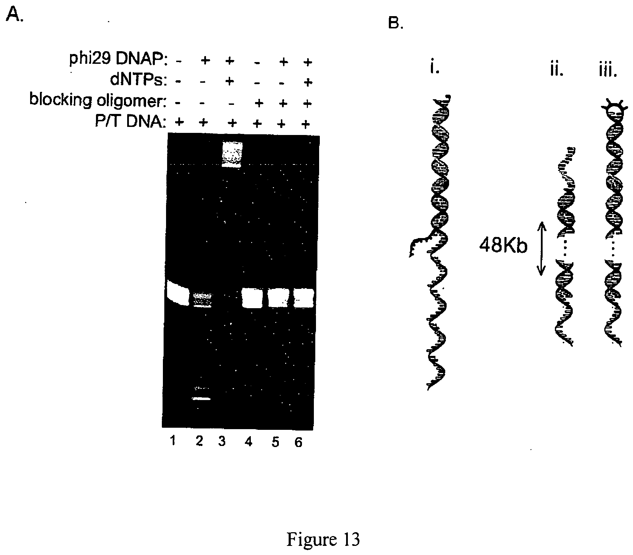

Fig. 13A shows a typical result using phi29 DNAP at an α-Hemolysin nanopore. Fig. 13B illustrates that the method may be used for ss-DNA and a blocking oligomer (i), ss-DNA and a blocking oligomer having a second primer binding (ii), and a ss-DNA forming a hairpin loop with itself (iii), further illustrating that the polynucleotide may be up to 48 kb in length, an additional advantage of the disclosure.

### Example XVI: DNA Polymerase-directed DNA Sequencing on a Nanopore using Dilute dNTP Substrate Ratios

In this experiment, we sequenced a short (∼20 mer) segment of a modified DNA template using enzyme-directed DNA syntheis through a nanopore. Here we captured pimer/template (p/t) DNA bound by phi29 DNAP on the nanopore with a 180 mV applied voltage (Fig. 14a, ii). The primer strand is protected from enzyme-directed DNA synthesis by a modified DNA strand (red) bound adjacent to the pimer/template junction. The template strand has five abasic residues (red circles) that act as a reporter for strand movement as they traverse the nanopore.

Capture of the DNA-enzymen complex reduces the ionic current through the nanopore frtom 60pA to ∼24 pA (Fig. 14a-b, ii). The applied voltage slowly ratchets the template forward through the nanopore, which removes the mdified strand and activates the p/t DNA for synthesis. This is reported by a 35 pA peak in current as five abasic residues (red circles) in the template traverse the nanopore (Fig 14a-b, ii-iv). DNA sysnthesis then initiates, which ratchess the template faster in reverse through the nanopore and results ina retrace of the 35 pA ionic current peak (Fig 14a-b, iv-vi). DNA synthesis stalls when the five abasic residues enter the enzyme active site after the addition of 25 nucleotides.

Thirty three discrete ionic current amplitudes (plotted in Fig 14c) are reported as the DNA template ratchets 25 bases forward and then backward through the nanopore. These amplitudes are symmetric about a 25 pA midpoint (arrow, position 0) that is indicative of the start of DNA synthesis. The rate of DNA synthesis, reported at positions 0-16, can be modulated by the concentration of dNTP substrates in the reaction. For example, a single dNTP is added to the primer strad approximately ever 20 milliseconds when the [dNTP] = 100 µM, and approximately every 2 seconds when [dNTP] = 1 µM, a 100-fold difference in reaction time.

When 100 nM of a single dNTP (for example, dTTP) is added to the nanopore reaction along with 100 µM all other dNTPs (dATP, dCTP, and dGTP), addition of each dTTP will take roughly 1,000 times longer than the addition of any other dNTP. Therefore the reported ionic current signal will stall at each position from 0-16 in Fig. 14c that is indicative of dTTP addition to the primer strand. When a set of four experiments are run in which a unique dNTP is dilute in each experiment, the series of dNTP additions to the template strand can be resolved and therefore the unkown template DNA can be sequenced.

Figs 14d-g summarize a set of four experiments where either 100 nM dTTP (Fig. 14d), dCTP (Fig. 14e), dGTP (Fig, 14f, or dATP (Fig. 14g) wre added to the nanopore reaction along with 100 mM of each of the three other dNTPs. The reported ionic current stalled at either one steady position or fluctuated between two positions during the addition of each dilute dNTP. In the case where the current fluctuated between two positions, the second position was labeled as the stall position. Using this criterion, we were able to reconstruct the template DNA sequence by reading the sequece of stals as 0 = C. 0-1 (1) = T, 1-2 (2) = A, and 2 = T, 2-3 (3) = C, and 3 = A, 3-4 (4) = C and 4 = T, 4-5 (5) = C, 5-6 (6) = T, 6-7 (7) = A, 7-8 (8) = G, 8-9 (9) = C, 10 = T, 10-11 (11) = A, 11-12 (12) = C, 12-13 (13) = T, 14 = A, 14-15 (15) = C. To summarize, the template sequence was reconstructed as CTATCACTCTAGACT*ACT*AC. Two dTs (marked here with asterisks) were undetected because the reported amplitudes at those positions were the same as the amplitudes of the previous dTs in the sequnce. These data show that we can accurately sequence short inserts of DNA in a modified template strand.

### Example XVII: Voltage-Activated Forward and Reverse Ratcheting of DNA on a Nanopore

Figure 15b illustrates features of blocking oligomers designed for use with phi29 DNAP. The DNA substrate is a 23-mer primer annealed to a synthetic 79-mer DNA template. To protect the DNA primer from phi29 DNAP-dependent extension and digestion in bulk phase, a blocking oligomer is annealed immediately adjacent to the DNA primer/template junction. Each blocking oligomer includes a ∼25nt complement to the template strand (Fig 15b(i)). In one case (Fig 15b(ii)) the 5' nucleotide of the blocking oligomer abuts the 3' end of the primer forming a nick. In the other case (Fig 15b(iii)) two acridine residues are attached to the 5' end of the blocking oligomer. One of these acridines substitutes for a nucleotide and abuts the primer 3' terminus; the other is an added 5'-overhang that is presumed to intercalate into the primer strand. Each blocking oligomer was appended with a 3'-C3 spacer (S) followed by seven abasic residues (shown as 'Xs'). This appended segment has two functions: protection of the blocking oligomer against exonucleolysis by phi29 DNAP; and facilitation of blocking oligomer removal as the DNA/phi29 DNAP complex is pulled into the nanopore by an applied voltage.

Figure 15c illustrates protection of DNA primers using these blocking oligomers. DNA substrates were incubated in nanopore buffer (+ 10 mM Mg²⁺) for 5 hours at 23 °C. Products were subsequently analyzed by polyacrylamide gel electrophoresis. Absent blocking oligomers, phi29 DNAP digested the DNA primer strands (-dNTP, lane 3) or extended them (+dNTP, lane 4). In contrast, when protected by either of the blocking oligomer constructs, the primer strands were not digested (-dNTP, lanes 6 and 9), nor extended (+dNTP, lanes 7 and 10) by phi29 DNAP.

Our next objective was to remove the blocking oligomer from each DNA template captured in the nanopore. We initially considered a proven strategy wherein active voltage control is used to unzip the blocking oligomer from the DNA template, followed by voltage polarity reversal to drive the newly exposed DNA primer-template junction into the *cis* well and 'fish' for a polymerase. We discovered, however, that active control was unnecessary for this application: When phi29 DNAP was added to the nanopore bath, it formed stable complexes with the DNA substrates in bulk phase that nonetheless could not be enzymatically modified due to the presence of blocking oligomers (Figure 19).

We took advantage of this discovery to pre-bind and then activate DNA substrates at the nanopore and then measure attendant replication of individual polymerase-bound DNA templates (Figure 16). The DNA substrate we used was a 79mer template bearing five abasic residues at positions +25 to +29 from the n=0 position (Figure 16a). This abasic insert serves as an ionic current reporter during strand displacement through the α-HL pore. The DNA template was annealed to a 23mer primer whose 3' terminus was protected by one of the blocking oligomers described above (Figure 15b(iii)).

An ionic current trace typical of 200 replication events from a representative experiment is shown in Figure 16b. Capture of a DNA template (Fig 16b(i)) resulted in a 23-24 pA ionic current that lasted several seconds (Figure 16b(ii)). Subsequently, under a sustained 180 mV load, the ionic current stepped through a series of discrete ionic current levels that traversed a 35 pA maximum (Fig 16b(iii)) before dropping to 22 pA and settling at a characteristic 25 pA amplitude (Fig 16b(iv)). Upon reaching the 25 pA amplitude, the ionic current reversed direction and retraced the 35 pA peak (Figure 16b(v)) at about ten times the speed of the first peak traversal before stalling at 24 pA (Figure 16b(vi)).

Our model to explain this pattern is comprised of five successive stages illustrated in Figure 16c: i) open channel; ii) nanopore capture of a polymerase-DNA complex bound to the blocking oligomer; iii) mechanical unzipping of the blocking oligomer by the applied voltage which ratchets the DNA template through the nanopore. This gives rise to the first 35 pA current peak as the abasic insert passes the major pore constriction; iv) release of the blocking oligomer thus exposing the deoxyribose terminus of the DNA primer within the catalytic site of phi29 DNAP; v) DNA replication by phi29 DNAP which ratchets the template in reverse direction through the nanopore giving rise to the second 35 pA current peak; vi) stalling of DNA replication when the abasic residues of the template strand reach the catalytic site of phi29 DNAP.

This model makes two predictions. First, because traversal of the second 35 pA ionic current peak would require DNA replication, this process should stall in the absence of key dNTP substrates. Results consistent with this prediction are described in the supplement (Figure 18). Second, our model predicts that progression through the putative replication-dependent peak should be influenced by composition of the DNA primer terminus. In particular, substitution of a 2',3'-dideoxycytidine monophosphate (ddCMP) primer terminus for the 2'-deoxycytidine monophosphate (dCMP) primer terminus should delay appearance of the second 35 pA current peak. This prediction also proved to be correct (Figure 16d). Using a ddCMP-modified primer, the first 35pA peak was traversed as in the control due to mechanical unzipping of the blocking oligomer, however the ionic current then stalled for several seconds at 25pA (arrow, position 0). Eventually, traversal of the second 35 pA peak was observed. This delay and recovery was due to removal of the terminal ddCMP by the phi29 DNAP exonuclease and subsequent strand elongation beginning at the newly exposed 3'-OH of the neighboring dGMP nucleotide.

From these experiments, we infered that phi29 DNAP can be used to control forward and reverse ratcheting of individual DNA templates through the α-HL pore at single nucleotide precision. However, for nanopore DNA sequencing, it is necessary to determine the error rate of this control process. In other words, when a single DNA molecule is ratcheted through the pore, what is the probability that correct nucleotide registry is lost due to backsliding (examining the same nucleotide position more than once) or due to skipping forward (missing a nucleotide position)? To address this question, we used a standard amplitude map to test the accuracy of the 200 translocation events (Figure 16) summarized earlier. The standard was established by first building a composite current amplitude series derived from X randomly selected traces (Figure 17a,b). Thirty-three reproducible amplitudes were resolved that were symmetric around the 25 pA midpoint (position 0). The accuracy of this map was independently verified by measuring current amplitude pauses during catalysis when one dNTP substrate at a time was reduced to 100 nM in the nanopore buffer while all other dNTPs were held at 200 uM (Figure 18). Results of this experiment are summarized in Figure 17b, where the letters G,A,T, and C denote DNA template bases in the phi29 DNAP polymerase catalytic domain where pauses were associated with a given ionic current level. In some cases, more than one letter was assigned to an amplitude because sequential template positions gave the same ionic current value. All 25 template nucleotide positions were thus accounted for among the 16 ionic current amplitudes that comprise the catalysis-driven side of the map.

We next determined how often each of the standardized amplitude positions was skipped or repeated as the other 190 DNA templates traversed back and forth through the α-HL pore (Figure 18c). In this analysis we used 3 ms as our minimum duration cutoff. It is also valid to calculate the probability of making nucleotide registry errors for each captured strand when both forward and reverse directions are considered together. Thus, Figure 18d shows the frequency of missing or repeating both corresponding positions (for example, position -15 and 15) in the ionic current series. On average, there is a 5% and 2% chance of missing or repeating both corresponding amplitudes, respectively.

Lastly, we modified the blocking oligomer to increase throughput. This was achieved by reducing the binding sequence of the blocking oligomer from 25 to 15 nucleotides (see Figure 19), which afforded equal protection of p/t DNA in bulk phase (see Figure 19) and allowed faster removal of the blocking oligomer, and thus activation of p/t DNA, on the nanopore. With this optimized blocking oligomer, a total of 500 molecules were processed over a 3.8 hour experiment (∼130 DNA templates per hour) on a single nanopore.

### Example XVIII: Other Enzyme Studies

The FPGA/FSM nanopore system can also be used for other enzyme studies. Applying voltage ramps upon capture of DNA/enzyme complexes can produce data to calculate bond energy landscapes using voltage force spectroscopy. Also, DNA's interaction with the pore can be characterized using feedback control of the applied voltage. Regulation of enzyme catalysis can be by achieved applying tension to DNA occupying the pore, counteracting the enzymes processive force.

### Example XIX: Isolation of Genomic DNA

Blood samples (2-3 ml) are collected from patients via the pulmonary catheter and stored in EDTA-containing tubes at -80°C until use. Genomic DNA is extracted from the blood samples using a DNA isolation kit according to the manufacturer's instruction (PUREGENE, Gentra Systems, Minneapolis MN). DNA purity is measured as the ratio of the absorbance at 260 and 280 nm (1 cm lightpath; A₂₆₀/A₂₈₀) measured with a Beckman spectrophotometer.

### Example XX: Identification of SNPs

A region of a gene from a patient's DNA sample is amplified by PCR using the primers specifically designed for the region. The PCR products are sequenced using methods as disclosed above. SNPs identified in the sequence traces are verified using Phred/Phrap/Consed software and compared with known SNPs deposited in the NCBI SNP databank.

### Example XXI: cDNA Library Construction

A cDNA library is constructed using RNA isolated from mammalian tissue. The frozen tissue is homogenized and lysed using a POLYTRON homogenizer (Brinkmann Instruments, Westbury N.J.) in guanidinium isothiocyanate solution. The lysates are centrifuged over a 5.7 M CsCl cushion using a SW28 rotor in an L8-70M Ultracentrifuge (Beckman Coulter, Fullerton Calif.) for 18 hours at 25,000 rpm at ambient temperature. The RNA is extracted with acid phenol, pH 4.7, precipitated using 0.3 M sodium acetate and 2.5 volumes of ethanol, resuspended in RNAse-free water, and treated with DNAse at 37° C RNA extraction and precipitation are repeated as before. The mRNA is isolated with the OLIGOTEX kit (Qiagen, Chatsworth Calif.) and used to construct the cDNA library.

The mRNA is handled according to the recommended protocols in the SUPERSCRIPT plasmid system (Invitrogen). The cDNAs are fractionated on a SEPHAROSE CL4B column (APB), and those cDNAs exceeding 400 bp are ligated into an expression plasmid. The plasmid is subsequently transformed into DH5αa competent cells (Invitrogen).

### Example XXII: Labeling of Probes and Hybridization Analyses

Nucleic acids are isolated from a biological source and applied to a substrate for standard hybridization protocols by one of the following methods. A mixture of target nucleic acids, a restriction digest of genomic DNA, is fractionated by electrophoresis through an 0.7% agarose gel in 1 x TAE [Tris-acetate-ethylenediamine tetraacetic acid (EDTA)] running buffer and transferred to a nylon membrane by capillary transfer using 20 x saline sodium citrate (SSC). Alternatively, the target nucleic acids are individually ligated to a vector and inserted into bacterial host cells to form a library. Target nucleic acids are arranged on a substrate by one of the following methods. In the first method, bacterial cells containing individual clones are robotically picked and arranged on a nylon membrane. The membrane is placed on bacterial growth medium, LB agar containing carbenicillin, and incubated at 37° C for 16 hours. Bacterial colonies are denatured, neutralized, and digested with proteinase K. Nylon membranes are exposed to UV irradiation in a STRATALINKER UV-crosslinker (Stratagene) to cross-link DNA to the membrane.

In the second method, target nucleic acids are amplified from bacterial vectors by thirty cycles of PCR using primers complementary to vector sequences flanking the insert. Amplified target nucleic acids are purified using SEPHACRYL-400 beads (Amersham Pharmacia Biotech). Purified target nucleic acids are robotically arrayed onto a glass microscope slide (Corning Science Products, Corning NY). The slide is previously coated with 0.05% aminopropyl silane (Sigma-Aldrich, St. Louis Mo.) and cured at 110° C. The arrayed glass slide (microarray) is exposed to UV irradiation in a STRATALINKER UV-crosslinker (Stratagene).

cDNA probes are made from mRNA templates. Five micrograms of mRNA is mixed with 1 µg random primer (Life Technologies), incubated at 70° C for 10 minutes, and lyophilized. The lyophilized sample is resuspended in 50 µl of 1 x first strand buffer (cDNA Synthesis systems; Life Technologies) containing a dNTP mix, [α-³² P]dCTP, dithiothreitol, and MMLV reverse transcriptase (Stratagene), and incubated at 42° C for 1-2 hours. After incubation, the probe is diluted with 42 µl dH₂O, heated to 95° C for 3 minutes, and cooled on ice. mRNA in the probe is removed by alkaline degradation. The probe is neutralized, and degraded mRNA and unincorporated nucleotides are removed using a PROBEQUANT G-50 MicroColumn (Amersham Pharmacia Biotech). Probes can be labeled with fluorescent markers, Cy3-dCTP or Cy5-dCTP (Amersham Pharmacia Biotech), in place of the radionucleotide, [³²P]dCTP.

Hybridization is carried out at 65° C in a hybridization buffer containing 0.5 M sodium phosphate (pH 7.2), 7% SDS, and 1 mM EDTA. After the substrate is incubated in hybridization buffer at 65° C for at least 2 hours, the buffer is replaced with 10 ml of fresh buffer containing the probes. After incubation at 65° C for 18 hours, the hybridization buffer is removed, and the substrate is washed sequentially under increasingly stringent conditions, up to 40 mM sodium phosphate, 1% SDS, 1 mM EDTA at 65° C. To detect signal produced by a radiolabeled probe hybridized on a membrane, the substrate is exposed to a PHOSPHORIMAGER cassette (Amersham Pharmacia Biotech), and the image is analyzed using IMAGEQUANT data analysis software (Amersham Pharmacia Biotech). To detect signals produced by a fluorescent probe hybridized on a microarray, the substrate is examined by confocal laser microscopy, and images are collected and analyzed using gene expression analysis software.

### Example XXIII: Complementary Polynucleotides

Molecules complementary to the polynucleotide, or a fragment thereof, are used to detect, decrease, or inhibit gene expression. Although use of oligonucleotides comprising from about 15 to about 30 base pairs is described, the same procedure is used with larger or smaller fragments or their derivatives (for example, peptide nucleic acids, PNAs). Oligonucleotides are designed using OLIGO 4.06 primer analysis software (National Biosciences) and SEQ ID NOs: 1-163. To inhibit transcription by preventing a transcription factor binding to a promoter, a complementary oligonucleotide is designed to bind to the most unique 5' sequence, most preferably between about 500 to 10 nucleotides before the initiation codon of the open reading frame. To inhibit translation, a complementary oligonucleotide is designed to prevent ribosomal binding to the mRNA encoding the mammalian protein.

### Example XXIV: Production of Specific Antibodies

A conjugate comprising a complex of polynucleotide and a binding protein thereof is purified using polyacrylamide gel electrophoresis and used to immunize mice or rabbits. Antibodies are produced using the protocols below. Rabbits are immunized with the complex in complete Freund's adjuvant. Immunizations are repeated at intervals thereafter in incomplete Freund's adjuvant. After a minimum of seven weeks for mouse or twelve weeks for rabbit, antisera are drawn and tested for antipeptide activity. Testing involves binding the peptide to plastic, blocking with 1% bovine serum albumin, reacting with rabbit antisera, washing, and reacting with radio-iodinated goat anti-rabbit IgG. Methods well known in the art are used to determine antibody titer and the amount of complex formation.

### Example XXV: Screening Molecules for Specific Binding with the Polynucleotide or Protein Conjugate

The polynucleotide, or fragments thereof, are labeled with ³²P-dCTP, Cy3-dCTP, or Cy5-dCTP (Amersham Pharmacia Biotech), or with BIODIPY or FITC (Molecular Probes, Eugene OR), respectively. Similarly, the conjugate comprising a complex of polynucleotide and a binding protein thereof can be labeled with radionucleide or fluorescent probes. Libraries of candidate molecules or compounds previously arranged on a substrate are incubated in the presence of labeled polynucleotide or protein. After incubation under conditions for either a polynucleotide or amino acid molecule, the substrate is washed, and any position on the substrate retaining label, which indicates specific binding or complex formation, is assayed, and the ligand is identified. Data obtained using different concentrations of the polynucleotide or protein are used to calculate affinity between the labeled polynucleotide or protein and the bound molecule.

The above description is intended to be illustrative, and not restrictive. Many other embodiments will be apparent to those of skill in the art upon reviewing the above description. The scope of the invention should, therefore, be determined with reference to the appended claims.

## Claims

1. A method of sequencing a target polynucleotide, comprising:
(a) contacting the target polynucleotide with a transmembrane pore and a Phi29 DNA polymerase such that the Phi29 DNA polymerase controls the movement of the target polynucleotide through the pore during the target polynucleotide interactions with the pore; and
(b) measuring a current passing through the pore during interaction and thereby determining the sequence of the target polynucleotide, wherein
steps (a) and (b) are carried out with a voltage applied across the pore while the Phi29 DNA polymerase is bound to the target polynucleotide, and further wherein steps (a) and (b) are carried out:
(I) in the absence of free nucleotides and the absence of an enzyme cofactor such that the Phi29 DNA polymerase moves the target polynucleotide through the pore with the field resulting from the applied voltage, optionally wherein the method further comprises: (c) lowering the voltage applied across the pore such that the Phi29 DNA polymerase moves the target polynucleotide through the pore in the opposite direction to steps (a) and (b) and nucleotides in the target polynucleotide interact with the pore; and (d) measuring the current passing through the pore during each interaction and thereby proof reading the sequence of the target polynucleotide obtained in step (b), wherein steps (c) and (d) are also carried out with a voltage applied across the pore;
or
(II) in the absence of free nucleotides and the presence of an enzyme cofactor such that the Phi29 DNA polymerase moves the target polynucleotide through the pore with the field resulting from the applied voltage, optionally wherein the method further comprises: (c) adding free nucleotides such that the Phi29 DNA polymerase moves the target polynucleotide through the pore in the opposite direction to steps (a) and (b) and nucleotides in the target polynucleotide interact with the pore; and (d) measuring the current passing through the pore during each interaction and thereby proof reading the sequence of the target polynucleotide obtained in step (b), wherein steps (c) and (d) are also carried out with a voltage applied across the pore;
or
(III) in the presence of free nucleotides and an enzyme cofactor such that the Phi29 DNA polymerase moves the target polynucleotide through the pore against the field resulting from the applied voltage, optionally wherein the method further comprises: (c) removing the free nucleotides such that the Phi29 DNA polymerase moves the target polynucleotide through the pore in the opposite direction to steps (a) and (b) and nucleotides in the target polynucleotide interact with the pore; and (d) measuring the current passing through the pore during each interaction and thereby proof reading the sequence of the target polynucleotide obtained in step (b), wherein steps (c) and (d) are also carried out with a voltage applied across the pore.

2. A method according to claim 1, which further comprises:
a) increasing the applied voltage across the pore to increase the rate of activity of the Phi29 DNA polymerase; or
b) the step of adding a blocking oligomer to regulate ssDNA movement through the pore.

3. A method according to claim 1, wherein:
a) at least a portion of the polynucleotide is double stranded; or
b) the Phi29 DNA polymerase comprises the sequence shown in SEQ ID NO: 4.

4. A method according to claim 1, wherein the pore is a transmembrane protein pore or a solid state pore.

5. A method according to claim 4, wherein:
a) the transmembrane protein pore is selected from a hemolysin, leukocidin, *Mycobacterium smegmatis* porin A (MspA), outer membrane porin F (OmpF), outer membrane porin G (OmpG), outer membrane phospholipase A, *Neisseria* autotransporter lipoprotein (NalP) and WZA; or
b) the transmembrane protein is (a) formed of eight identical subunits as shown in SEQ ID NO: 2 or (b) a variant thereof in which one or more of the seven subunits has at least 50% homology to SEQ ID NO: 2 based on amino acid identity over the entire sequence and retains pore activity; or
c) the transmembrane protein is (a) α-hemolysin formed of seven identical subunits as shown in SEQ ID NO: 2 or (b) a variant thereof in which one or more of the seven subunits has at least 50% homology to SEQ ID NO: 2 based on amino acid identity over the entire sequence and retains pore activity.

6. A system for determining the nucleotide sequence of a polynucleotide in a sample, the system comprising:
an electrical source, an anode, a cathode,
a *cis* chamber, a *trans* chamber, wherein the *cis* and the *trans* chambers are separated by a thin film, the thin film having a nanopore,
a conducting solvent,
a Phi29 DNA polymerase for controlling movement of the polynucleotide through the nanopore along a field resulting from an applied voltage, and
a plurality of dNTP molecules.

7. The system of claim 6 wherein:
a) the system further comprises at least one species of ddNTP molecule; or
b) in the plurality of dNTP molecules one dNTP molecule has a concentration at least two orders of magnitude lower than the concentration of the other dNTP molecules; or
c) the conducting solvent is an aqueous solvent.

8. The system of claim 7 further comprising a blocking oligomer which binds to the polynucleotide and is modified to increase throughput.

9. A kit for sequencing a target polynucleotide comprising (a) an apparatus comprising an electrical source, an anode, a cathode, a *cis* chamber and a *trans* chamber, wherein the *cis* and the *trans* chambers are separated by a thin film, the thin film having a nanopore; and (b) a Phi29 DNA polymerase.

## Patentansprüche

1. Verfahren zum Sequenzieren eines Ziel-Polynukleotid, umfassend:
(a) Inkontaktbringen des Ziel-Polynukleotids mit einer Transmembranpore und einer DNA-Polymerase Phi29, sodass die DNA-Polymerase Phi29 die Bewegung des Ziel-Polynukleotids durch die Pore während der Interaktionen des Ziel-Polynukleotid mit der Pore steuert; und
(b) Messen eines Stroms, der während einer Interaktion durch die Pore fließt, und dadurch Bestimmen der Sequenz des Ziel-Polynukleotids, wobei die Schritte (a) und (b) mit einer angelegten Spannung durch die Pore durchgeführt werden, während die DNA-Polymerase Phi29 an das Ziel-Polynukleotid gebunden ist, und ferner wobei Schritte (a) und (b) durchgeführt werden:
(I) in der Abwesenheit von freien Nukleotiden und der Abwesenheit von einem Enzym-Cofaktor, sodass die DNA-Polymerase Phi29 das Ziel-Polynukleotid mit dem Feld, das aus der angelegten Spannung resultiert, durch die Pore bewegt, optional wobei das Verfahren ferner Folgendes umfasst: (c) Verringern der angelegten Spannung durch die Pore, sodass die DNA-Polymerase Phi29 das Ziel-Polynukleotid durch die Pore in die entgegengesetzte Richtung zu den Schritten (a) und (b) bewegt und Nukleotide in dem Ziel-Polynukleotid mit der Pore interagieren; und (d) Messen des Stroms, der während jeder Interaktion durch die Pore fließt und dadurch Prüflesen der Sequenz des Ziel-Polynukleotids, das in Schritt (b) erlangt wird, wobei die Schritte (c) und (d) auch mit einer angelegten Spannung durch die Pore durchgeführt werden;
oder
(II) in der Abwesenheit von freien Nukleotiden und der Anwesenheit von einem Enzym-Cofaktor, sodass die DNA-Polymerase Phi29 das Ziel-Polynukleotid mit dem Feld, das aus der angelegten Spannung resultiert, durch die Pore bewegt, optional wobei das Verfahren ferner Folgendes umfasst: (c) Hinzufügen freier Nukleotide, sodass die DNA-Polymerase Phi29 das Ziel-Polynukleotid durch die Pore in die entgegengesetzte Richtung zu den Schritten (a) und (b) bewegt und Nukleotide in dem Ziel-Polynukleotid mit der Pore interagieren; und (d) Messen des Stroms, der während jeder Interaktion durch die Pore fließt und dadurch Prüflesen der Sequenz des Ziel-Polynukleotids, das in Schritt (b) erlangt wird, wobei die Schritte (c) und (d) auch mit einer angelegten Spannung durch die Pore durchgeführt werden;
oder
(III) in der Anwesenheit von freien Nukleotiden und einem Enzym-Cofaktor, sodass die DNA-Polymerase Phi29 das Ziel-Polynukleotid gegen das Feld, das aus der angelegten Spannung resultiert, durch die Pore bewegt, optional wobei das Verfahren ferner Folgendes umfasst: (c) Entfernen freier Nukleotide, sodass die DNA-Polymerase Phi29 das Ziel-Polynukleotid durch die Pore in die entgegengesetzte Richtung zu den Schritten (a) und (b) bewegt und Nukleotide in dem Ziel-Polynukleotid mit der Pore interagieren; und (d) Messen des Stroms, der während jeder Interaktion durch die Pore fließt und dadurch Prüflesen der Sequenz des Ziel-Polynukleotids, das in Schritt (b) erlangt wird, wobei die Schritte (c) und (d) auch mit einer angelegten Spannung durch die Pore durchgeführt werden;

2. Verfahren nach Anspruch 1, das ferner Folgendes umfasst:
a) Erhöhen der angelegten Spannung durch die Pore, um die Aktivitätsrate der DNA-Polymerase Phi29 zu steigern; oder
b) den Schritt eines Hinzufügens eines blockierenden Oligomers zum Regulieren einer ssDNA-Bewegung durch die Pore.

3. Verfahren nach Anspruch 1, wobei:
a) mindestens ein Abschnitt des Polynukleotids doppelsträngig ist; oder
b) die DNA-Polymerase Phi29 die in SEQ ID NO: 4 gezeigte Sequenz umfasst.

4. Verfahren nach Anspruch 1, wobei die Pore eine Transmembranprotein-Pore oder eine Festkörper-Pore ist.

5. Verfahren nach Anspruch 4, wobei:
a) die Transmembranprotein-Pore ausgewählt ist aus einem Hämolysin, Leukozidin, *Mycobacterium smegmatis* Porin A (MspA), äußere Membran Porin F (OmpF), äußere Membran Porin G (OmpG), äußere Membran Phospholipase A, Autotransporter-Lipoprotein Neisseria (NalP) und WZA; oder
b) das Transmembranprotein (a) aus acht identischen Untereinheiten gebildet ist, wie gezeigt in SEQ ID NO: 2 oder (b) aus einer Variante davon, bei der eine oder mehrere der sieben Untereinheiten mindestens 50 % Homologie mit SEQ ID NO: 2 aufweist, beruhend auf Aminosäurenidentität über die gesamte Sequenz, und Porenaktivität beibehält; oder
c) das Transmembranprotein (a) α-Hämolysin ist, das aus sieben identischen Untereinheiten gebildet ist, wie gezeigt in SEQ ID NO: 2 oder (b) aus einer Variante davon, bei der eine oder mehrere der sieben Untereinheiten mindestens 50 % Homologie mit SEQ ID NO: 2 aufweist, beruhend auf Aminosäurenidentität über die gesamte Sequenz, und Porenaktivität beibehält.

6. System zum Bestimmen der Nukleotidsequenz eines Polynukleotids in einer Probe, wobei das System Folgendes umfasst:
eine Stromquelle, eine Anode, eine Kathode,
eine *cis*-Kammer, eine *trans*-Kammer, wobei die *cis-* und die *trans*-Kammer durch eine Dünnschicht getrennt sind, wobei die Dünnschicht eine Nanopore aufweist,
ein leitendes Lösungsmittel,
eine DNA-Polymerase Phi29 zum Steuern einer Bewegung des Polynukleotids durch die Nanopore entlang eines Felds, das aus einer angelegten Spannung resultiert, und
eine Vielzahl von dNTP-Molekülen.

7. System nach Anspruch 6, wobei:
a) das System ferner mindestens eine Spezies von ddNTP-Molekül umfasst; oder
b) in der Vielzahl von dNTP-Molekülen ein dNTP-Molekül eine Konzentration mindestens zwei Größenordnungen niedriger als die Konzentration der anderen dNTP-Moleküle aufweist; oder
c) das leitende Lösungsmittel ein wässriges Lösungsmittel ist.

8. System nach Anspruch 7, ferner umfassend ein blockierendes Oligomer, das an das Polynukleotid bindet und modifiziert ist, um einen Durchsatz zu steigern.

9. Kit zum Sequenzieren eines Ziel-Polynukleotids, umfassend (a) eine Vorrichtung, die eine Stromquelle, eine Anode, eine Kathode, *cis*-Kammer und eine *trans*-Kammer, wobei die *cis-* und die *trans*-Kammer durch eine Dünnschicht getrennt sind, wobei die Dünnschicht eine Nanopore aufweist; und (b) eine DNA-Polymerase Phi29.

## Revendications

1. Procédé de séquençage d'un polynucléotide cible, comprenant :
(a) la mise en contact du polynucléotide cible avec un pore transmembranaire et une ADN polymérase Phi29 de sorte que l'ADN Phi29 polymérase commande le déplacement du polynucléotide cible à travers le pore pendant les interactions de polynucléotide cible avec le pore ; et
(b) la mesure d'un courant passant à travers le pore pendant l'interaction et ainsi la détermination de la séquence du polynucléotide cible,
les étapes (a) et (b) étant réalisées avec une tension appliquée au pore tandis que l'ADN polymérase Phi29 est liée au polynucléotide cible, et les étapes (a) et (b) étant en outre réalisées :
(I) en l'absence de nucléotides libres et en l'absence d'un cofacteur d'enzyme de sorte que l'ADN Phi29 polymérase déplace le polynucléotide cible à travers le pore avec le champ résultant de la tension appliquée, le procédé comprenant éventuellement en outre : (c) l'abaissement de la tension appliquée au pore de sorte que l'ADN polymérase Phi29 déplace le polynucléotide cible à travers le pore dans la direction opposée aux étapes (a) et (b) et que des nucléotides dans le polynucléotide cible interagissent avec le pore ; et (d) la mesure du courant passant à travers le pore pendant chaque interaction et ainsi la vérification de la séquence du polynucléotide cible obtenu à l'étape (b), les étapes (c) et (d) étant également réalisées avec une tension appliquée au pore ;
ou
(II) en l'absence de nucléotides libres et en présence d'un cofacteur d'enzyme de sorte que l'ADN polymérase Phi29 déplace le polynucléotide cible à travers le pore avec le champ résultant de la tension appliquée, le procédé comprenant éventuellement en outre : (c) l'ajout de nucléotides libres de sorte que l'ADN polymérase Phi29 déplace le polynucléotide cible à travers le pore dans la direction opposée aux étapes (a) et (b) et que des nucléotides libres interagissent avec le pore ; et (d) la mesure du courant passant à travers le pore pendant chaque interaction et ainsi la vérification de la séquence du polynucléotide cible obtenu à l'étape (b), les étapes (c) et (d) étant également réalisées avec une tension appliquée au pore ;
ou
(III) en présence de nucléotides libres et d'un cofacteur d'enzyme de sorte que l'ADN polymérase Phi29 déplace le polynucléotide cible à travers le pore contre le champ résultant de la tension appliquée, le procédé comprenant éventuellement en outre : (c) le retrait des nucléotides libres de sorte que l'ADN polymérase Phi29 déplace le polynucléotide cible à travers le pore dans la direction opposée aux étapes (a) et (b) et que des nucléotides dans le polynucléotide cible interagissent avec le pore ; et (d) la mesure du courant passant à travers le pore pendant chaque interaction et ainsi la vérification de la séquence du polynucléotide cible obtenu à l'étape (b), les étapes (c) et (d) étant également réalisées avec une tension appliquée au pore.

2. Procédé selon la revendication 1, qui comprend en outre :
a) l'augmentation de la tension appliquée au pore pour augmenter le taux d'activité de l'ADN polymérase Phi29 ; ou
b) l'étape d'ajout d'un oligomère de blocage pour réguler le déplacement d'ADNsb à travers le pore.

3. Procédé selon la revendication 1, dans lequel :
a) au moins une partie du polynucléotide est à double brin ; ou
b) l'ADN polymérase Phi29 comprend la séquence de SEQ ID NO : 4.

4. Procédé selon la revendication 1, dans lequel le pore est un pore de protéine transmembranaire ou un pore à l'état solide.

5. Procédé selon la revendication 4, dans lequel :
a) le pore de protéine transmembranaire est choisi parmi une hémolysine, une leukocidine, une porine de *Mycobacterium smegmatis* A (MspA), une porine de membrane externe F (OmpF), une porine de membrane externe G (OmpG), une phospholipase de membrane externe A, une lipoprotéine autotransporteuse de *Neisseria* (NalP) et WZA ; ou
b) la protéine transmembranaire est (a) formée de huit sous-unités identiques, comme montré dans SEQ ID NO : 2 ou (b) un variant de celle-ci dans lequel au moins une des sept sous-unités présente une homologie d'au moins 50 % avec SEQ ID NO : 2 sur la base de l'identité d'acides aminés sur l'ensemble de la séquence et conserve son activité de pore ; ou
c) la protéine transmembranaire est (a) une α-hémolysine formée de sept sous-unités identiques comme montré dans SEQ ID NO : 2 ou (b) un variant de celle-ci dans lequel au moins une des sept sous-unités présente une homologie d'au moins 50 % avec SEQ ID NO : 2 sur la base de l'identité d'acides aminés sur l'ensemble de la séquence et conserve son activité de pore.

6. Système permettant de déterminer la séquence nucléotidique d'un polynucléotide dans un échantillon, le système comprenant :
une source électrique, une anode, une cathode,
une chambre *cis,* une chambre *trans,* les chambres *cis* et *trans* étant séparées par un film mince, le film mince ayant un nanopore,
un solvant conducteur,
une ADN polymérase Phi29 destinée à réguler le déplacement du polynucléotide à travers le nanopore suivant un champ résultant d'une tension appliquée, et
une pluralité de molécules dNTP.

7. Système de la revendication 6, dans lequel :
a) le système comprend en outre au moins une espèce de molécule ddNTP ; ou
b) dans la pluralité de molécules dNTP, une molécule dNTP a une concentration d'au moins deux ordres de grandeur plus basse que la concentration des autres molécules dNTP ; ou
c) le solvant conducteur est un solvant aqueux.

8. Système de la revendication 7, comprenant en outre un oligomère de blocage qui se lie au polynucléotide et est modifié pour augmenter le rendement.

9. Kit permettant de séquencer un polynucléotide cible comprenant (a) un appareil comprenant une source électrique, une anode, une cathode, une chambre *cis,* une chambre *trans,* les chambres *cis* et *trans* étant séparées par un film mince, le film mince ayant un nanopore ; et (b) une ADN polymérase Phi29.


### SEQUENCE LISTING

<110> The Regents of the university of California
   cherf, Gerald Maxwell
   Lieberman, Kate R
   Lam, Christopher Evan
   Doody, Michael
   Akeson, Mark A
<120> Control of DNA Movement in a Nanopore at One Nucleotide Precision by a Processive Enzyme
<130> UCSC2011-156 WO
<150> 61/402,903
   <151> 2010-09-07
<150> 61/574,237
   <151> 2011-07-30
<150> 61/574,238
   <151> 2011-07-30
<150> 61/574,236
   <151> 2011-07-30
<150> 61/574,240
   <151> 2011-07-30
<150> 61/574,239
   <151> 2011-07-30
<150> 61/574,235
   <151> 2011-07-30
<150> 61/574,233
   <151> 2011-07-30
<160> 28
<170> PatentIn version 3.5
<210> 1
   <211> 885
   <212> DNA
   <213> Staphylococcus aureus
<400> 1
   atggcagatt ctgatattaa tattaaaacc ggtactacag atattggaag caatactaca     60
   gtaaaaacag gtgatttagt cacttatgat aaagaaaatg gcatgcacaa aaaagtattt    120
   tatagtttta tcgatgataa aaatcacaat aaaaaactgc tagttattag aacaaaaggt    180
   accattgctg gtcaatatag agtttatagc gaagaaggtg ctaacaaaag tggtttagcc    240
   tggccttcag cctttaaggt acagttgcaa ctacctgata atgaagtagc tcaaatatct    300
   gattactatc caagaaattc gattgataca aaaaactata tgagtacttt aacttatgga    360
   ttcaacggta atgttactgg tgatgataca ggaaaaattg gcggccttat tggtgcaaat    420
   gtttcgattg gtcatacact gaactatgtt caacctgatt tcaaaacaat tttagagagc    480
   ccaactgata aaaaagtagg ctggaaagtg atatttaaca atatggtgaa tcaaaattgg    540
   ggaccatacg atcgagattc ttggaacccg gtatatggca atcaactttt catgaaaact    600
   agaaatggtt ctatgaaagc agcagataac ttccttgatc ctaacaaagc aagttctcta    660
   ttatcttcag ggttttcacc agacttcgct acagttatta ctatggatag aaaagcatcc    720
   aaacaacaaa caaatataga tgtaatatac gaacgagttc gtgatgatta ccaattgcat    780
   tggacttcaa caaattggaa aggtaccaat actaaagata aatggacaga tcgttcttca    840
   gaaagatata aaatcgattg ggaaaaagaa gaaatgacaa attaa                    885
<210> 2
   <211> 293
   <212> PRT
   <213> Staphylococcus aureus
<400> 2
   Ala Asp Ser Asp Ile Asn Ile Lys Thr Gly Thr Thr Asp Ile Gly Ser
   1               5                   10                  15
   Asn Thr Thr Val Lys Thr Gly Asp Leu Val Thr Tyr Asp Lys Glu Asn
               20                  25                  30
   Gly Met His Lys Lys Val Phe Tyr Ser Phe Ile Asp Asp Lys Asn His
           35                  40                  45
   Asn Lys Lys Leu Leu Val Ile Arg Thr Lys Gly Thr Ile Ala Gly Gln
       50                  55                  60
   Tyr Arg Val Tyr Ser Glu Glu Gly Ala Asn Lys Ser Gly Leu Ala Trp
   65                  70                  75                  80
   Pro Ser Ala Phe Lys Val Gln Leu Gln Leu Pro Asp Asn Glu Val Ala
                   85                  90                  95
   Gln Ile Ser Asp Tyr Tyr Pro Arg Asn Ser Ile Asp Thr Lys Asn Tyr
               100                 105                 110
   Met Ser Thr Leu Thr Tyr Gly Phe Asn Gly Asn Val Thr Gly Asp Asp
           115                 120                 125
   Thr Gly Lys Ile Gly Gly Leu Ile Gly Ala Asn Val Ser Ile Gly His
       130                 135                 140
   Thr Leu Asn Tyr Val Gln Pro Asp Phe Lys Thr Ile Leu Glu Ser Pro
   145                 150                 155                 160
   Thr Asp Lys Lys Val Gly Trp Lys Val Ile Phe Asn Asn Met Val Asn
                   165                 170                 175
   Gln Asn Trp Gly Pro Tyr Asp Arg Asp Ser Trp Asn Pro Val Tyr Gly
               180                 185                 190
   Asn Gln Leu Phe Met Lys Thr Arg Asn Gly Ser Met Lys Ala Ala Asp
           195                 200                 205
   Asn Phe Leu Asp Pro Asn Lys Ala Ser Ser Leu Leu Ser Ser Gly Phe
       210                 215                 220
   Ser Pro Asp Phe Ala Thr Val Ile Thr Met Asp Arg Lys Ala Ser Lys
   225                 230                 235                 240
   Gln Gln Thr Asn Ile Asp Val Ile Tyr Glu Arg Val Arg Asp Asp Tyr
                   245                 250                 255
   Gln Leu His Trp Thr Ser Thr Asn Trp Lys Gly Thr Asn Thr Lys Asp
               260                 265                 270
   Lys Trp Thr Asp Arg Ser Ser Glu Arg Tyr Lys Ile Asp Trp Glu Lys
           275                 280                 285
   Glu Glu Met Thr Asn
       290
<210> 3
   <211> 1830
   <212> DNA
   <213> Bacteriophage phi29
<400> 3
   atgaaacaca tgccgcgtaa aatgtatagc tgcgcgtttg aaaccacgac caaagtggaa      60
   gattgtcgcg tttgggccta tggctacatg aacatcgaag atcattctga atacaaaatc     120
   ggtaacagtc tggatgaatt tatggcatgg gtgctgaaag ttcaggcgga tctgtacttc     180
   cacaacctga aatttgatgg cgcattcatt atcaactggc tggaacgtaa tggctttaaa     240
   tggagcgcgg atggtctgcc gaacacgtat aataccatta tctctcgtat gggccagtgg     300
   tatatgattg atatctgcct gggctacaaa ggtaaacgca aaattcatac cgtgatctat     360
   gatagcctga aaaaactgcc gtttccggtg aagaaaattg cgaaagattt caaactgacg     420
   gttctgaaag gcgatattga ttatcacaaa gaacgtccgg ttggttacaa aatcaccccg     480
   gaagaatacg catacatcaa aaacgatatc cagatcatcg cagaagcgct gctgattcag     540
   tttaaacagg gcctggatcg catgaccgcg ggcagtgata gcctgaaagg tttcaaagat     600
   atcatcacga ccaaaaaatt caaaaaagtg ttcccgacgc tgagcctggg tctggataaa     660
   gaagttcgtt atgcctaccg cggcggtttt acctggctga acgatcgttt caaagaaaaa     720
   gaaattggcg agggtatggt gtttgatgtt aatagtctgt atccggcaca gatgtacagc     780
   cgcctgctgc cgtatggcga accgatcgtg ttcgagggta aatatgtttg ggatgaagat     840
   tacccgctgc atattcagca catccgttgt gaatttgaac tgaaagaagg ctatattccg     900
   accattcaga tcaaacgtag tcgcttctat aagggtaacg aatacctgaa aagctctggc     960
   ggtgaaatcg cggatctgtg gctgagtaac gtggatctgg aactgatgaa agaacactac    1020
   gatctgtaca acgttgaata catcagcggc ctgaaattta aagccacgac cggtctgttc    1080
   aaagatttca tcgataaatg gacctacatc aaaacgacct ctgaaggcgc gattaaacag 1140
   ctggccaaac tgatgctgaa cagcctgtat ggcaaattcg cctctaatcc ggatgtgacc 1200
   ggtaaagttc cgtacctgaa agaaaatggc gcactgggtt ttcgcctggg cgaagaagaa 1260
   acgaaagatc cggtgtatac cccgatgggt gttttcatta cggcctgggc acgttacacg 1320
   accatcaccg cggcccaggc atgctatgat cgcattatct actgtgatac cgattctatt 1380
   catctgacgg gcaccgaaat cccggatgtg attaaagata tcgttgatcc gaaaaaactg 1440
   ggttattggg cccacgaaag tacgtttaaa cgtgcaaaat acctgcgcca gaaaacctac 1500
   atccaggata tctacatgaa agaagtggat ggcaaactgg ttgaaggttc tccggatgat 1560
   tacaccgata tcaaattcag tgtgaaatgc gccggcatga cggataaaat caaaaaagaa 1620
   gtgaccttcg aaaacttcaa agttggtttc agccgcaaaa tgaaaccgaa accggtgcag 1680
   gttccgggcg gtgtggttct ggtggatgat acgtttacca ttaaatctgg cggtagtgcg 1740
   tggagccatc cgcagttcga aaaaggcggt ggctctggtg gcggttctgg cggtagtgcc 1800
   tggagccacc cgcagtttga aaaataataa 1830
<210> 4
   <211> 608
   <212> PRT
   <213> Bacteriophage phi29
<400> 4
   Met Lys His Met Pro Arg Lys Met Tyr Ser Cys Ala Phe Glu Thr Thr
   1               5                   10                  15
   Thr Lys Val Glu Asp Cys Arg Val Trp Ala Tyr Gly Tyr Met Asn Ile
               20                  25                  30
   Glu Asp His Ser Glu Tyr Lys Ile Gly Asn Ser Leu Asp Glu Phe Met
           35                  40                  45
   Ala Trp Val Leu Lys Val Gln Ala Asp Leu Tyr Phe His Asn Leu Lys
       50                  55                  60
   Phe Asp Gly Ala Phe Ile Ile Asn Trp Leu Glu Arg Asn Gly Phe Lys
   65                  70                  75                  80
   Trp Ser Ala Asp Gly Leu Pro Asn Thr Tyr Asn Thr Ile Ile Ser Arg
                   85                  90                  95
   Met Gly Gln Trp Tyr Met Ile Asp Ile Cys Leu Gly Tyr Lys Gly Lys
               100                 105                 110
   Arg Lys Ile His Thr Val Ile Tyr Asp Ser Leu Lys Lys Leu Pro Phe
           115                 120                 125
   Pro Val Lys Lys Ile Ala Lys Asp Phe Lys Leu Thr Val Leu Lys Gly
       130                 135                 140
   Asp Ile Asp Tyr His Lys Glu Arg Pro Val Gly Tyr Lys Ile Thr Pro
   145 150 155 160
   Glu Glu Tyr Ala Tyr Ile Lys Asn Asp Ile Gln Ile Ile Ala Glu Ala
   165 170 175
   Leu Leu Ile Gln Phe Lys Gln Gly Leu Asp Arg Met Thr Ala Gly Ser
   180 185 190
   Asp Ser Leu Lys Gly Phe Lys Asp Ile Ile Thr Thr Lys Lys Phe Lys
   195 200 205
   Lys Val Phe Pro Thr Leu Ser Leu Gly Leu Asp Lys Glu Val Arg Tyr
   210 215 220
   Ala Tyr Arg Gly Gly Phe Thr Trp Leu Asn Asp Arg Phe Lys Glu Lys
   225 230 235 240
   Glu Ile Gly Glu Gly Met Val Phe Asp Val Asn Ser Leu Tyr Pro Ala
   245 250 255
   Gln Met Tyr Ser Arg Leu Leu Pro Tyr Gly Glu Pro Ile Val Phe Glu
   260 265 270
   Gly Lys Tyr Val Trp Asp Glu Asp Tyr Pro Leu His Ile Gln His Ile
   275 280 285
   Arg Cys Glu Phe Glu Leu Lys Glu Gly Tyr Ile Pro Thr Ile Gln Ile
   290 295 300
   Lys Arg Ser Arg Phe Tyr Lys Gly Asn Glu Tyr Leu Lys Ser Ser Gly
   305 310 315 320
   Gly Glu Ile Ala Asp Leu Trp Leu Ser Asn Val Asp Leu Glu Leu Met
   325 330 335
   Lys Glu His Tyr Asp Leu Tyr Asn Val Glu Tyr Ile Ser Gly Leu Lys
   340 345 350
   Phe Lys Ala Thr Thr Gly Leu Phe Lys Asp Phe Ile Asp Lys Trp Thr
   355 360 365
   Tyr Ile Lys Thr Thr Ser Glu Gly Ala Ile Lys Gln Leu Ala Lys Leu
   370 375 380
   Met Leu Asn Ser Leu Tyr Gly Lys Phe Ala Ser Asn Pro Asp Val Thr
   385 390 395 400
   Gly Lys Val Pro Tyr Leu Lys Glu Asn Gly Ala Leu Gly Phe Arg Leu
   405 410 415
   Gly Glu Glu Glu Thr Lys Asp Pro Val Tyr Thr Pro Met Gly Val Phe
               420                 425                 430
   Ile Thr Ala Trp Ala Arg Tyr Thr Thr Ile Thr Ala Ala Gln Ala Cys
           435                 440                 445
   Tyr Asp Arg Ile Ile Tyr Cys Asp Thr Asp Ser Ile His Leu Thr Gly
       450                 455                 460
   Thr Glu Ile Pro Asp Val Ile Lys Asp Ile Val Asp Pro Lys Lys Leu
   465                 470                 475                 480
   Gly Tyr Trp Ala His Glu Ser Thr Phe Lys Arg Ala Lys Tyr Leu Arg
                   485                 490                 495
   Gln Lys Thr Tyr Ile Gln Asp Ile Tyr Met Lys Glu Val Asp Gly Lys
               500                 505                 510
   Leu Val Glu Gly Ser Pro Asp Asp Tyr Thr Asp Ile Lys Phe Ser Val
           515                 520                 525
   Lys Cys Ala Gly Met Thr Asp Lys Ile Lys Lys Glu Val Thr Phe Glu
       530                 535                 540
   Asn Phe Lys Val Gly Phe Ser Arg Lys Met Lys Pro Lys Pro Val Gln
   545                 550                 555                 560
   Val Pro Gly Gly Val Val Leu Val Asp Asp Thr Phe Thr Ile Lys Ser
                   565                 570                 575
   Gly Gly Ser Ala Trp Ser His Pro Gln Phe Glu Lys Gly Gly Gly Ser
               580                 585                 590
   Gly Gly Gly Ser Gly Gly Ser Ala Trp Ser His Pro Gln Phe Glu Lys
           595                 600                 605
<210> 5
   <211> 67
   <212> DNA
   <213> synthetic
<220>
   <221> misc_feature
   <222> (19)..(23)
   <223> n is abasic nucleotide
<400> 5
   actatcatta tctacatcnn nnncatcact actccgcatg caggtagcct tttggctacc 60
tgcatgc 67
<210> 6
   <211> 67
   <212> DNA
   <213> synthetic
<220>
   <221> misc_feature
   <222> (25)..(29)
   <223> n is abasic nucleotide
<400> 6
   actatcatta tctacatcca ttacnnnnnt actccgcatg caggtagcct tttggctacc 60
   tgcatgc 67
<210> 7
   <211> 67
   <212> DNA
   <213> synthetic
<220>
   <221> misc_feature
   <222> (24)..(28)
   <223> n is abasic nucleotide
<400> 7
   actatcatta tctacatcca ttannnnnct actccgcatg caggtagcct tttggctacc 60
   tgcatgc 67
<210> 8
   <211> 67
   <212> DNA
   <213> Synthetic
<220>
   <221> misc_feature
   <222> (23)..(27)
   <223> n is abasic nucleotide
<400> 8
   actatcatta tctacatcca ttnnnnnact actccgcatg caggtagcct tttggctacc 60
   tgcatgc 67
<210> 9
   <211> 67
   <212> DNA
   <213> synthetic
<220>
   <221> misc_feature
   <222> (22)..(26)
   <223> n is abasic nucleotide
<400> 9
   actatcatta tctacatcca tnnnnncact actccgcatg caggtagcct tttggctacc 60
   tgcatgc 67
<210> 10
   <211> 67
   <212> DNA
   <213> Synthetic
<220>
   <221> misc_feature
   <222> (21)..(25)
   <223> n is abasic nucleotide
<400> 10
   actatcatta tctacatcca nnnnntcact actccgcatg caggtagcct tttggctacc 60
   tgcatgc 67
<210> 11
   <211> 67
   <212> DNA
   <213> synthetic
<220>
   <221> misc_feature
   <222> (20)..(24)
   <223> n is abasic nucleotide
<400> 11
   actatcatta tctacatccn nnnnatcact actccgcatg caggtagcct tttggctacc 60
   tgcatgc 67
<210> 12
   <211> 67
   <212> DNA
   <213> synthetic
<220>
   <221> misc_feature
   <222> (19)..(23)
   <223> n is abasic nucleotide
<400> 12
   actatcatta tctacatcnn nnncatcact actccgcatg caggtagcct tttggctacc 60
   tgcatgc 67
<210> 13
   <211> 67
   <212> DNA
   <213> Synthetic
<220>
   <221> misc_feature
   <222> (18)..(22)
   <223> n is abasic nucleotide
<400> 13
   actatcatta tctacatnnn nnacatcact actccgcatg caggtagcct tttggctacc 60
   tgcatgc 67
<210> 14
   <211> 67
   <212> DNA
   <213> Synthetic
<220>
   <221> misc_feature
   <222> (17)..(21)
   <223> n is abasic nucleotide
<400> 14
   actatcatta tctacannnn ntacatcact actccgcatg caggtagcct tttggctacc 60
   tgcatgc 67
<210> 15
   <211> 67
   <212> DNA
   <213> Synthetic
<220>
   <221> misc_feature
   <222> (16)..(20)
   <223> n is abasic nucleotide
<400> 15
   actatcatta tctacnnnnn ttacatcact actccgcatg caggtagcct tttggctacc 60
   tgcatgc 67
<210> 16
   <211> 67
   <212> DNA
   <213> Synthetic
<220>
   <221> misc_feature
   <222> (15)..(19)
   <223> n is abasic nucleotide
<400> 16
   actatcatta tctannnnna ttacatcact actccgcatg caggtagcct tttggctacc 60
   tgcatgc 67
<210> 17
   <211> 67
   <212> DNA
   <213> Synthetic
<220>
   <221> misc_feature
   <222> (14)..(18)
   <223> n is abasic nucleotide
<400> 17
   actatcatta tctnnnnnca ttacatcact actccgcatg caggtagcct tttggctacc 60
   tgcatgc 67
<210> 18
   <211> 63
   <212> DNA
   <213> Synthetic
<220>
   <221> misc_feature
   <222> (9)..(13)
   <223> n is abasic nucleotide
<400> 18
   tctacatcnn nnncacacat cacacaacca cgcatgcagg tagccttttg gctacctgca 60
   tgc 63
<210> 19
   <211> 67
   <212> DNA
   <213> synthetic
<220>
   <221> misc_feature
   <222> (16)..(20)
   <223> n is abasic nucleotide
<400> 19
   actatcatta tctacnnnnn ttacatcact actccgcatg caggtagcct tttggctacc 60
   tgcatgc 67
<210> 20
   <211> 73
   <212> DNA
   <213> Synthetic
<220>
   <221> misc_feature
   <222> (19)..(23)
   <223> n is abasic nucleotide
<400> 20
   actatcatta tctacatcnn nnncacacat cacacaacca cgcatgcagg tagccttttg 60
   gctacctgca tgc 73
<210> 21
   <211> 85
   <212> DNA
   <213> Synthetic
<220>
   <221> misc_feature
   <222> (24)..(28)
   <223> n is abasic nucleotide
<400> 21
   ctcacctatc cttccactca actnnnnntc tacatccatt acatcactac tccgcatgca 60
   ggtagccttt tggctacctg catgc 85
<210> 22
   <211> 94
   <212> DNA
   <213> Synthetic
<220>
   <221> misc_feature
   <222> (40)..(44)
   <223> n is abasic nucleotide
<220>
   <221> misc_feature
   <222> (93)..(93)
   <223> n is abasic nucleotide
<400> 22
   ctcacctatc cttccactca tactatcatt atctacatcn nnnntaccat tcattcagat 60
   ctcactatcg cattctcatg caggtcgtag ccns 94
<210> 23
   <211> 23
   <212> DNA
   <213> Synthetic
<400> 23
   ggctacgacc tgcatgagaa tgc 23
<210> 24
   <211> 32
   <212> DNA
   <213> Synthetic
<220>
   <221> misc_feature
   <222> (25)..(31)
   <223> n is abasic nucleotide
<400> 24
   atagtgagat ctgaatgaat ggtannnnnn ns 32
<210> 25
   <211> 108
   <212> DNA
   <213> Synthetic
<220>
   <221> misc_feature
   <222> (31)..(35)
   <223> n is abasic nucleotide
<220>
   <221> misc_feature
   <222> (71)..(75)
   <223> n is abasic nucleotide
<400> 25
   tatctacatc tatctacatc tatctacatc nnnnntacca ttcattcaga tctcactatc 60
   tatctacatc nnnnntatcc atctctatct cacctatcct tccactca 108
<210> 26
   <211> 33
   <212> DNA
   <213> synthetic
<220>
   <221> misc_feature
   <222> (26)..(32)
   <223> n is abasic nucleotide
<400> 26
   gatagtgaga tctgaatgaa tggtannnnn nns 33
<210> 27
   <211> 34
   <212> DNA
   <213> Synthetic
<220>
   <221> misc_feature
   <222> (1)..(2)
   <223> n is abasic nucleotide
<220>
   <221> misc_feature
   <222> (27)..(33)
   <223> n is abasic nucleotide
<400> 27
   nnatagtgag atctgaatga atggtannnn nnns 34
<210> 28
   <211> 70
   <212> DNA
   <213> Synthetic
<220>
   <221> misc_feature
   <222> (16)..(20)
   <223> n is abasic nucleotide
<220>
   <221> misc_feature
   <222> (69)..(69)
   <223> n is abasic nucleotide
<400> 28
   atcattatct acatcnnnnn taccattcat tcagatctca ctatcgcatt ctcatgcagg 60
   tcgtagccns 70